(19) Europäisches Patentamt
European Patent Office
Office européen des brevets

(11) EP 4 711 457 A1

(12) EUROPEAN PATENT APPLICATION
published in accordance with Art. 153(4) EPC

(43) Date of publication:
**18.03.2026 Bulletin 2026/12**

(21) Application number: **24803114.8**

(22) Date of filing: **11.05.2024**

(51) International Patent Classification (IPC):
***C12N 15/113*** *(2010.01)* ***A61K 48/00*** *(2006.01)*
***C12N 15/12*** *(2006.01)*

(52) Cooperative Patent Classification (CPC):
**A61K 48/00; C07K 14/435; C12N 15/113**

(86) International application number:
**PCT/CN2024/092668**

(87) International publication number:
**WO 2024/230836 (14.11.2024 Gazette 2024/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **11.05.2023 CN 202310532120**

(71) Applicants:
- **Rona Bioscience, Limited**
  **Admiralty, Hong Kong (HK)**
- **Keymed Biosciences (US) Inc.**
  **County of Sussex, Delaware 19958 (US)**

(72) Inventors:
- **HUANG, Jinyu**
  **Shanghai 201315 (CN)**
- **MENG, Guofeng**
  **Shanghai 201315 (CN)**
- **CHEN, Bo**
  **Chengdu, Sichuan 610219 (CN)**
- **LIU, Wei**
  **Chengdu, Sichuan 610219 (CN)**

(74) Representative: **Maiwald GmbH**
**Elisenhof**
**Elisenstraße 3**
**80335 München (DE)**

Remarks:
The complete document including Reference Table(s) and the Sequence Listing(s) can be downloaded from the EPO website

# (54) DSRNA MOLECULES FOR REGULATING MASP2 GENE ACTIVITY

(57) Provided are a double-stranded RNA used to inhibit the expression of mannan-binding lectin associated serine protease 2 (MASP2) in cells, a cell containing nucleotides encoding the same, and a method of using the dsRNA or cell to treat diseases or symptoms mediated by or associated with MASP2 expression in subjects.

EP 4 711 457 A1

## Description

### TECHNICAL FIELD

The present disclosure relates to the field of RNA interference.

### Background

The complement system consists of more than 30 plasma-bound and cell-bound proteins and plays an important role in congenital and acquired immunity. The proteins of the complement system function in a series of enzyme cascade reactions through a variety of protein interactions and cleavage events.

The complement system is activated through three pathways: the classical pathway, the lectin pathway, and the complement alternative pathway. The classical pathway is typically triggered by host's antibodies that bind to an exogenous antigen and therefore typically requires exposure to the antigen to generate a specific antibody response and is thus a part of the acquired immune system. The lectin pathway and complement bypass pathway are parts of the innate immune system.

Mannan-binding lectin-associated serine protease-2 (MASP-2) is involved in the lectin pathway of the complement system. Inhibition of MASP-2 does not interfere with the antibody-dependent classical complement activation pathway.

The gene encoding human MASP2 is located on human chromosome 1p36.22 and is referred to as *MASP2.* The propeptide encoded by this gene undergoes proteolysis to produce A and B chains, which then heterodimerize to form the mature serine protease MASP2 of 76 kDa. MASP2 cleaves complement components C2 and C4 in the lectin pathway of the complement system to produce C3 convertase. The protease MASP2 also plays a role in the coagulation cascade by cleaving prothrombin to form thrombin. MASP-2 exhibits thrombin-like activity. High concentrations of MASP-2 are associated with the risk of future venous thromboembolism. Inhibition of MASP2 levels protects against thrombosis. MASP2 is highly expressed in the liver. MASP2 is also found in the brain, gallbladder, heart, kidney and circulatory system. Alternative splicing of this gene yields a variety of transcriptional variants, at least one of which encodes an isoform formed by proteolytic treatment, known as Map19, which is present in plasma.

Mannose-binding lectin (mannose-binding lectin 2, MBL) induces the autoactivation of MASP2 through conformational changes. The activated form of MASP2 can initiate complement activation and function to kill target cells.

The complement system must be accurately regulated to ensure selective targeting of invading microorganisms and avoid autoimmune damage (Ricklin et al, Nat. Immunol. 11: 785-797, 2010). Abnormally regulated complement activity, as well as non-declining complement activity, can lead to autoimmune diseases, causing uncontrolled inflammatory responses and tissue destruction. Inappropriate activation of MASP2 may lead to the occurrence of many diseases, including IgA nephropathy and arthritis.

Several inhibitors for the inhibition of MASP-2 have been studied currently. Antibody-class inhibitors are, for example, monoclonal antibodies targeting MASP-2 disclosed in U.S. Pat. No. 9,011,860. Small molecule inhibitors are, for example, small molecule inhibitors of MASP-2 disclosed in WO2021113682A1 and WO2022096394A1.

Accordingly, there is a need in the art for compositions and methods for treating diseases, disorders, and conditions that benefit from the inhibition of MASP-2.

Reducing MASP-2 expression by a small interfering RNA (siRNA) based on RNA interference mechanism is a novel approach to treat diseases, disorders and conditions associated with MASP-2 activity.

### Summary of the Disclosure

The present disclosure provides novel double-stranded RNAs (dsRNAs) for inhibiting the expression of mannan-binding lectin-associated serine protease 2 (MASP2) in a cell, as well as a cell, a kit, and a pharmaceutical compositions comprising the same, and a method of inhibiting or reducing mannan-binding lectin-associated serine protease 2 (MASP2) gene expression or treating diseases or disorders benefiting from reduced mannan-binding lectin-associated serine protease 2 (MASP2) expression with the siRNA, cell, kit or pharmaceutical composition.

In a first aspect, the present disclosure provides a double stranded nucleotide (dsRNA) for inhibiting the expression of mannan-binding lectin-associated serine protease 2 (MASP2) in a cell, comprising a sense strand and an antisense strand that form a double-stranded region, wherein said sense strand and said antisense strand each independently have a length of 15-30 nucleotides, and said antisense strand comprises a nucleotide sequence of at least 15 contiguous nucleotides of the nucleotide sequence set forth in any one of SEQ ID Nos: 22-40. In some embodiments, the sense strand comprises a nucleotide sequence of at least 15 contiguous nucleotides of the nucleotide sequence set forth in any one of SEQ ID Nos: 2-20.

In some embodiments, the sense strand and the antisense strand are each independently 17-27 nucleotides in

length, preferably 18-25 nucleotides, more preferably 19-21 nucleotides in length. In some embodiments, the sense strand is 15-27 nucleotides, preferably 17-25 nucleotides, more preferably 18-23 nucleotides, more preferably 19-21 nucleotides, most preferably 19 amino acids in length. In some embodiments, the antisense strand is 15-27 nucleotides, preferably 17-25 nucleotides, more preferably 18-23 nucleotides, more preferably 19-22 nucleotides, most preferably 20 or 21 amino acids in length.

In some embodiments, the dsRNA is an siRNA.

In some embodiments, the antisense strand comprises a nucleotide sequence of at least 16 contiguous nucleotides, a nucleotide sequence of at least 17 contiguous nucleotides, a nucleotide sequence of at least 18 contiguous nucleotides, a nucleotide sequence of at least 19 contiguous nucleotides, or a nucleotide sequence of at least 20 contiguous nucleotides of the nucleotide sequence set forth in any one of SEQ ID Nos: 22-40, preferably the antisense strand comprises the nucleotide sequence set forth in any one of SEQ ID NOs 22-40.

In some embodiments, the sense strand comprises a nucleotide sequence of at least 16 contiguous nucleotides, a nucleotide sequence of at least 17 contiguous nucleotides, or a nucleotide sequence of at least 18 contiguous nucleotides of the nucleotide sequence set forth in any one of SEQ ID Nos: 2-20, preferably the antisense strand comprises the nucleotide sequence set forth in any one of SEQ ID Nos: 2-20.

In some embodiments, the siRNA comprises the paired sense strand sequence and antisense strand sequence of any one of the following pairs:

(1) sense strand: SEQIDNO 2, antisense strand: SEQIDNO 22;
(2) sense strand: SEQIDNO 3, antisense strand: SEQIDNO 23;
(3) sense strand: SEQIDNO 4, antisense strand: SEQIDNO 24;
(4) sense strand: SEQIDNO 5, antisense strand: SEQIDNO 25;
(5) sense strand: SEQIDNO 6, antisense strand: SEQIDNO 26;
(6) sense strand: SEQIDNO 7, antisense strand: SEQIDNO 27;
(7) sense strand: SEQIDNO 8, antisense strand: SEQIDNO 28;
(8) sense strand: SEQIDNO 9, antisense strand: SEQIDNO 29;
(9) sense strand: SEQIDNO 10,antisense strand: SEQIDNO 30;
(10) sense strand: SEQIDNO 11,antisense strand: SEQIDNO 31;
(11) sense strand: SEQIDNO 12,antisense strand: SEQIDNO 32;
(12) sense strand: SEQIDNO 13,antisense strand: SEQIDNO 33;
(13) sense strand: SEQIDNO 14,antisense strand: SEQIDNO 34;
(14) sense strand: SEQIDNO 15,antisense strand: SEQIDNO 35;
(15) sense strand: SEQIDNO 16,antisense strand: SEQIDNO 36;
(16) sense strand: SEQIDNO 17,antisense strand: SEQIDNO 37;
(17) sense strand: SEQIDNO 18,antisense strand: SEQIDNO 38;
(18) sense strand: SEQIDNO 19,antisense strand: SEQIDNO 39; and
(19) sense strand: SEQIDNO 20,antisense strand: SEQIDNO 40.

In some embodiments, substantially all nucleotides of the sense strand and substantially all nucleotides of the antisense strand are modified nucleotides, or all nucleotides of the sense strand and all nucleotides of the antisense strand are modified nucleotides.

In some embodiments, the sense strand and the antisense strand each independently comprise one or more nucleotide modifications selected from the group consisting of a 2'-O-methyl-modified nucleotide, a 2'-fluoro-modified nucleotide, a 2'-deoxy-modified nucleotide, and a phosphorothioate internucleotide linkage modification.

In some embodiments, the sense strand and/or antisense strand comprises an SCP-modified nucleotide.

In some embodiments, the 3' and/or 5' ends of the sense strand and/or the antisense strand comprise 1-5 phosphorothioate internucleotide linkages, preferably 2-3 phosphorothioate internucleotide linkages.

In some embodiments, the antisense strand is 21 nucleotides in length and has

(i) 2'-O-methyl modified nucleotides at positions 1, 3, 5, 7, 9, 11, 13, 15, 17, 19, and 21, and 2'-fluoro modified nucleotides at positions 2, 4, 6, 8, 10, 12, 14, 16, 18, and 20 (numbered from the 5' end); and/or

(ii) phosphorothioate internucleotide linkages between nucleotide positions 1 and 2, between nucleotide positions 2 and 3, between nucleotide positions 19 and 20, and between nucleotide positions 20 and 21 (numbered from the 5' end).

In some embodiments, the antisense strand is 21 nucleotides in length and has

(i) an SCP modification at position 1;

(ii) 2'-fluoro modifications at positions 2, 4, 6, 8, 10, 12, 14, 16, and 18 (numbered from the 5' end);

(iii) 2'-O-methyl modifications at positions 3, 5, 7, 9, 11, 13, 15, 17, 19, 20, and 21 (numbered from the 5' end); and/or

(iv) phosphorothioate internucleotide linkages between nucleotide positions 1 and 2, between nucleotide positions 2 and 3, between nucleotide positions 19 and 20, and between nucleotide positions 20 and 21 (numbered from the 5' end).

In some embodiments, the antisense strand is 21 nucleotides in length and has

(i) 2'-deoxy modifications at positions 2, 5, 7, and 12 (numbered from the 5' end);

(ii) an SCP modification at position 1 (numbered from the 5' end);

(iii) a 2'-fluoro modification at position 14 (numbered from the 5' end);

(iv) 2'-O-methyl modifications at positions 3, 4, 6, 8, 9, 10, 11, 13, 15, 16, 17, 18, 19, 20, and 21 (numbered from the 5' end); and/or

(v) phosphorothioate internucleotide linkages between nucleotide positions 1 and 2, between nucleotide positions 2 and 3, between nucleotide positions 19 and 20, and between nucleotide positions 20 and 21 (numbered from the 5' end).

In some embodiments, the sense strand is 19 nucleotides in length and has:

(i) 2'-O-methyl modified nucleotides at positions 1 to 6, and 10 to 19, and 2'-fluoro modified nucleotides at positions 7 to 9 (numbered from the 5' end); and/or

(ii) phosphorothioate internucleotide linkages between nucleotide positions 1 and 2, between nucleotide positions 2 and 3, and between nucleotide positions 18 and 19 (numbered from the 5' end).

In some embodiments, the sense strand is 19 nucleotides in length and has:

(i) 2'-O-methyl modified nucleotides at positions 1 to 6, and 10 to 19, and 2'-fluoro modified nucleotides at positions 7 to 9 (numbered from the 5' end); and/or

(ii) phosphorothioate internucleotide linkages between nucleotide positions 1 and 2, between nucleotide positions 2 and 3, between nucleotide positions 17 and 18, and between nucleotide positions 18 and 19 (numbered from the 5' end).

In some embodiments, the siRNA comprises any one of the antisense strand sequences selected from Table 5. In other embodiments, the siRNA comprises any one of the sense strand sequences selected from Table 4. In some embodiments, the siRNA comprises any one of the paired sense strand sequences and antisense strand sequences shown in Table 6.

In some embodiments, the antisense strand is selected from any one of the modified nucleotide sequences set forth in SEQ ID Nos: 100-122. In some embodiments, the sense strand is selected from any one of the modified nucleotide sequences set forth in SEQ ID Nos: 80-98.

In some preferred embodiments, the dsRNA comprises the paired modified sense strand sequence and modified antisense strand sequence of any one of the following pairs:

| | | | |
|---|---|---|---|
| sense strand: | SEQIDNO 80, | antisense strand: | SEQIDNO 100; |
| sense strand: | SEQIDNO 81, | antisense strand: | SEQIDNO 101; |
| sense strand: | SEQIDNO 82, | antisense strand: | SEQIDNO 102; |
| sense strand: | SEQIDNO 83, | antisense strand: | SEQIDNO 103; |
| sense strand: | SEQIDNO 84, | antisense strand: | SEQIDNO 104; |

(continued)

| | | | |
|---|---|---|---|
| sense strand: | SEQIDNO 85, | antisense strand: | SEQIDNO 105; |
| sense strand: | SEQIDNO 86, | antisense strand: | SEQIDNO 106; |
| sense strand: | SEQIDNO 87, | antisense strand: | SEQIDNO 107; |
| sense strand: | SEQIDNO 88, | antisense strand: | SEQIDNO 108; |
| sense strand: | SEQIDNO 89, | antisense strand: | SEQIDNO 109; |
| sense strand: | SEQIDNO 90, | antisense strand: | SEQIDNO 110; |
| sense strand: | SEQIDNO 91, | antisense strand: | SEQIDNO 111; |
| sense strand: | SEQIDNO 92, | antisense strand: | SEQIDNO 112; |
| sense strand: | SEQIDNO 93, | antisense strand: | SEQIDNO 113; |
| sense strand: | SEQIDNO 94, | antisense strand: | SEQIDNO 114; |
| sense strand: | SEQIDNO 95, | antisense strand: | SEQIDNO 115; |
| sense strand: | SEQIDNO 96, | antisense strand: | SEQIDNO 116; |
| sense strand: | SEQIDNO 97, | antisense strand: | SEQIDNO 117; and |
| sense strand: | SEQIDNO 98, | antisense strand: | SEQIDNO 118. |

In some embodiments, the dsRNA is further conjugated to a ligand moiety comprising N-acetylgalactosamine, preferably the sense strand is conjugated to the ligand moiety, further preferably the 3' end of the sense strand is conjugated to the ligand moiety.

In some embodiments, the ligand targets asialoglycoprotein receptor (ASGPR).

In some embodiments, the ligand is GL6 having the structure of:

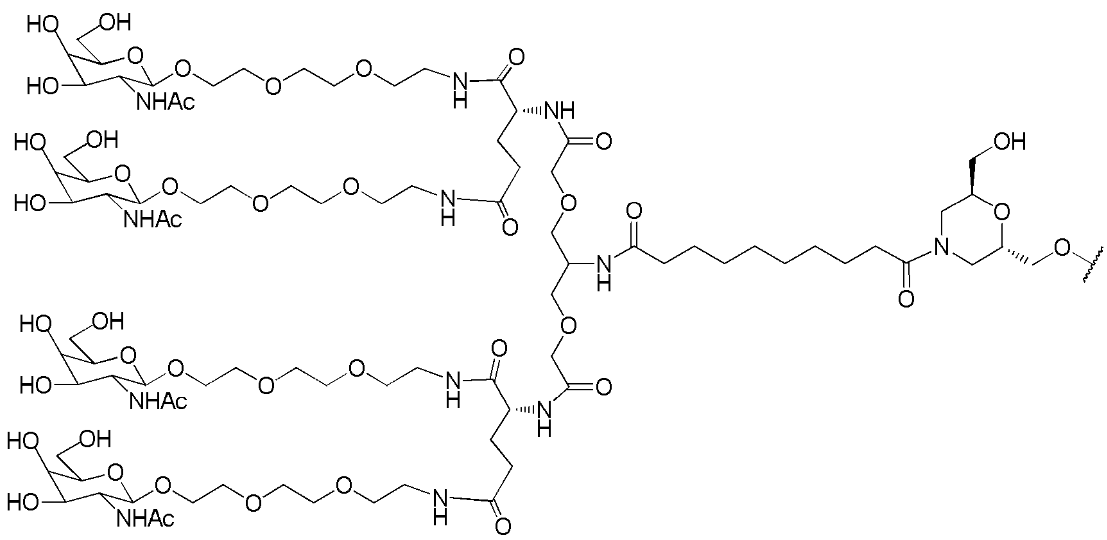

,

wherein

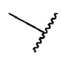

represents the point of attachment to the sense strand (preferably the 3' end of the sense strand) of the dsRNA via a phosphate group or a phosphorothioate group.

In some embodiments, the antisense strand comprises a modified nucleotide sequence selected from any one of SEQ ID Nos: 100-122. In some embodiments, the sense strand comprises a modified nucleotide sequence selected from any one of SEQ ID Nos: 60-78.

In some embodiments, the dsRNA comprises the paired modified sense strand sequence and modified antisense strand sequence of any one of the following pairs:

| | | | |
|---|---|---|---|
| sense strand: | SEQIDNO 60, | antisense strand: | SEQIDNO 100; |
| sense strand: | SEQIDNO 61, | antisense strand: | SEQIDNO 101; |

(continued)

| | | | |
|---|---|---|---|
| sense strand: | SEQIDNO 62, | antisense strand: | SEQIDNO 102; |
| sense strand: | SEQIDNO 63, | antisense strand: | SEQIDNO 103; |
| sense strand: | SEQIDNO 64, | antisense strand: | SEQIDNO 104; |
| sense strand: | SEQIDNO 65, | antisense strand: | SEQIDNO 105; |
| sense strand: | SEQIDNO 66, | antisense strand: | SEQIDNO 106; |
| sense strand: | SEQIDNO 67, | antisense strand: | SEQIDNO 107; |
| sense strand: | SEQIDNO 68, | antisense strand: | SEQIDNO 108; |
| sense strand: | SEQIDNO 69, | antisense strand: | SEQIDNO 109; |
| sense strand: | SEQIDNO 70, | antisense strand: | SEQIDNO 110; |
| sense strand: | SEQIDNO 71, | antisense strand: | SEQIDNO 111; |
| sense strand: | SEQIDNO 72, | antisense strand: | SEQIDNO 112; |
| sense strand: | SEQIDNO 73, | antisense strand: | SEQIDNO 113; |
| sense strand: | SEQIDNO 74, | antisense strand: | SEQIDNO 114; |
| sense strand: | SEQIDNO 75, | antisense strand: | SEQIDNO 115; |
| sense strand: | SEQIDNO 76, | antisense strand: | SEQIDNO 116; |
| sense strand: | SEQIDNO 77, | antisense strand: | SEQIDNO 117; |
| sense strand: | SEQIDNO 78, | antisense strand: | SEQIDNO 118; |
| sense strand: | SEQIDNO 66, | antisense strand: | SEQIDNO 119; |
| sense strand: | SEQIDNO 66, | antisense strand: | SEQIDNO 120; |
| sense strand: | SEQIDNO 66, | antisense strand: | SEQIDNO 121; and |
| sense strand: | SEQIDNO 66, | antisense strand: | SEQIDNO 122. |

In another aspect, the disclosure provides a cell comprising the dsRNA of the disclosure.

In another aspect, the disclosure provides a pharmaceutical composition comprising the dsRNA of the disclosure or the cell of the disclosure, and optionally a pharmaceutically acceptable carrier or excipient.

In another aspect, the disclosure provides a kit comprising the dsRNA of the disclosure, the cell of the disclosure, or the pharmaceutical composition of the disclosure.

In another aspect, the disclosure provides a method of treating a disease or disorder in a subject that benefits from reduced expression of mannan-binding lectin-associated serine protease 2 (MASP2), comprising the step of administering the dsRNA of the disclosure, the cell of the disclosure, or the pharmaceutical composition of the disclosure to the subject.

In another aspect, the present disclosure provides a method of preventing at least one symptom in a subject suffering from a disease or disorder benefiting from reduced expression of mannan-binding lectin-associated serine protease 2 (MASP2), comprising the step of administering the dsRNA of the disclosure, the cell of the disclosure, or the pharmaceutical composition of the disclosure to the subject.

In some embodiments, the disease or disorder benefiting from reduced expression of mannan-binding lectin-associated serine protease 2 (MASP2) is a MASP2-mediated or MASP2-related disease.

In some embodiments, the MASP2-mediated or MASP2-related disease is selected from the group consisting of arthritis, IgA nephropathy, thrombotic microangiopathy, venous embolism, diabetic nephropathy, and membranous nephropathy.

In another aspect, the disclosure provides a method of reducing the level of mannan-binding lectin-associated serine protease 2 (MASP2) in a subject, comprising the step of administering the dsRNA of the disclosure, the cell of the disclosure, or the pharmaceutical composition of the disclosure to the subject.

## Detailed Description of the Disclosure

The following describes the embodiments of the present disclosure through specific examples. Those skilled in the art can easily understand other advantages and effects of the present disclosure from the disclosure of the present specification. The present disclosure can also be implemented or applied through other different specific embodiments. Various details in this specification can also be modified or changed in various ways based on different viewpoints and applications without departing from the spirit of the present disclosure.

It should be understood that the protection scope of the present disclosure is not limited to the following specific embodiments; it should also be understood that the terms used in the embodiments of the present disclosure are for describing specific embodiments, not for limiting the protection scope of the present disclosure.

In the specification and claims of the present disclosure, the singular forms "a", "an", and "the" include plural referents unless the context clearly dictates otherwise.

When the embodiments give numerical ranges, it should be understood that, unless otherwise stated in the present disclosure, both endpoints of each numerical range and any value between the two endpoints can be selected. Unless defined otherwise, all technical and scientific terms used herein have the same meaning as commonly understood by one of ordinary skill in the art. In addition to the specific methods, equipment, and materials used in the embodiments, those skilled in the art can also use methods, equipment, and materials described in the embodiments of the present disclosure based on their understanding of the prior art and the description of the present disclosure. Any methods, equipment, and materials that are similar or equivalent to the prior art to implement the present disclosure shall fall within the protection scope of the present disclosure. Embodiments of the present disclosure are described in greater detail below.

**Definition**

A "double-stranded region" refers herein to a region comprising two nucleic acid strands that are antiparallel and complementary or substantially complementary to each other.

As used herein, the term "double-stranded RNA" or "dsRNA" refers to a ribonucleic acid molecule or a complex of ribonucleic acid molecules comprising a double-stranded region as defined above. The two portions forming the double-stranded region may be two different moieties of a larger RNA molecule, or they may be separate RNA molecules.

When the two portions are separate RNA molecules, the dsRNA herein is referred to as a Small interfering RNA or a short interfering RNA, abbreviated as siRNA.

When the two portions are two different moieties of a larger molecule, that is, when the 3' end of one moiety is connected to the 5' end of the other moiety by one or more uninterrupted nucleotides therebetween to form a double-stranded region, the uninterrupted nucleotide(s) used for this connection is(are) referred to as a "hairpin loop". When the two moieties are covalently linked by means other than a hairpin loop to form a double-stranded region, the linking structure is referred to as a "linker". Such a dsRNA, when introduced into a cell, may be cleaved by an endoribonuclease called Dicer enzyme within the cell into siRNA.

The term "siRNA" herein is a class of dsRNA molecule comprising a sense strand and an antisense strand, which can mediate the silencing of target RNA (e.g., mRNA, e.g., transcript of a protein-encoding gene) complementary or substantially complementary to the antisense strand. A siRNA is generally double-stranded, including an antisense strand complementary to the target RNA thereof and a sense strand complementary or substantially complementary to this antisense strand. For convenience, such an mRNA is also referred to herein as an mRNA to be silenced, and such a gene is also called a target gene. Typically, the RNA to be silenced is an endogenous gene or a pathogen gene. In addition, RNAs other than mRNA (for example, tRNA) and viral RNA may also be targeted.

As used herein, the term "antisense strand" refers to a strand in an siRNA, wherein said strand contains a region that is completely or substantially complementary to the target sequence thereof.

As used herein, the term "complementary region" refers to a region on an antisense strand that is completely or substantially complementary to the target mRNA sequence thereof. In cases where a complementary region is incompletely complementary to the target sequence thereof, a mismatch may be located in an internal or terminal region of the molecule. Typically, the most tolerated mismatch is located in a terminal region, e.g., within 5, 4, 3, 2 or 1 nucleotide at the 5' and/or 3' end. The portion of an antisense strand most sensitive to a mismatch is called the "seed region". For example, in a siRNA containing a strand of 19 nt, position 19 (from the 5' to the 3') may tolerate some mismatches.

As used herein, the term "complementary" refers to the ability of a first polynucleotide to hybridize with a second polynucleotide under certain conditions, such as stringent conditions. For example, stringent conditions may include 400 mM NaCl, 40 mM PIPES, pH 6.4, 1 mM EDTA, and 50°C or 70°C for 12-16 hours.

As used herein, with respect to fulfilling the above required capabilities related to the hybridization ability thereof, the "complementary" sequences may also include or be entirely composed of non-Watson-Crick base pairs and/or base pairs formed from non-natural as well as modified nucleotides. Such non-Watson-Crick base pairs include, but are not limited to, G:U wobble base pairing or Hoogsteen base pairing.

As used herein, a polynucleotide that is "at least partially complementary" or "substantially complementary" to a messenger RNA (mRNA) refers to a polynucleotide that is substantially complementary to a contiguous portion of an mRNA of interest (e.g., an mRNA encoding MASP2). For example, a polynucleotide is at least partially complementary to an mRNA encoding MASP2, when the sequence thereof is substantially complementary to an uninterrupted portion of the MASP2 mRNA.

The terms "complementary," "completely complementary," and "substantially complementary" as used herein may be used with respect to base pairing between the sense strand and the antisense strand of an siRNA, or between the antisense strand of an siRNA, and a target sequence.

As used herein, the term "sense strand" refers to one strand of an siRNA that comprises a region substantially

complementary to the region of an antisense strand as defined herein.

A "nucleoside" is a compound comprising two components: one is a purine base or a pyrimidine base, and the other is a ribose or a deoxyribose. A "nucleotide" is a compound comprising three components: one is a purine base or a pyrimidine base, another is a ribose or deoxyribose, and the third is a phosphoric acid. An "oligonucleotide" refers to, for example, a nucleic acid molecule (RNA or DNA) having a length of less than 100, 200, 300, or 400 nucleotides.

The term "base" is a fundamental building block of nucleosides, nucleotides, and nucleic acids; also referred to as "nitrogenous base," as it always contains nitrogen. Unless otherwise specified, the capital letters herein, i.e., A, U, T, G, and C, denote the bases of nucleotides, representing adenine, uracil, thymine, guanine and cytosine, respectively.

As used herein, the term "nucleotide overhang" refers to at least one unpaired nucleotide that protrudes from the double-stranded region of the siRNA. Nucleotide overhangs exist, for example, when the 3'-end of one strand of the siRNA extends beyond the 5'-end of the other strand, or vice versa. The siRNA may comprise an overhang having at least one nucleotide, an overhang having at least two nucleotides, an overhang having at least three nucleotides, an overhang having at least four nucleotides, or an overhang having at least five nucleotides or more. A nucleotide overhang may comprise or consist of a nucleotide/modified nucleotide (including a deoxynucleotide/nucleoside). One or more overhangs can be on the sense strand or the antisense strand, or any combination thereof. An overhang having one or more nucleotides may be present at the 5'-end, 3'-end, or both ends of the antisense or sense strand of the siRNA.

"Blunt end" means that there are no unpaired nucleotides, i.e., no nucleotide overhang at that end of the double-stranded siRNA. A "blunt-ended siRNA" is a siRNA that is double-stranded throughout its length, i.e., there is no nucleotide overhang at either end of the molecule.

Substantially all nucleotides of the siRNA of the present disclosure are modified. For example, substantially all nucleotides of the sense strand are modified nucleotides, or substantially all nucleotides of the antisense strand are modified nucleotides, or substantially all nucleotides of both the sense and antisense strands are modified nucleotides. In other embodiments of the present disclosure, all nucleotides of the siRNA of the present disclosure are modified nucleotides. For example, all nucleotides of the sense strand are modified nucleotides, or all nucleotides of the antisense strand are modified nucleotides, or all nucleotides of both the sense and antisense strands are modified nucleotides. As used herein, "substantially all nucleotides are modified" refers to that the majority, but not necessarily all, the nucleotides of the siRNA of the present disclosure are modified and may include no more than 5, 4, 3, 2, or 1 unmodified nucleotide(s).

A "modified nucleotide" herein include, but are not limited to, a 2'-O-alkyl modified nucleotide (e.g., a 2'-O-methyl modified nucleotide, or a 2'-methoxyethyl modified nucleotide), a 2'-fluoro modified nucleotide, a 2'-deoxy modified nucleotide, an inosine ribonucleotide, an abasic nucleotide, an inverted abasic deoxyribonucleotide, a nucleotide comprising a phosphorothioate group, a vinylphosphate modified nucleotide, a locked nucleotide, an unlocked nucleotide, a 2'-amino modified nucleotide, a 2'-C-alkyl modified nucleotide, a 2'-O-allyl modified nucleotide, a morpholino nucleotide, a phosphoramidate, a non-natural base comprising nucleotide, a terminal nucleotide linked to a cholesteryl derivative or a dodecanoic acid bisdecylamide group, a deoxyribonucleotide, a 3'-terminal deoxythymine (dT) nucleotide, a conformationally restricted nucleotide, a constrained ethyl nucleotide, a 2'-hydroxy modified nucleotide, a nucleotide comprising methylphosphonate group, a nucleotide comprising 5'-phosphate, a nucleotide comprising 5'-phosphate mimic, a glycol modified nucleotide (GNA), an SCP modified nucleotide and a 2-O-(N-methylacetamide) modified nucleotide, and the like.

For example, a "2'-fluoro modified nucleotide" refers to a nucleotide in which the hydroxyl at the 2' position of the ribosyl in a nucleotide is replaced with a fluorine atom. A "2'-O-methyl-modified nucleotide" refers to a nucleotide in which the 2'-hydroxyl of the ribosyl group is replaced with a methoxyl.

A "nucleotide comprising a phosphorothioate group" refers to a nucleotide in which one or more oxygen atoms on the phosphate in a nucleotide are replaced with a sulfur atom. A "modification of a phosphorothioate internucleotide linkage" refers to a modification in which two adjacent nucleotides are linked by a phosphorothioate.

In some embodiments, the sense strand of the siRNA of the present disclosure has a modification of at least two phosphorothioate internucleotide linkages at positions 1 to 5 (numbered from the 5' end) and/or has a modification of at least two phosphorothioate internucleotide linkages at positions 1 to 5 (numbered from the 3' end), and/or the antisense strand of the siRNA of the present disclosure has a modification of at least two phosphorothioate internucleotide linkages at positions 1 to 5 (numbered from the 5' end) and/or has a modification of at least two phosphorothioate internucleotide linkages at positions 1 to 5 (numbered from the 3' end).

As used herein, the term "ligand moiety" refers to a chemical component conjugated with a siRNA, wherein the moiety is capable of altering the distribution, targeting, or lifespan of the siRNA. In some embodiments, the ligand moiety offers enhanced affinity for selected targets, such as molecules, cells or cell types, and compartments (e.g., cellular or organ compartments, tissues, organs, or regions of the body), compared to for example a siRNA without such a ligand moiety. In some embodiments, the ligand moiety targets the asialoglycoprotein receptor (ASGPR) on hepatocytes. Binding of the ligand moiety to ASGPR mediates internalization via clathrin-coated vesicles. Maturation of endosomes leads to a decrease in the pH of lysosomes, which promotes the dissociation of the ligand-receptor complex, thereby releasing the siRNA. Conjugation of the ligand moiety targeting the asialoglycoprotein receptor (ASGPR) on hepatocytes leads to efficacy and stability of the siRNA, in vivo or intracellularly. This facilitates subcutaneous administration of the

siRNA.

As used herein, the term "inhibition" is used interchangeably with "reduction," "silencing," "downregulation," and other similar terms and includes any level of inhibition.

The phrase "inhibiting the expression of mannan-binding lectin-associated serine protease 2 *(MASP2)"* refers to inhibiting the expression of any *MASP2* gene as well as variants or mutants of the *MASP2* gene. Thus, the *MASP2* gene may be a wild-type *MASP2* gene, a mutant *MASP2* gene, or a transgenic *MASP2* gene in the case of a genetically manipulated cell, cell population, or organism.

"Inhibition of *MASP2* gene expression" includes any level of inhibition of the *MASP2* gene, such as at least partial inhibition of *MASP2* gene expression. *MASP2* gene expression can be assessed based on the level or change in the level of any variable associated with *MASP2* gene expression, such as *MASP2* mRNA level or MASP2 protein level. This level may be assessed in a single cell or population of cells (including, for example, samples derived from a subject).

Inhibition can be assessed by a reduction in absolute or relative levels of one or more variables associated with MASP2 expression compared to control levels. The control level can be any type of control level utilized in the art, such as a pre-dose baseline level or a level determined from a similar untreated or control (e.g., a buffer-only control or an inert control)-treated subject, cell, or sample.

A "hydroxyl protecting group" refers to a group that can prevent a hydroxyl from undergoing chemical reactions and can be removed under specific conditions to restore the hydroxyl. The hydroxy-protecting groups mainly include silane-type, acyl-type, or ether-type protecting groups, preferably the following: trimethylsilyl (TMS), triethylsilyl (TES), dimethylisopropylsilyl (DMIPS), diethylisopropylsilyl (DEIPS), tert-butyldimethylsilyl (TBDMS), tert-butyldiphenylsilyl (TBDPS), triisopropylsilyl (TIPS), acetyl (Ac), chloroacetyl, dichloroacetyl, trichloroacetyl, trifluoroacetyl (TFA), benzoyl, p-methoxybenzoyl, 9-fluorenylmethoxycarbonyl (Fmoc), allyloxycarbonyl (Alloc), 2,2,2-trichloroethoxycarbonyl (Troc), benzyloxycarbonyl (Cbz), tert-butoxycarbonyl (Boc), benzyl (Bn), p-methoxybenzyl (PMB), allyl, triphenylmethyl (Tr), di-p-methoxytrityl (DMTr), methoxymethyl (MOM), benzyloxymethyl (BOM), 2,2,2-trichloroethoxymethyl, 2-methoxyethoxymethyl (MEM), methylthiomethyl (MTM), p-methoxybenzyloxymethyl (PMBM).

"Halo-" or "halogen" refers to (substitution by) fluorine (F), chlorine (Cl), bromine (Br), and iodine (I).

"$C_{1-6}$ haloalkyl" refers to the aforementioned "$C_{1-6}$ alkyl", with one or more halogen groups. In some embodiments, a $C_{1-4}$ haloalkyl is particularly preferred, and a $C_{1-2}$ haloalkyl is even more preferred. Exemplary haloalkyls include, but are not limited to: $-CF_3$, $-CH_2F$, $-CHF_2$, $-CHFCH_2F$, - $CH_2CHF_2$, $-CF_2CF_3$, $-CCl_3$, $-CH_2Cl$, $-CHCl_2$, and 2,2,2-trifluoro-1,1-dimethyl-ethyl. The haloalkyls may be substituted at any substitutable connection site, for example, 1 to 5 substituent(s), 1 to 3 substituent(s), or 1 substituent.

"$C_{1-6}$ alkylene" refers to a divalent group formed by removing another hydrogen of $C_{1-6}$ alkyl and can be substituted or unsubstituted. In some embodiments, $C_{1-4}$ alkylene, $C_{2-4}$ alkylene, and $C_{1-2}$ alkylene are preferred. The unsubstituted alkylenes include, but are not limited to: methylene group ($-CH_2-$), ethylene group ($-CH_2CH_2-$), propylene group ($-CH_2CH_2CH_2-$), butylene group ($-CH_2CH_2CH_2CH_2-$), pentylene group ($-CH_2CH_2CH_2CH_2CH_2-$), and hexylene group ($-CH_2CH_2CH_2CH_2CH_2CH_2-$). Examples of said substituted alkylenes, such as a alkylene substituted by one or more alkyl (methyl) groups, include but are not limited to: substituted methylene ($-CH(CH_3)-$, and $-C(CH_3)_2-$), substituted ethylene ($-CH(CH_3)CH_2-$, $-CH_2CH(CH_3)-$, $-C(CH_3)_2CH_2-$, and - $CH_2C(CH_3)_2-$), substituted propylene ($-CH(CH_3)CH_2CH_2-$, $-CH_2CH(CH_3)CH_2-$, $-CH_2CH_2CH(CH_3)-$, $-C(CH_3)_2CH_2CH_2-$, $-CH_2C(CH_3)_2CH_2-$, $-CH_2CH_2C(CH_3)_2-$), etc.

As used herein, the terms "treat," "treatment," and the like refer to the administration of an agent or the performing of a procedure in order to achieve effects. These effects may be prophylactic in terms of complete or partial prevention of a disease or a symptom thereof, and/or may be therapeutic in terms of affecting partial or complete cure of a disease and/or a symptom thereof. As used herein, "treating" may include treating a disease or disorder (e.g., cancer) in a mammal, particularly a human, and comprises: (a) preventing the occurrence of a disease or the symptom thereof (e.g., including a disease that may be associated with or caused by a primary disease) in a subject who is susceptible to but has not been diagnosed with the disease; (b) inhibiting, i.e. preventing the progression of, a disease; (c) relieving, i.e. causing regression of, a disease. Treatment may refer to any reference to success in treating or ameliorating or preventing a cancer, including any objective or subjective parameter, such as elimination; remission; diminishing of a symptom, or making the disease condition more tolerable to the patient; slowing down the deterioration or decline; or debilitation or reduction of the worsening endpoint. Treatment or amelioration of a symptom is based on one or more objective or subjective parameters, including the results of the examination by a doctor. Accordingly, the term "treatment" includes administration of the antibody or the composition or the conjugate disclosed herein to prevent or delay, alleviate or arrest, or inhibit the development of the symptom or condition associated with a disease (e.g., cancer). The term "therapeutic effect" refers to the reduction, elimination, or prevention of a disease, a symptom of the disease, or a side effect of the disease in a subject.

As used herein, the term "effective amount" refers to an amount sufficient to effect treatment of a disease when administered to a subject for treating the disease.

As used herein, the term "subject" refers to any mammalian subject for whom diagnosis, cure, or treatment is desired. A "mammal" for therapeutic purposes refers to any animal classified as a mammal, including humans, domestic and farm animals, as well as laboratory animals, zoo animals, sports animals, or pet animals, such as dogs, horses, cats,

cows, sheep, goats, pigs, mice, rats, rabbits, guinea pigs, monkeys, and the like.

## I. dsRNA

The present disclosure provides a double-stranded nucleotide (dsRNA) for inhibiting the expression of mannan-binding lectin-associated serine protease 2 (MASP2) in a cell, comprising a sense strand and an antisense strand that form a double-stranded region, wherein the sense strand and the antisense strand are each independently 15-30 nucleotides in length, and the antisense strand comprises a nucleotide sequence of at least 15 contiguous nucleotides of the nucleotide sequence set forth in any one of SEQ ID Nos: 22-40.

In some embodiments, the double-stranded region formed by the sense strand and the antisense strand is completely complementary. In other embodiments, the double-stranded region formed by the sense strand and the antisense strand is substantially complementary and may contain 1, 2, 3, 4, or 5 non-complementary sites.

In some specific embodiments, the sense strand comprises a nucleotide sequence of at least 15 contiguous nucleotides of the nucleotide sequence set forth in any one of SEQ ID Nos: 2-20.

In some embodiments, the sense strand and the antisense strand are each independently 17-27 nucleotides in length, preferably 18-25 nucleotides, more preferably 19-21 nucleotides in length.

In some embodiments, the length of the double-stranded region is 15-25 nucleotide pairs, preferably 16-23 nucleotide pairs, more preferably 18-20 nucleotide pairs.

In some embodiments, the dsRNA of the disclosure is an siRNA. In other embodiments, a hairpin loop is formed between the sense strand and the antisense strand of the dsRNA of the disclosure.

One or both of the sense strand and the antisense strand comprise a 3' overhang and/or a 5' overhang of at least 1 nucleotide; for example, one or both of the sense strand and the antisense strand comprise(s) a 3' overhang and/or a 5' overhang of at least 1 nucleotide. In some preferred embodiments, the antisense strand comprises a 3' overhang and/or a 5' overhang of at least 2 nucleotides, and preferably, the antisense strand comprises a 3' overhang and/or a 5' overhang of 2 nucleotides. In some embodiments, the sense strand and the antisense strand are of the same length. In some embodiments, the full length of the sense strand is complementary to the full length of the antisense strand to form a double strand, i.e., having blunt ends. In some other embodiments, the sense strand and the antisense strand are of the same length, and a portion of the sense strand is complementary to a portion of the antisense strand, that is, both the sense strand and the antisense strand have 5' overhangs. In some embodiments, the sense strand and the antisense strand are of different lengths. In a preferred embodiment, the 5' end of the antisense strand has an overhang of at least 1 nucleotide, more preferably 2 or 3 nucleotides.

The dsRNA of the present disclosure includes a dsRNA having a nucleotide overhang at one end (i.e., an agent having one overhang and one blunt end) or having nucleotide overhangs at both ends. For example, the 5'-end of the sense strand of a dsRNA comprises an overhang having one or more nucleotides and the 3'-end of the sense strand comprises an overhang having one or more nucleotides. For example, the 5'-end of the antisense strand of a dsRNA comprises an overhang having one or more nucleotides and the 3'-end of the antisense strand comprises an overhang having one or more nucleotides. For example, the 5'-end of the sense strand of a dsRNA comprises an overhang having one or more nucleotides and the 5'-end of the antisense strand comprises an overhang having one or more nucleotides. For example, the 3'-end of the sense strand of a dsRNA comprises an overhang having one or more nucleotides and the 3'-end of the antisense strand comprises an overhang having one or more nucleotides. For example, the 5'-end of the sense strand of a dsRNA comprises an overhang having one or more nucleotides and the 3'-end of the sense strand comprises a blunt end. For example, the 3'-end of the sense strand of a dsRNA comprises an overhang having one or more nucleotides and the 3'-end of the sense strand comprises a blunt end. For example, the 5'-end of the antisense strand of a dsRNA comprises an overhang having one or more nucleotides and the 3'-end of the antisense strand comprises a blunt end. For example, the 3'-end of the antisense strand of a dsRNA comprises an overhang having one or more nucleotides and the 5'-end of the antisense strand comprises a blunt end.

In some embodiments, the antisense strand comprises a nucleotide sequence of at least 16 contiguous nucleotides, a nucleotide sequence of at least 17 contiguous nucleotides, a nucleotide sequence of at least 18 contiguous nucleotides, a nucleotide sequence of at least 19 contiguous nucleotides, or a nucleotide sequence of at least 20 contiguous nucleotides of the nucleotide sequence set forth in any one of SEQ ID Nos: 22-40, preferably the antisense strand comprises the nucleotide sequence set forth in any one of SEQ ID Nos: 22-40.

In some embodiments, the sense strand comprises a nucleotide sequence of at least 16 contiguous nucleotides, a nucleotide sequence of at least 17 contiguous nucleotides, or a nucleotide sequence of at least 18 contiguous nucleotides of the nucleotide sequence set forth in any one of SEQ ID Nos: 2-20, preferably the antisense strand comprises the nucleotide sequence set forth in any one of SEQ ID Nos: 2-20.

In some embodiments, the siRNA comprises the paired sense strand sequences and antisense strand sequence of any one of the following pairs:

(1) sense strand: SEQIDNO 2, antisense strand: SEQIDNO 22;
(2) sense strand: SEQIDNO 3, antisense strand: SEQIDNO 23;
(3) sense strand: SEQIDNO 4, antisense strand: SEQIDNO 24;
(4) sense strand: SEQIDNO 5, antisense strand: SEQIDNO 25;
(5) sense strand: SEQIDNO 6, antisense strand: SEQIDNO 26;
(6) sense strand: SEQIDNO 7, antisense strand: SEQIDNO 27;
(7) sense strand: SEQIDNO 8, antisense strand: SEQIDNO 28;
(8) sense strand: SEQIDNO 9, antisense strand: SEQIDNO 29;
(9) sense strand: SEQIDNO 10,antisense strand: SEQIDNO 30;
(10) sense strand: SEQIDNO 11,antisense strand: SEQIDNO 31;
(11) sense strand: SEQIDNO 12,antisense strand: SEQIDNO 32;
(12) sense strand: SEQIDNO 13,antisense strand: SEQIDNO 33;
(13) sense strand: SEQIDNO 14,antisense strand: SEQIDNO 34;
(14) sense strand: SEQIDNO 15,antisense strand: SEQIDNO 35;
(15) sense strand: SEQIDNO 16,antisense strand: SEQIDNO 36;
(16) sense strand: SEQIDNO 17,antisense strand: SEQIDNO 37;
(17) sense strand: SEQIDNO 18,antisense strand: SEQIDNO 38;
(18) sense strand: SEQIDNO 19,antisense strand: SEQIDNO 39; and
(19) sense strand: SEQIDNO 20,antisense strand: SEQIDNO 40.

## II. Modification of Nucleotides

In some embodiments, substantially all nucleotides of the sense strand and substantially all nucleotides of the antisense strand are modified nucleotides. In some embodiments, at least 80% of the nucleotides of the sense strand are modified nucleotides, and/or at least 80%, at least 85%, at least 90%, at least 92%, at least 95% of the nucleotides of the antisense strand are modified nucleotides. In some embodiments, at least 80% of the nucleotides of the antisense strand are modified nucleotides, and/or at least 80%, at least 85%, at least 90%, at least 92%, at least 95% of the nucleotides of the antisense strand are modified nucleotides. In some embodiments, at least 80% of the nucleotides of the sense strand are modified nucleotides, and/or at least 80%, at least 85%, at least 90%, at least 92%, at least 95% of the nucleotides of the antisense strand are modified nucleotides, and at least 80% of the nucleotides of the antisense strand are modified nucleotides, and/or at least 80%, at least 85%, at least 90%, at least 92%, at least 95% of the nucleotides of the antisense strand are modified nucleotides.

In some embodiments, all nucleotides of the sense strand are modified nucleotides and/or all nucleotides of the antisense strand are modified nucleotides.

The modification of the nucleotide of the present disclosure may be a modification on the phosphate group, the ribose group and/or the base group of the nucleotide.

In some specific embodiments, the sense strand and the antisense strand each independently comprise one or more nucleotide modifications selected from the group consisting of: 2'-O-alkyl-modified nucleotides (e.g., 2'-O-methyl-modified nucleotides, 2'-methoxyethyl-modified nucleotides), 2'-fluoro-modified nucleotides, 2'-deoxy-modified nucleotides, inosine ribonucleotides, abasic nucleotides, inverted abasic deoxyribonucleotides, a nucleotide comprising phosphorothioate groups, vinyl phosphate-modified nucleotides, locked nucleotides, unlocked nucleotides, 2'-amino-modified nucleotides, 2'-C-alkyl-modified nucleotides, 2'-O-allyl-modified nucleotides, morpholino-modified nucleotides, phosphoramidate, nucleotides containing non-natural bases, terminal nucleotides linked to cholesterol-based derivatives or didecanoyl lauroyl groups, deoxyribonucleotides, 3'-terminal deoxythymidine (dT) nucleotides, conformationally restricted nucleotides, restricted ethyl nucleotides, 2'-hydroxyl-modified nucleotides, nucleotides containing methylphosphonate groups, nucleotides containing 5'-phosphate, nucleotides containing 5'-phosphate mimics, glycol-modified nucleotides (GNA), and 2-O-(N-methylacetamide)-modified nucleotides.

In some preferred embodiments, the sense strand and the antisense strand each independently comprise one or more nucleotide modifications selected from the group consisting of 2'-O-methyl-modified nucleotides, 2'-fluoro-modified nucleotides, 2'-deoxy-modified nucleotides, SCP-modified nucleotides, and phosphorothioate internucleotide linkage modifications.

In some preferred embodiments, the sense strand and/or the antisense strand comprises at least two 2'-fluoro-modified nucleotides. In some preferred embodiments, the sense strand and/or the antisense strand comprises at least 8 2'-O-methyl-modified nucleotides. In some preferred embodiments, the 3' and/or 5' ends of the sense strand and/or the antisense strand comprise 1-5 phosphorothioate groups, preferably 2-3 phosphorothioate groups. In some preferred embodiments, the sense strand and/or antisense strand comprises an adenine deoxyribonucleotide, a thymine deoxyribonucleotide, a guanine deoxyribonucleotide, and/or a cytosine deoxyribonucleotide. In a more preferred embodiment, the sense strand and/or antisense strand comprises a thymine deoxyribonucleotide. In a most preferred embodiment, the

sense strand comprises a thymine deoxyribonucleotide.

In some embodiments, the antisense strand is 21 nucleotides in length and has

(i) 2'-O-methyl modified nucleotides at positions 1, 3, 5, 7, 9, 11, 13, 15, 17, 19, and 21, and 2'-fluoro modified nucleotides at positions 2, 4, 6, 8, 10, 12, 14, 16, 18, and 20 (numbered from the 5' end); and/or

(ii) phosphorothioate internucleotide linkages between nucleotide positions 1 and 2, between nucleotide positions 2 and 3, between nucleotide positions 19 and 20, and between nucleotide positions 20 and 21 (numbered from the 5' end).

In some embodiments, the antisense strand is 21 nucleotides in length and has

(i) an SCP modification at position 1 (numbered from the 5' end);

(ii) 2'-fluoro modifications at positions 2, 4, 6, 8, 10, 12, 14, 16, and 18 (numbered from the 5' end);

(iii) 2'-O-methyl modifications at positions 3, 5, 7, 9, 11, 13, 15, 17, 19, 20, and 21 (numbered from the 5' end); and/or

(iv) phosphorothioate internucleotide linkages between nucleotide positions 1 and 2, between nucleotide positions 2 and 3, between nucleotide positions 19 and 20, and between nucleotide positions 20 and 21 (numbered from the 5' end).

In some embodiments, the antisense strand is 21 nucleotides in length and has

(i) 2'-deoxy modifications at positions 2, 5, 7, and 12 (numbered from the 5' end);

(ii) an SCP modification at position 1 (numbered from the 5' end);

(iii) a 2'-fluoro modification at position 14 (numbered from the 5' end);

(iv) 2'-O-methyl modifications at positions 3, 4, 6, 8, 9, 10, 11, 13, 15, 16, 17, 18, 19, 20, and 21 (numbered from the 5' end); and/or

(v) phosphorothioate internucleotide linkages between nucleotide positions 1 and 2, between nucleotide positions 2 and 3, between nucleotide positions 19 and 20, and between nucleotide positions 20 and 21 (numbered from the 5' end).

In some embodiments, the sense strand is 19 nucleotides in length and has:

(i) 2'-O-methyl modified nucleotides at positions 1 to 6, and 10 to 19, and 2'-fluoro modified nucleotides at positions 7-9 (numbered from the 5' end); and/or

(ii) phosphorothioate internucleotide linkages between nucleotide positions 1 and 2, between nucleotide positions 2 and 3, and between nucleotide positions 18 and 19 (numbered from the 5' end).

In some embodiments, the sense strand is 19 nucleotides in length and has:

(i) 2'-O-methyl modified nucleotides at positions 1 to 6, and 10 to 19, and 2'-fluoro modified nucleotides at positions 7-9 (numbered from the 5' end); and/or

(ii) phosphorothioate internucleotide linkages between nucleotide positions 1 and 2, between nucleotide positions 2 and 3, between nucleotide positions 17 and 18, and between nucleotide positions 18 and 19 (numbered from the 5' end).

In some embodiments, the siRNA comprises any one of the antisense strand sequences selected from Table 5. In other embodiments, the siRNA comprises any one of the sense strand sequences selected from Table 4. In some embodiments, the siRNA comprises any one of the paired sense strand and antisense strand sequences shown in Table 6.

### III. Ligand Moiety

The dsRNA of the disclosure is further conjugated to a ligand moiety comprising N-acetylgalactosamine. In a preferred embodiment, the sense strand of the dsRNA is conjugated to the ligand moiety. In some preferred embodiments, the 3' end of the sense strand is conjugated to the ligand moiety. In other preferred embodiments, the 5' end of the sense strand is conjugated to the ligand moiety.

In some embodiments, the ligand moiety comprises a conjugate group represented by formula (X'):

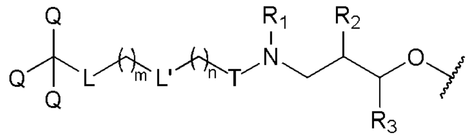

(X')

wherein

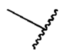

represents the point of attachment to the dsRNA;
Q is independently H,

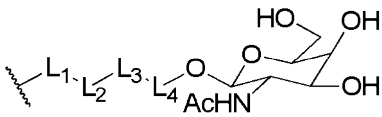

,

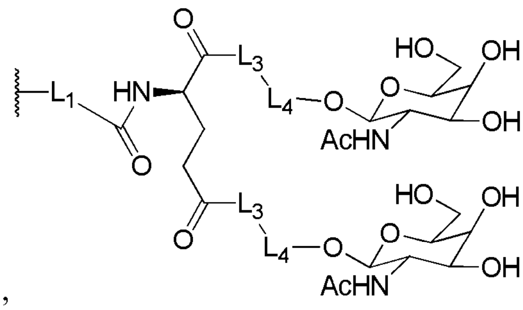

,

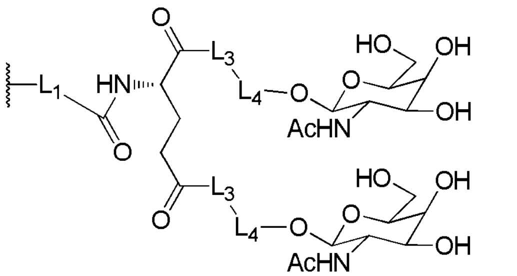

,

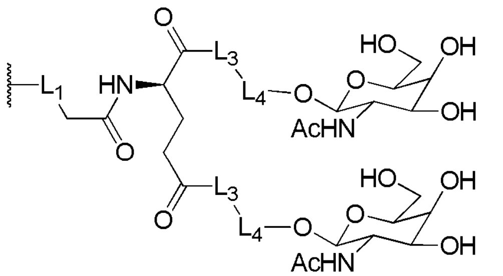

,

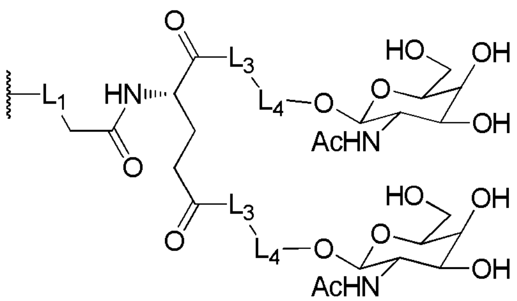

,

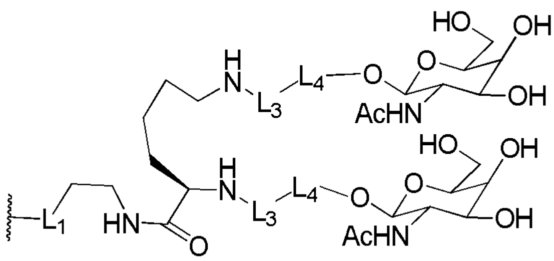

or

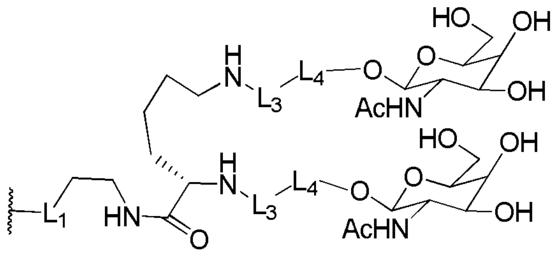

;

wherein $L_1$ is a chemical bond, $-CH_2-$, $-CH_2CH_2-$, -C(O)-, $-CH_2O-$, $-CH_2O-CH_2CH_2O-$ or - NHC(O)-(CH2NHC(O))$_a$-;
$L_2$ is a chemical bond or $-CH_2CH_2C(O)-$;
$L_3$ is a chemical bond, $-(NHCH_2CH_2)_b-$, $-(NHCH_2CH_2CH_2)_b-$ or $-C(O)CH_2-$;
L4 is $-(OCH_2CH_2)_c-$, $-(OCH_2CH_2CH_2)_c-$, $-(OCH_2CH_2CH_2CH_2)_c-$, $-(OCH_2CH_2CH_2CH_2CH_2)_c-$ or $-NHC(O)-(CH_2)_d-$;
wherein a = 0, 1, 2, or 3;
b = 1, 2, 3, 4 or 5;
c = 1, 2, 3, 4 or 5;
d = 1, 2, 3, 4, 5, 6, 7, or 8;
L is a chemical bond, $-CH_2O-$ or -NHC(O)-;
L' is a chemical bond, -C(O)NH-, -NHC(O)- or $-O(CH_2CH_2O)_e-$;
wherein e is 1, 2, 3, 4 or 5;
T is a chemical bond, $-CH_2-$, -C(O)-, -M-, $-CH_2-M-$ or -C(O)-M-;
wherein M is

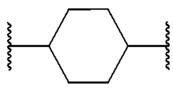
, 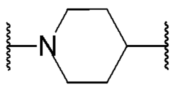
, 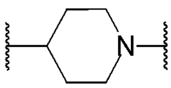
or 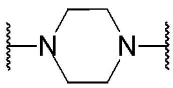
;

$R_1$ and $R_2$ together form $-CH_2CH_2O-$ or $-CH_2CH(R)-O-$, and $R_3$ is H;
or $R_1$ and $R_3$ together form $-C_{1-2}$ alkylene-, and $R_2$ is H;
wherein R is-OR', $-CH_2OR'$ or $-CH_2CH_2OR'$, wherein R' is H, a hydroxyl protecting group, or a solid support, preferably wherein the hydroxyl protecting group is $-C(O)CH_2CH_2C(O)OH$ or 4,4'-dimethoxytrityl;
m = 0, 1, 2, 3, 4, 5, 6, 7, 8, 9 or 10;
n = 0, 1, 2, 3, 4, 5, 6, 7, 8, 9, or 10.

In some embodiments, the conjugating group is represented by Formula (I'):

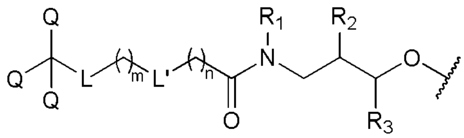

(I')

wherein

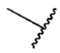

represents the point of attachment to the dsRNA;

Q is independently H,

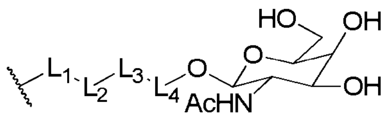,
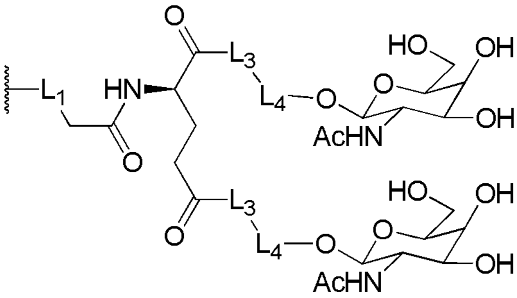,

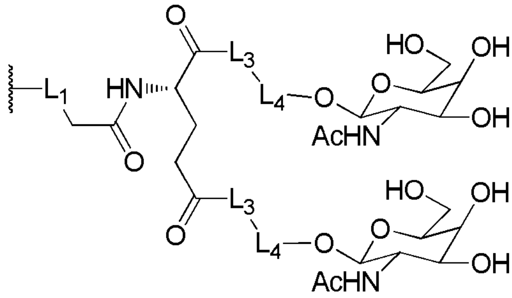,
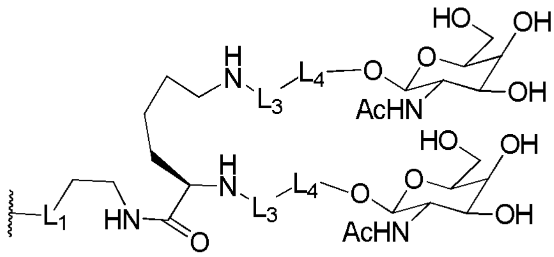

or

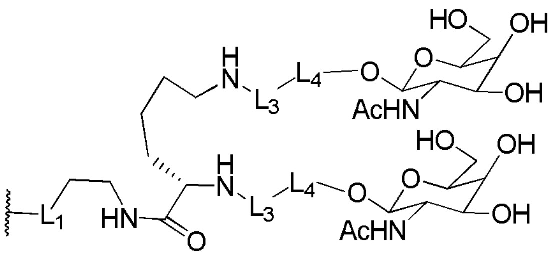;

wherein $L_1$ is a chemical bond, $-CH_2-$, $-CH_2CH_2-$, -C(O)- , $-CH_2O-$, $-CH_2O-CH_2CH_2O-$ or - $NHC(O)-(CH_2NHC(O))_a-$;

$L_2$ is a chemical bond or $-CH_2CH_2C(O)-$;

$L_3$ is a chemical bond, $-(NHCH_2CH_2)_b-$, $-(NHCH_2CH_2CH_2)_b-$ or $-C(O)CH_2-$;

$L_4$ is $-(OCH_2CH_2)_c-$, $-(OCH_2CH_2CH_2)_c-$, $-(OCH_2CH_2CH_2CH_2)_c-$, $-(OCH_2CH_2CH_2CH_2CH_2)_c-$ or $-NHC(O)-(CH_2)_d-$;

wherein a = 0, 1, 2, or 3;

b = 1, 2, 3, 4 or 5;

c = 1, 2, 3, 4 or 5;

d = 1, 2, 3, 4, 5, 6, 7, or 8;

L is $-CH_2O-$ or -NHC(O)-;

L 'is a chemical bond, -C(O)NH- or -NHC(O)-;

$R_1$ and $R_2$ together form $-CH_2CH_2O-$ or $-CH_2CH(R)-O-$, and $R_3$ is H;

or R1 and R3 together form-C1-2 alkylene-, and R2 is H;

wherein R is -OR', $-CH_2OR'$ or $-CH_2CH_2OR'$, wherein R' is H, a hydroxyl protecting group, or a solid support, wherein the hydroxyl protecting group is preferably $-C(O)CH_2CH_2C(O)OH$ or 4,4'-dimethoxytrityl;

m = 0, 1, 2, 3, 4, 5, 6, 7, 8, 9 or 10;

n = 0, 1, 2, 3, 4, 5, 6, 7, 8, 9, or 10.

In some specific embodiments, wherein,

Q is independently H or

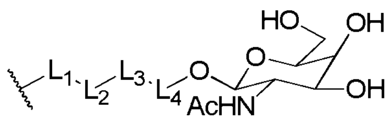
;

wherein $L_1$ is $-CH_2O-$ or $-NHC(O)-(CH_2NHC(O))_a-$;
$L_2$ is $-CH_2CH_2C(O)-$;
$L_3$ is $-(NHCH_2CH_2)_b-$ or $-(NHCH_2CH_2CH_2)_b-$;
$L_4$ is $-(OCH_2CH_2)_c-$ or $-NHC(O)-(CH_2)_d-$;
wherein a = 0, 1, 2, or 3;
b = 1, 2, 3, 4 or 5;
c = 1, 2, 3, 4 or 5;
d = 1, 2, 3, 4, 5, 6, 7, or 8;
L is $-CH_2O-$;
L' is a chemical bond;
$R_1$ and $R_2$ together form $-CH_2CH_2O-$ or $-CH_2CH(R)-O-$, and $R_3$ is H;
or $R_1$ and $R_3$ together form $-C_{1-2}$ alkylene-, and $R_2$ is H;
wherein R is -OR', $-CH_2OR'$ or $-CH_2CH_2OR'$, wherein R' is H, a hydroxyl protecting group, or a solid support, wherein the hydroxyl protecting group is preferably $-C(O)CH_2CH_2C(O)OH$ or 4,4' -dimethoxytrityl;
m = 0, 1, 2, 3, 4, 5, 6, 7, 8, 9 or 10;
n = 0, 1, 2, 3, 4, 5, 6, 7, 8, 9, or 10.

In some embodiments, the conjugating group is represented by Formula (I'-1), Formula (I'-2), or Formula (I'-3):

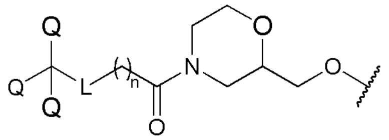
(I'-1),

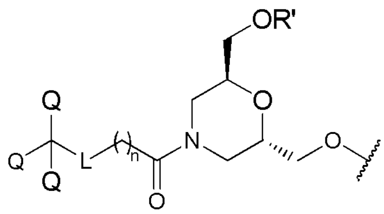
(I'-2)

or

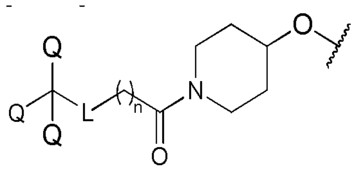
(I'-3)

wherein

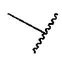

represents the point of attachment to the dsRNA;

Q is

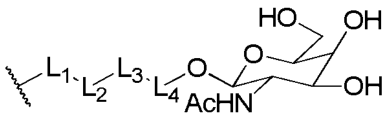;

wherein $L_1$ is $-CH_2O-$ or $-NHC(O)-$;
$L_2$ is $-CH_2CH_2C(O)-$;
$L_3$ is $-(NHCH_2CH_2)_b-$ or $-(NHCH_2CH_2CH_2)_b-$;
$L_4$ is $-(OCH_2CH_2)_c-$ or $-NHC(O)-(CH_2)_d-$;
wherein b = 1, 2, 3, 4 or 5;
c = 1, 2, 3, 4 or 5;
d = 1, 2, 3, 4, 5, 6, 7, or 8;
L is $-CH_2O-$;
R' is H, a hydroxyl protecting group, or a solid support, preferably the hydroxyl protecting group is $-C(O)CH_2CH_2C(O)OH$ or 4,4'-dimethoxytrityl;
n = 0, 1, 2, 3, 4, 5, 6, 7, 8, 9, or 10.

In some specific embodiments, wherein,

Q is independently H,

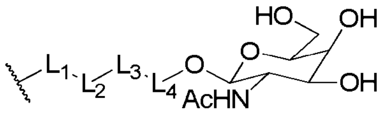,

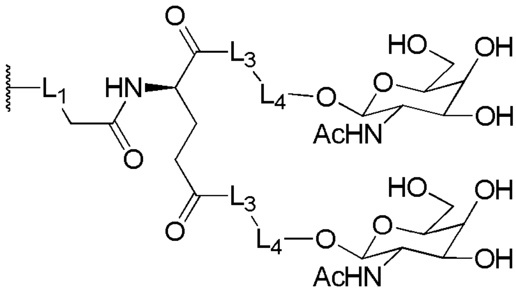,

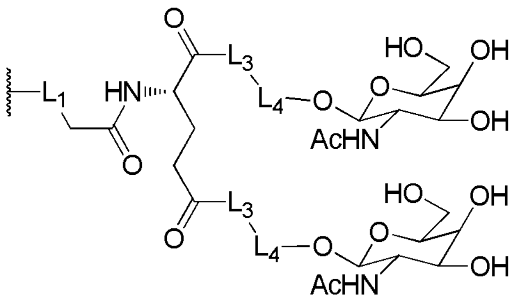,

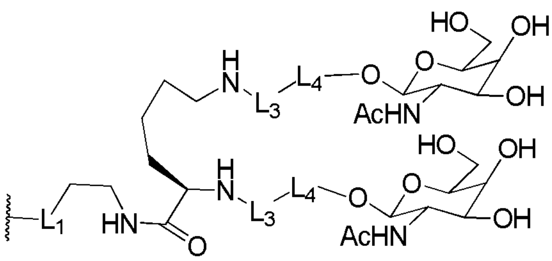

or

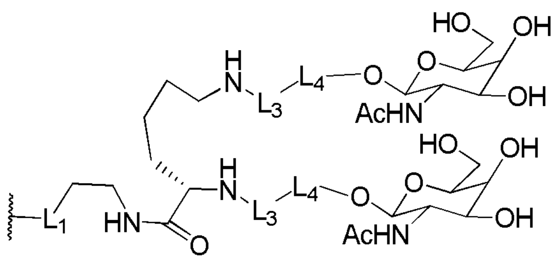;

wherein $L_1$ is $-CH_2O-$, $-CH_2O-CH_2CH_2O-$ or $-NHC(O)-(CH_2NHC(O))_a-$;
$L_3$ is $-(NHCH_2CH_2)_b-$, $-(NHCH_2CH_2CH_2)_b-$ or $-C(O)CH_2-$;

$L_4$ is $-(OCH_2CH_2)_c-$ or $-NHC(O)-(CH_2)_d-$;
wherein a = 0, 1, 2, or 3;
b = 1, 2, 3, 4 or 5;
c = 1, 2, 3, 4 or 5;
d = 1, 2, 3, 4, 5, 6, 7, or 8;
L is $-CH_2O-$ or -NHC(O)-;
L' is a chemical bond or -C(O)NH-;
$R_1$ and $R_2$ together form $-CH_2CH_2O-$ or $-CH_2CH(R)-O-$, and $R_3$ is H;
or $R_1$ and $R_3$ together form $-C_{1-2}$ alkylene-, and $R_2$ is H;
wherein R is -OR', $-CH_2OR'$ or $-CH_2CH_2OR'$, wherein R' is H, a hydroxyl protecting group, or a solid support, preferably the hydroxyl protecting group is $-C(O)CH_2CH_2C(O)OH$ or 4,4'-dimethoxytrityl;
m = 0, 1, 2, 3, 4, 5, 6, 7, 8, 9 or 10;
n = 0, 1, 2, 3, 4, 5, 6, 7, 8, 9, or 10.

In some embodiments, the conjugating group is represented by Formula (II'-1) or Formula (II'-2):

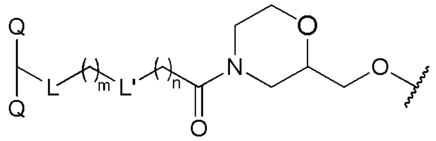
(II'-1) or
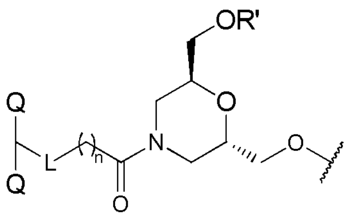
(II'-2)

wherein

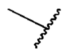

represents the point of point of attachmentto the dsRNA;

Q is independently

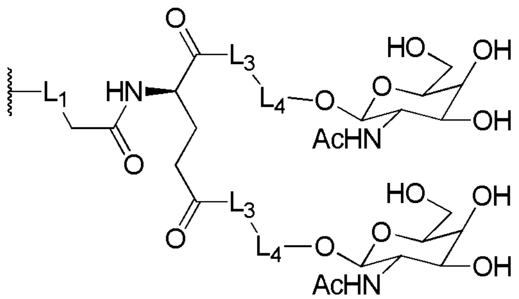
,
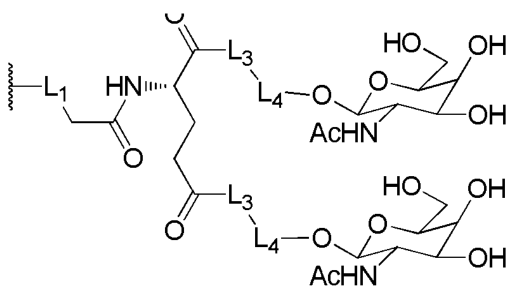
,
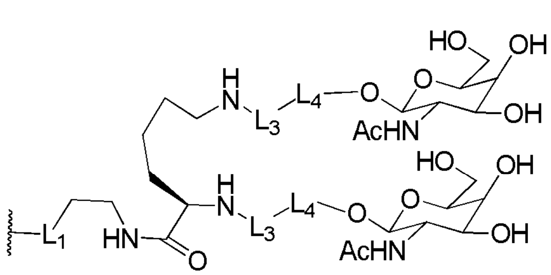

or

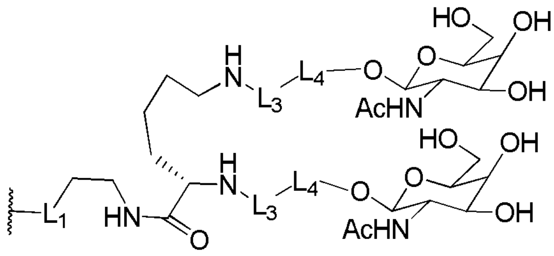

;

wherein $L_1$ is $-CH_2O-$ or $-CH_2O-CH_2CH_2O-$;

$L_3$ is $-(NHCH_2CH_2)_b-$, $-(NHCH_2CH_2CH_2)_b-$ or $-C(O)CH_2-$;

$L_4$ is $-(OCH_2CH_2)_c-$ or $-NHC(O)-(CH_2)_d-$;

wherein b = 1, 2, 3, 4 or 5;

c = 1, 2, 3, 4 or 5;

d = 1, 2, 3, 4, 5, 6, 7, or 8;

L is -NHC(O)-;

L' is a chemical bond or -C(O)NH-;

R' is H, a hydroxyl protecting group, or a solid support, preferably the hydroxyl protecting group is $-C(O)CH_2CH_2C(O)OH$ or 4,4'-dimethoxytrityl;

m = 0, 1, 2, 3, 4, 5, 6, 7, 8, 9 or 10;

n = 0, 1, 2, 3, 4, 5, 6, 7, 8, 9, or 10.

In some specific embodiments, wherein,

Q is independently H,

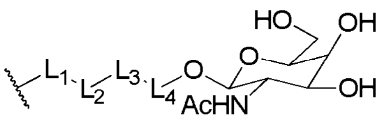

,

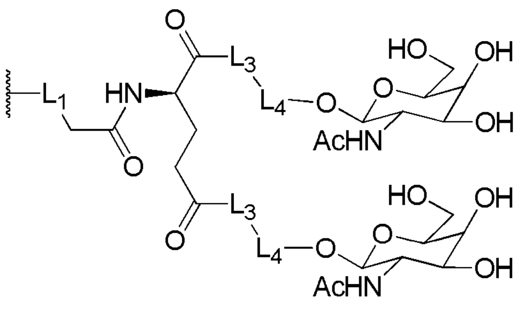

,

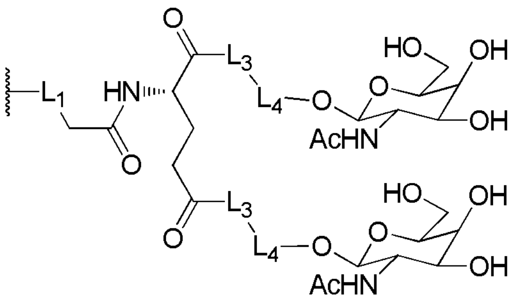

,

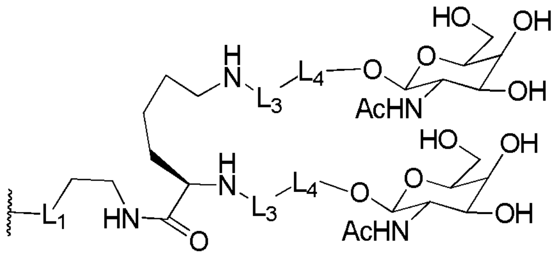

or

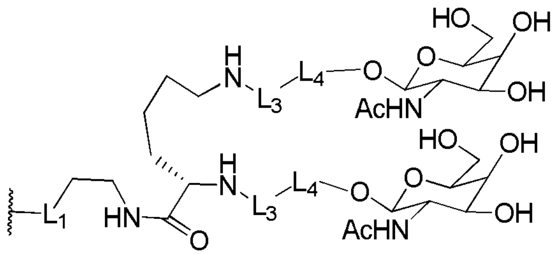

;

wherein $L_1$ is $-CH_2-$, $-C(O)-$, $-CH_2O-$, $-CH_2O-CH_2CH_2O-$ or $-NHC(O)-(CH_2NHC(O))_a-$;
$L_2$ is a chemical bond;
$L_3$ is $-(NHCH_2CH_2)_b-$, $-(NHCH_2CH_2CH_2)_b-$ or $-C(O)CH_2-$;
$L_4$ is $-(OCH_2CH_2)_c-$ or $-NHC(O)-(CH_2)_d-$;
wherein a = 0, 1, 2, or 3;
b = 1, 2, 3, 4 or 5;
c = 1, 2, 3, 4 or 5;
d = 1, 2, 3, 4, 5, 6, 7, or 8;
L is $-CH_2O-$ or $-NHC(O)-$;
L' is a chemical bond or $-C(O)NH-$;
$R_1$ and $R_2$ together form $-CH_2CH_2O-$ or $-CH_2CH(R)-O-$, and $R_3$ is H;
or $R_1$ and $R_3$ together form $-C_{1-2}$ alkylene-, and $R_2$ is H;
wherein R is -OR', $-CH_2OR'$ or $-CH_2CH_2OR'$, wherein R' is H, a hydroxyl protecting group, or a solid support, preferably the hydroxyl protecting group is $-C(O)CH_2CH_2C(O)OH$ or 4,4'-dimethoxytrityl, ;
m = 0, 1, 2, 3, 4, 5, 6, 7, 8, 9 or 10;
n = 0, 1, 2, 3, 4, 5, 6, 7, 8, 9, or 10.

In some embodiments, wherein the conjugating group is as represented by Formula (II'-2):

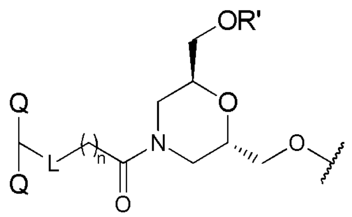

(II'-2)

wherein

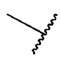

represents the point of attachement to the dsRNA;
Q is independently

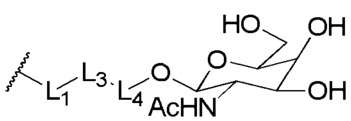

,

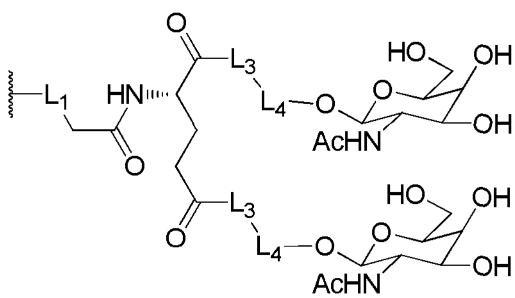

or

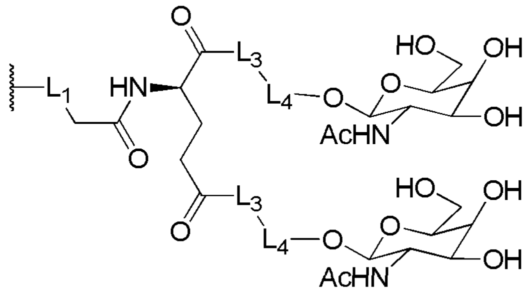
;

wherein $L_1$ is $-CH_2-$ or$-C(O)-$;
$L_3$ is $-(NHCH_2CH_2)_b-$;
$L_4$ is $-(OCH_2CH_2)_c-$;
wherein b = 1, 2, 3, 4 or 5;
c = 1, 2, 3, 4 or 5;
L is $-CH_2O-$ or $-NHC(O)-$;
R' is H, a hydroxyl protecting group, or a solid support, preferably the hydroxyl protecting group is $-C(O)CH_2CH_2C(O)OH$ or 4,4'-dimethoxytrityl, ;
n = 0, 1, 2, 3, 4, 5, 6, 7, 8, 9, or 10.

In some specific embodiments, wherein:

Q is independently H,

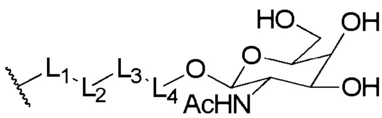
,
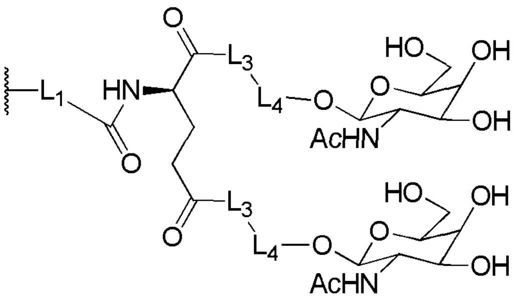
,

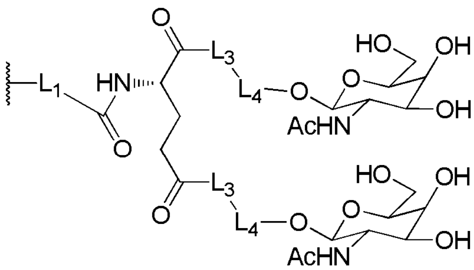
,
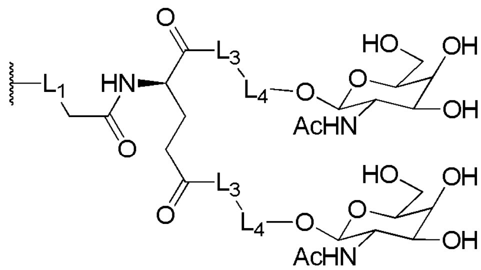
,

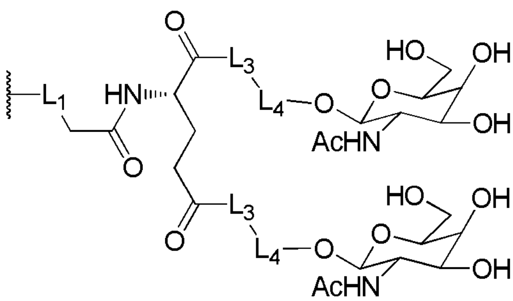
,
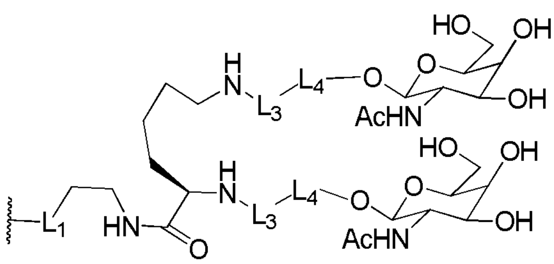

or

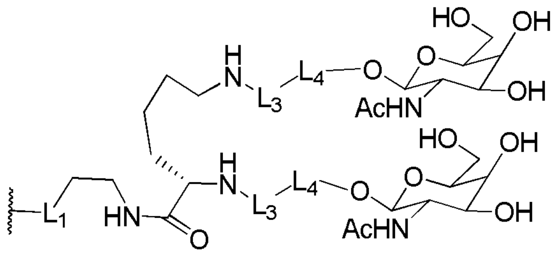

;

wherein $L_1$ is a chemical bond, $-CH_2-$, $-CH_2CH_2-$, -C(O)-, $-CH_2O-$, $-CH_2O-CH_2CH_2O-$ or - NHC(O)-$(CH2NHC(O))_a$-;

$L_2$ is a chemical bond or $-CH_2CH_2C(O)-$;

$L_3$ is a chemical bond, $-(NHCH_2CH_2)_b-$, $-(NHCH_2CH_2CH_2)_b-$ or $-C(O)CH_2-$;

L4 is $-(OCH_2CH_2)_c-$, $-(OCH_2CH_2CH_2)_c-$, $-(OCH_2CH_2CH_2CH_2)_c-$, $-(OCH_2CH_2CH_2CH_2CH_2)_c-$ or $-NHC(O)-(CH_2)_d-$;

wherein a = 0, 1, 2, or 3;

b = 1, 2, 3, 4 or 5;

c = 1, 2, 3, 4 or 5;

d = 1, 2, 3, 4, 5, 6, 7, or 8;

L is a chemical bond, $-CH_2O-$ or -NHC(O)-;

L' is a chemical bond, -C(O)NH-, -NHC(O)- or $-O(CH_2CH_2O)_e-$;

wherein e is 1, 2, 3, 4 or 5;

T is a chemical bond, $-CH_2-$, -M-, $-CH_2-M-$ or -C(O)-M-;

wherein M is

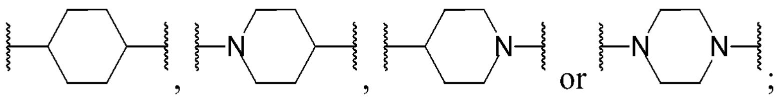

R₁ and R₂

$R_1$ and $R_2$ together form $-CH_2CH_2O-$ or $-CH_2CH(R)-O-$, and $R_3$ is H;

or $R_1$ and $R_3$ together form $-C_{1-2}$ alkylene-, and $R_2$ is H;

wherein R is -OR', $-CH_2OR'$ or $-CH_2CH_2OR'$, wherein R' is H, a hydroxyl protecting group, or a solid support, preferably the hydroxyl protecting group is $-C(O)CH_2CH_2C(O)OH$ or 4,4'-dimethoxytrityl;

m = 0, 1, 2, 3, 4, 5, 6, 7, 8, 9 or 10;

n = 0, 1, 2, 3, 4, 5, 6, 7, 8, 9, or 10.

In some specific embodiments, wherein,
T is-M-, $-CH_2-M-$, or-C(O)-M-, wherein M is

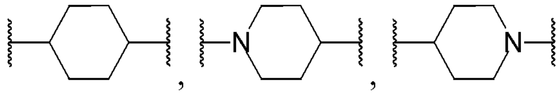

or

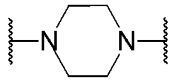.

In some specific embodiments, wherein,

Q is independently H or

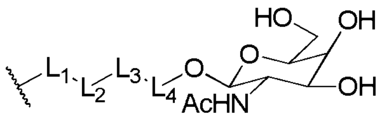;

wherein $L_1$ is $-CH_2O-$ or $-NHC(O)-(CH_2NHC(O))_a-$;
$L_2$ is $-CH_2CH_2C(O)-$;
$L_3$ is $-(NHCH_2CH_2)_b-$ or $-(NHCH_2CH_2CH_2)_b-$;
$L_4$ is $-(OCH_2CH_2)_c-$ or $-NHC(O)-(CH_2)_d-$;
wherein a = 0, 1, 2, or 3;
b = 1, 2, 3, 4 or 5;
c = 1, 2, 3, 4 or 5;
d = 1, 2, 3, 4, 5, 6, 7, or 8;
L is a chemical bond or $-CH_2O-$;
L' is a chemical bond or $-O(CH_2CH_2O)_e-$;
wherein e is 1, 2, 3, 4 or 5;
$R_1$ and $R_2$ together form $-CH_2CH_2O-$ or $-CH_2CH(R)-O-$, and $R_3$ is H;
or $R_1$ and $R_3$ together form $-C_{1-2}$ alkylene-, and $R_2$ is H;
wherein R is -OR', $-CH_2OR'$ or $-CH_2CH_2OR'$, wherein R' is H, a hydroxyl protecting group, or a solid support, preferably the hydroxyl protecting group is $-C(O)CH_2CH_2C(O)OH$ or 4,4'-dimethoxytrityl;
m = 0, 1, 2, 3, 4, 5, 6, 7, 8, 9 or 10;
n = 0, 1, 2, 3, 4, 5, 6, 7, 8, 9, or 10;
wherein T is as defined in the above embodiments.

In some embodiments, wherein the conjugating group is as represented by Formula (III'-1), Formula (III'-2), or Formula (III'-3):

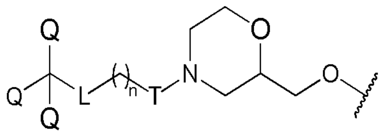
(III'-1),

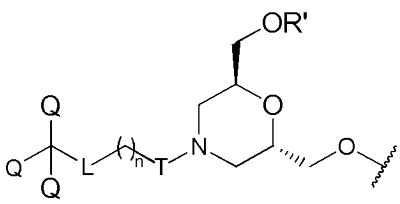
(III'-2)

or

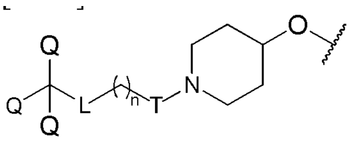
(III'-3)

wherein Q is

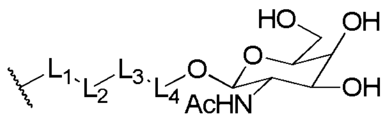;

wherein $L_1$ is $-CH_2O-$ or $-NHC(O)-$;

$L_2$ is $-CH_2CH_2C(O)-$;

$L_3$ is $-(NHCH_2CH_2)_b-$ or $-(NHCH_2CH_2CH_2)_b-$;

$L_4$ is $-(OCH_2CH_2)_c-$ or $-NHC(O)-(CH_2)_d-$;

wherein b = 1, 2, 3, 4 or 5;

c = 1, 2, 3, 4 or 5;

d = 1, 2, 3, 4, 5, 6, 7, or 8;

L is a chemical bond or $-CH_2O-$;

wherein R' is H, a hydroxyl protecting group or a solid support, preferably the hydroxyl protecting group is $-C(O)CH_2CH_2C(O)OH$ or 4,4'-dimethoxytrityl, ;

n = 0, 1, 2, 3, 4, 5, 6, 7, 8, 9, or 10;

wherein T is as defined in the above embodiments.

In some specific embodiments, wherein,

Q is independently H,

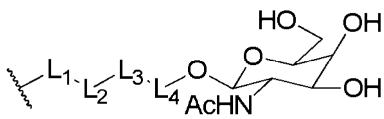,

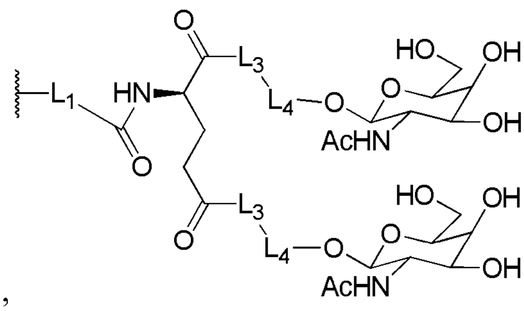,

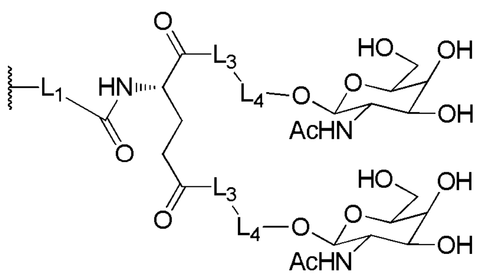,

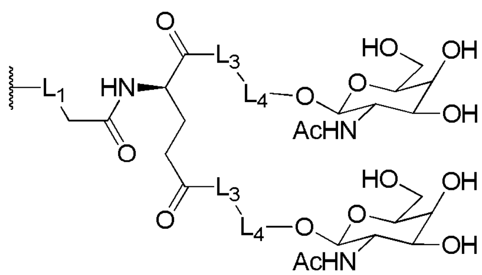,

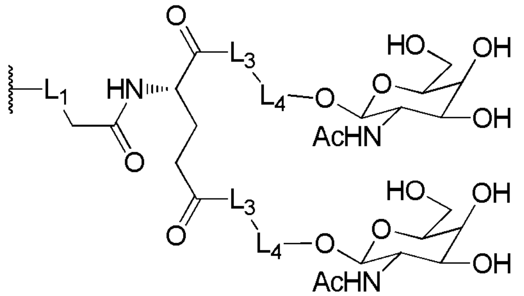,
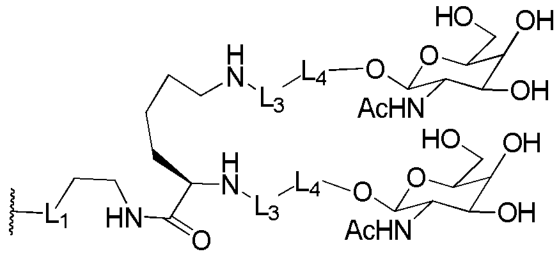

or

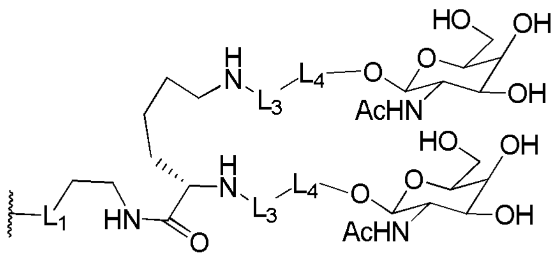;

wherein $L_1$ is $-CH_2-$, $-CH_2O-$ or -C(O)-;
$L_2$ is a chemical bond;
$L_3$ is $-(NHCH_2CH_2)_b-$, $-(NHCH_2CH_2CH_2)_b-$ or $-C(O)CH_2-$;
$L_4$ is $-(OCH_2CH_2)_c-$ or $-NHC(O)-(CH_2)_d-$;
wherein b = 1, 2, 3, 4 or 5;
c = 1, 2, 3, 4 or 5;
d = 1, 2, 3, 4, 5, 6, 7, or 8;
L is a chemical bond or -NHC(O)-;
L' is a chemical bond;
$R_1$ and $R_2$ together form $-CH_2CH_2O-$ or $-CH_2CH(R)-O-$, and $R_3$ is H;
or $R_1$ and $R_3$ together form $-C_{1-2}$ alkylene-, and $R_2$ is H;
wherein R is -OR', $-CH_2OR'$ or $-CH_2CH_2OR'$ or a solid support, wherein R' is H, a hydroxyl protecting group, preferably the hydroxyl protecting group is $-C(O)CH_2CH_2C(O)OH$ or 4,4'-dimethoxytrityl;
m = 0, 1, 2, 3, 4, 5, 6, 7, 8, 9 or 10;
n = 0, 1, 2, 3, 4, 5, 6, 7, 8, 9, or 10;
wherein T is as defined in the above embodiments.

In some embodiments, wherein the conjugating group is as shown in Formula (IV-1) or Formula (IV-2):

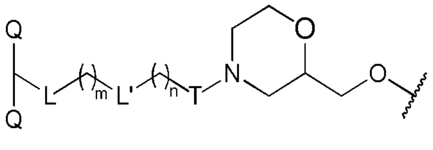 (IV-1) or 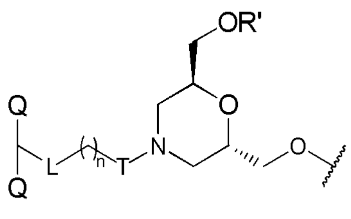 (IV-2)

wherein,

Q is independently

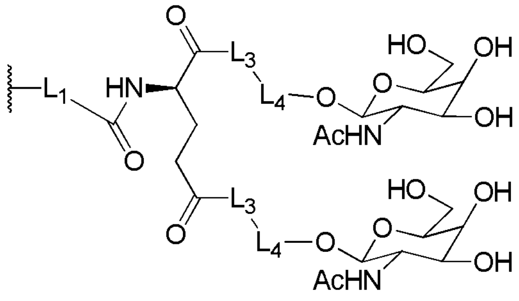

,

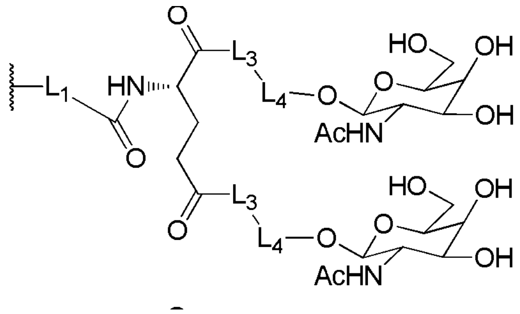

,

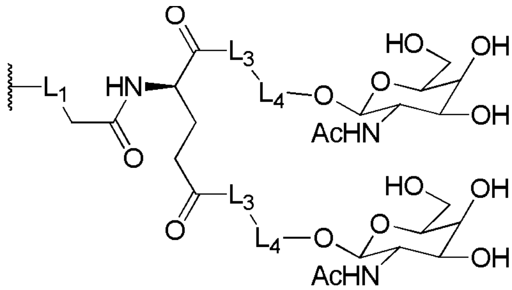

or

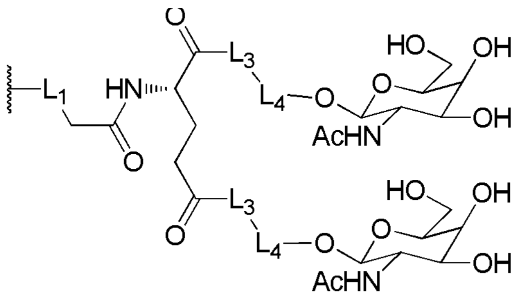

;

wherein $L_1$ is $-CH_2-$, $-CH_2O-$ or -C(O)-;

$L_3$ is $-(NHCH_2CH_2)_b-$, $-(NHCH_2CH_2CH_2)_b-$ or $-C(O)CH_2-$;

$L_4$ is $-(OCH_2CH_2)_c-$ or $-NHC(O)-(CH_2)_d-$;

wherein b = 1, 2, 3, 4 or 5;

c = 1, 2, 3, 4 or 5;

d = 1, 2, 3, 4, 5, 6, 7, or 8;

L is a chemical bond or -NHC(O)-;

L' is a chemical bond;

wherein R' is H, a hydroxyl protecting group, or a solid support, preferably the hydroxyl protecting group is -C(O) $CH_2CH_2C(O)OH$ or 4,4'-dimethoxytrityl;

m = 0, 1, 2, 3, 4, 5, 6, 7, 8, 9 or 10;

n = 0, 1, 2, 3, 4, 5, 6, 7, 8, 9, or 10;

wherein T is as defined in the above embodiments.

In some specific embodiments, wherein:

Q is independently H,

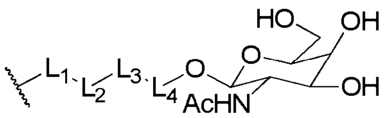,

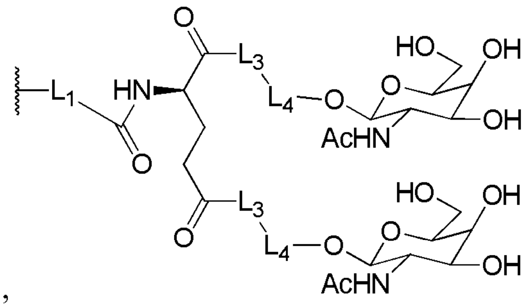,

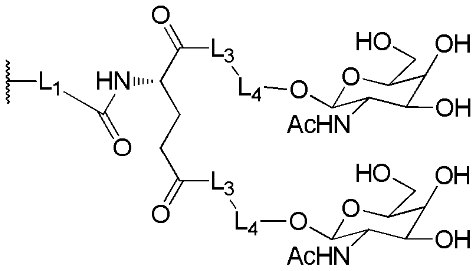,

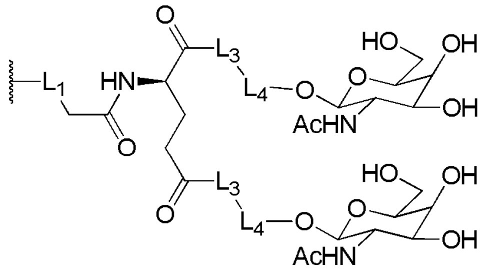,

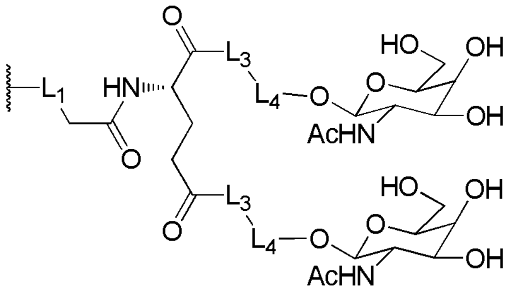,

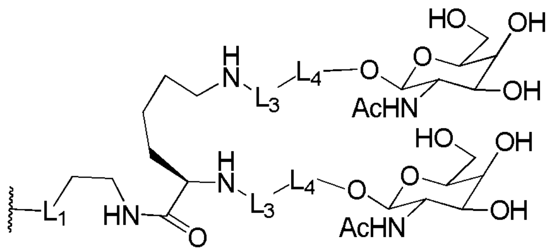

or

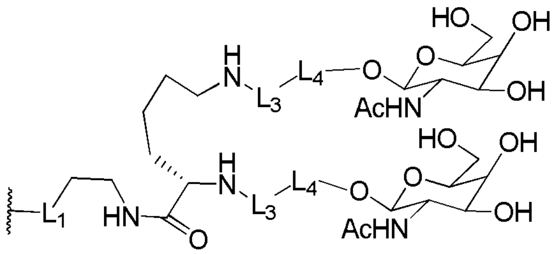;

wherein $L_1$ is a chemical bond, $-CH_2-$, $-CH_2CH_2-$, -C(O)-, $-CH_2O-$, $-CH_2O-CH_2CH_2O-$ or - NHC(O)-(CH2NHC(O))$_a$-;

$L_2$ is a chemical bond or $-CH_2CH_2C(O)-$;

$L_3$ is a chemical bond, $-(NHCH_2CH_2)_b-$, $-(NHCH_2CH_2CH_2)_b-$或$-C(O)CH_2-$;

$L_4$ is $-(OCH_2CH_2)_c-$, $-(OCH_2CH_2CH_2)_c-$, $-(OCH_2CH_2CH_2CH_2)_c-$, $-(OCH_2CH_2CH_2CH_2CH_2)_c-$ or $-NHC(O)-(CH_2)_d-$;

wherein a = 0, 1, 2, or 3;

b = 1, 2, 3, 4 or 5;

c = 1, 2, 3, 4 or 5;

d = 1, 2, 3, 4, 5, 6, 7, or 8;

L is a chemical bond, $-CH_2O-$ or -NHC(O)-;

L' is $-O(CH_2CH_2O)_e-$;

wherein e is 1, 2, 3, 4 or 5;

T is a chemical bond, $-CH_2-$, -C(O)-, -M-, $-CH_2-M-$ or -C(O)-M-;

wherein M is

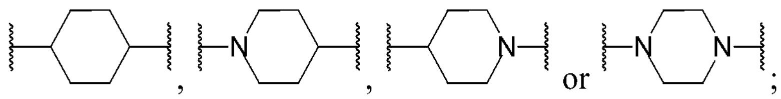

, , or ;

$R_1$ and $R_2$ together form $-CH_2CH_2O-$ or $-CH_2CH(R)-O-$, and $R_3$ is H;

or $R_1$ and $R_3$ together form $-C_{1-2}$ alkylene-, and $R_2$ is H;

wherein R is -OR', $-CH_2OR'$ or $-CH_2CH_2OR'$, wherein R' is H, a hydroxyl protecting group, or a solid support, preferably the hydroxyl protecting group is $-C(O)CH_2CH_2C(O)OH$ or 4,4'-dimethoxytrityl;

m = 0, 1, 2, 3, 4, 5, 6, 7, 8, 9 or 10;

n = 0, 1, 2, 3, 4, 5, 6, 7, 8, 9, or 10.

In some preferred embodiments, wherein the conjugating group is selected from Table 1 below:

| No. | Structure |
|---|---|
| 1 | 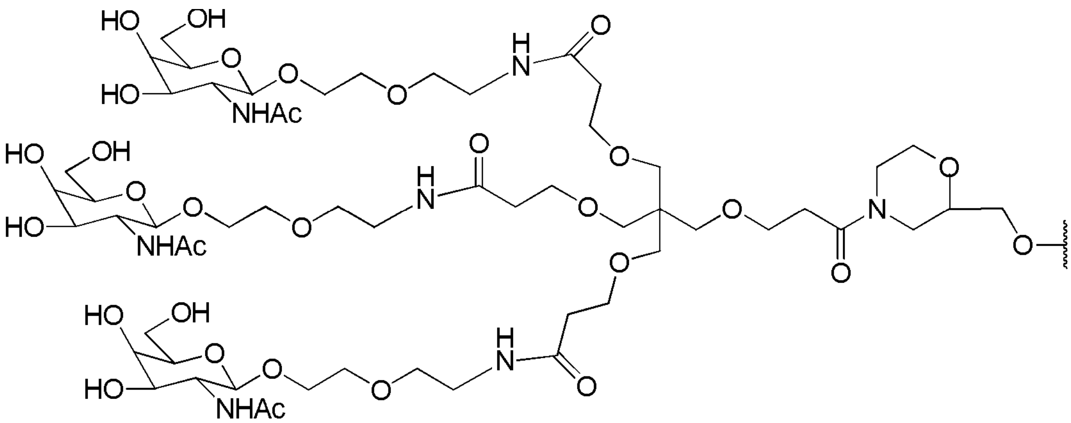 |

(continued)

| No. | Structure |
|---|---|
| 2 | 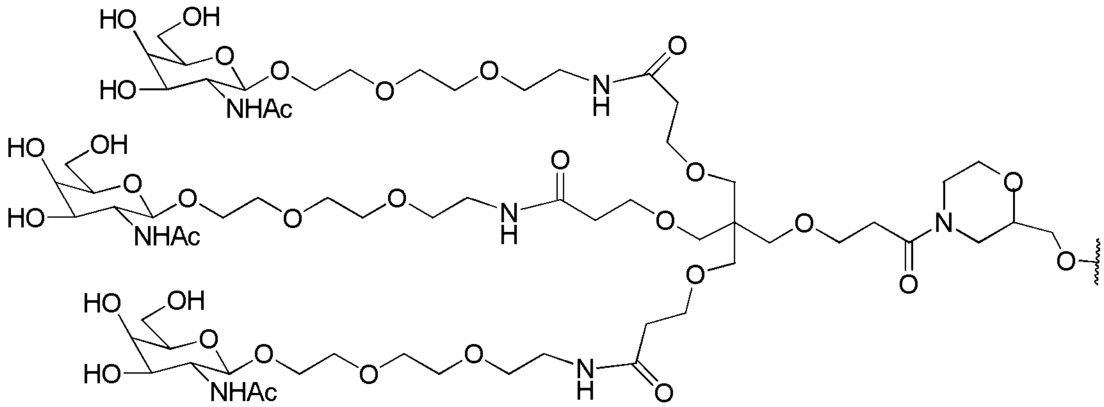 |
| 3 | 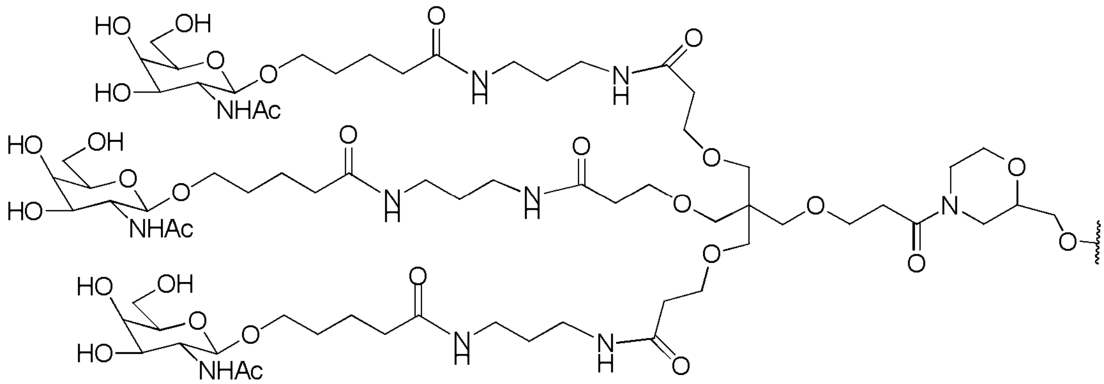 |
| 4 | 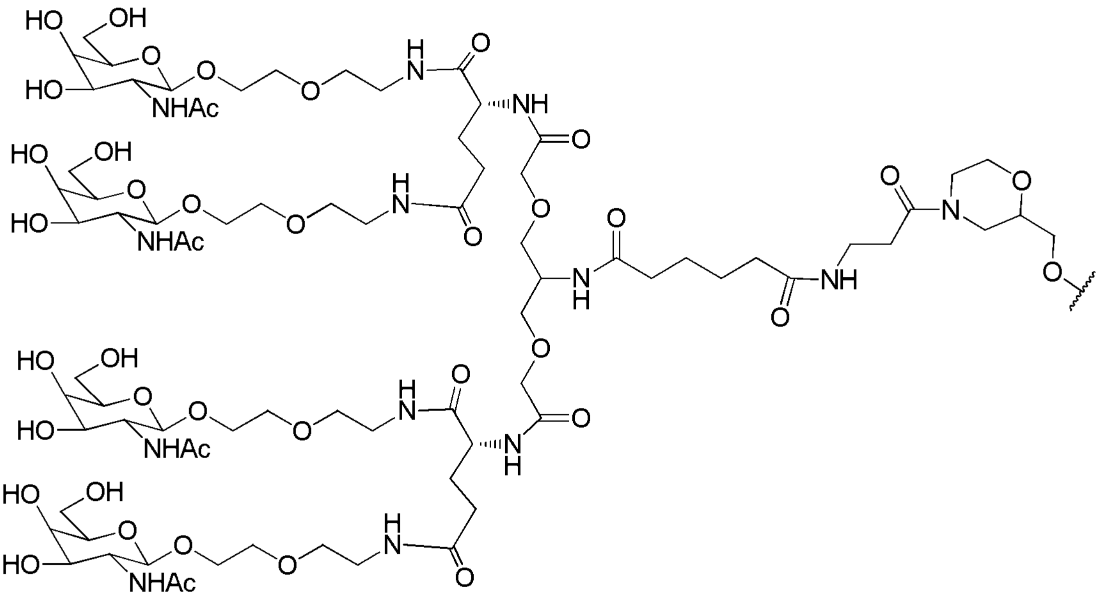 |

(continued)

| No. | Structure |
|---|---|
| 5 | 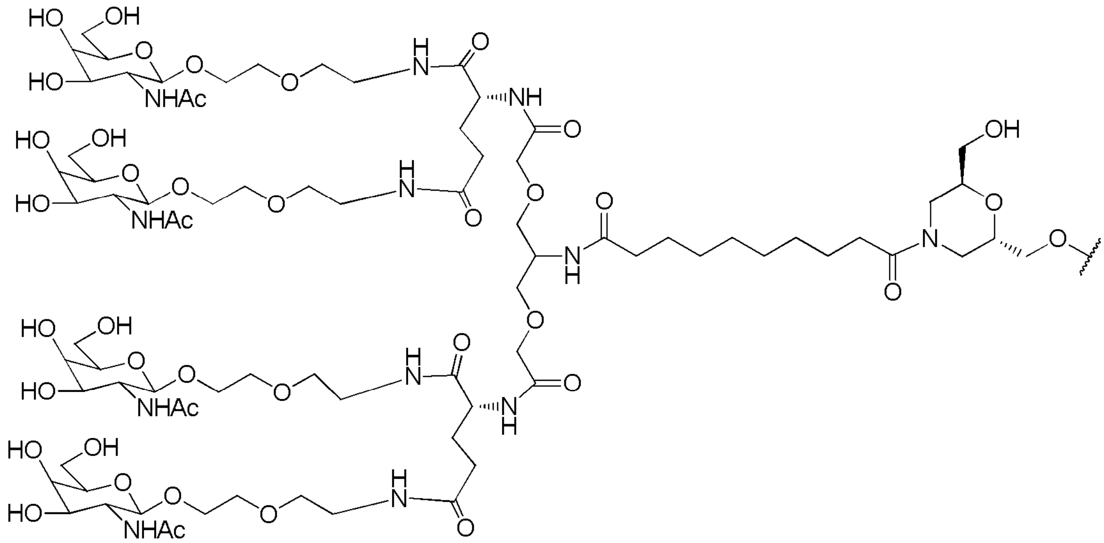 |
| 6 | 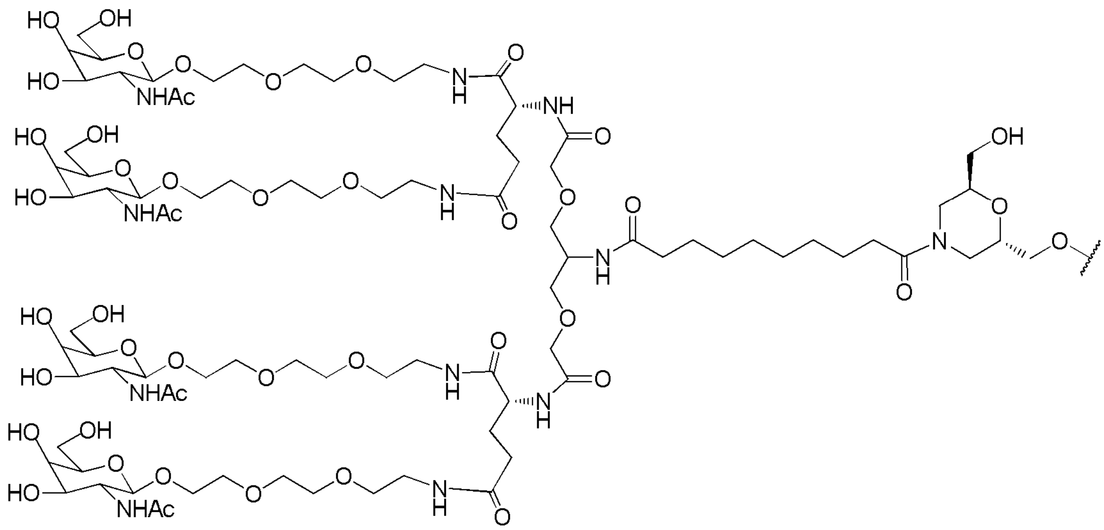 |
| 7 | 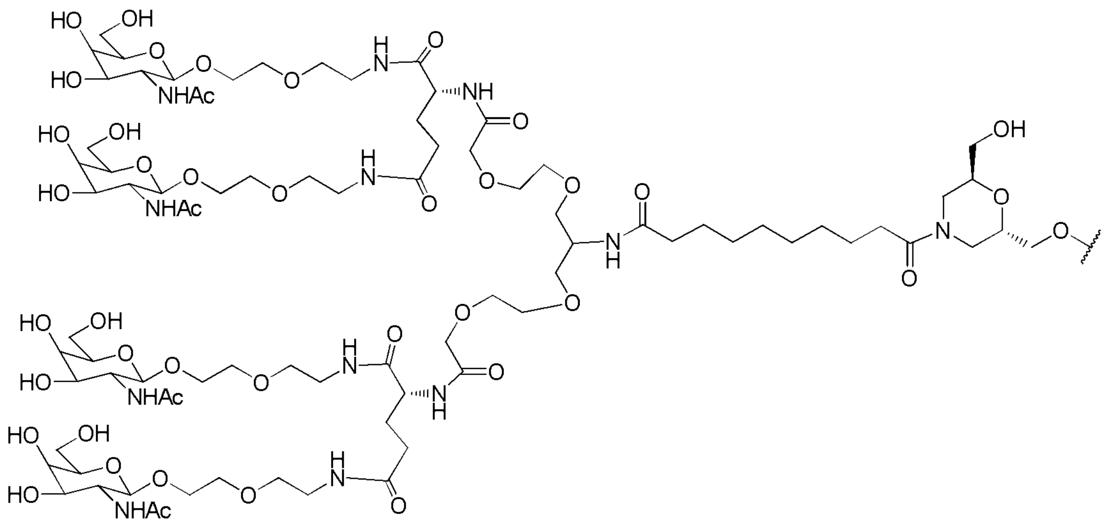 |

(continued)

| No. | Structure |
|---|---|
| 8 | 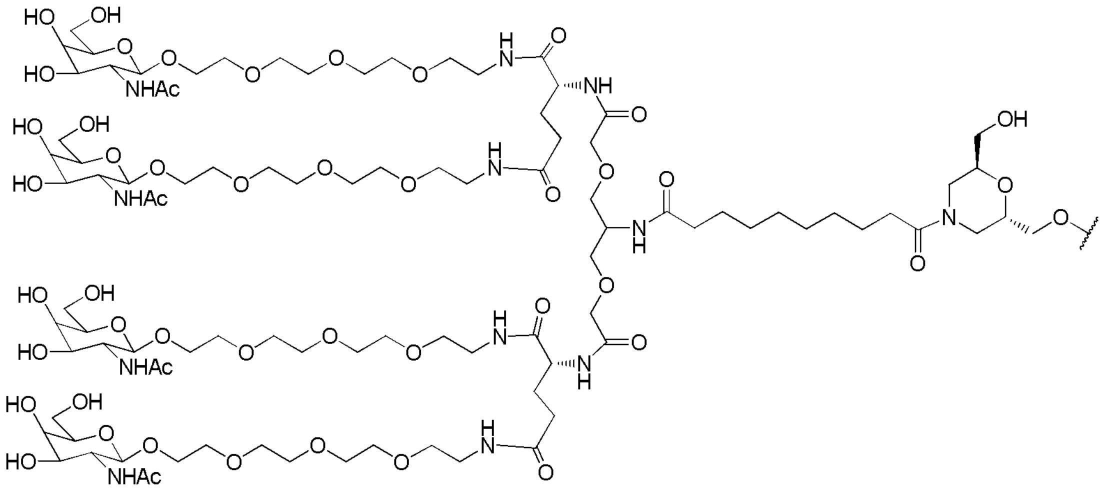 |
| 9 | 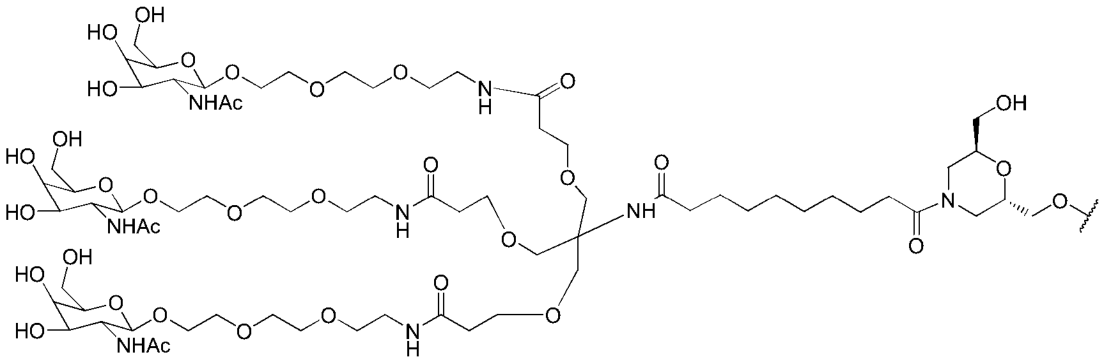 |
| 10 | 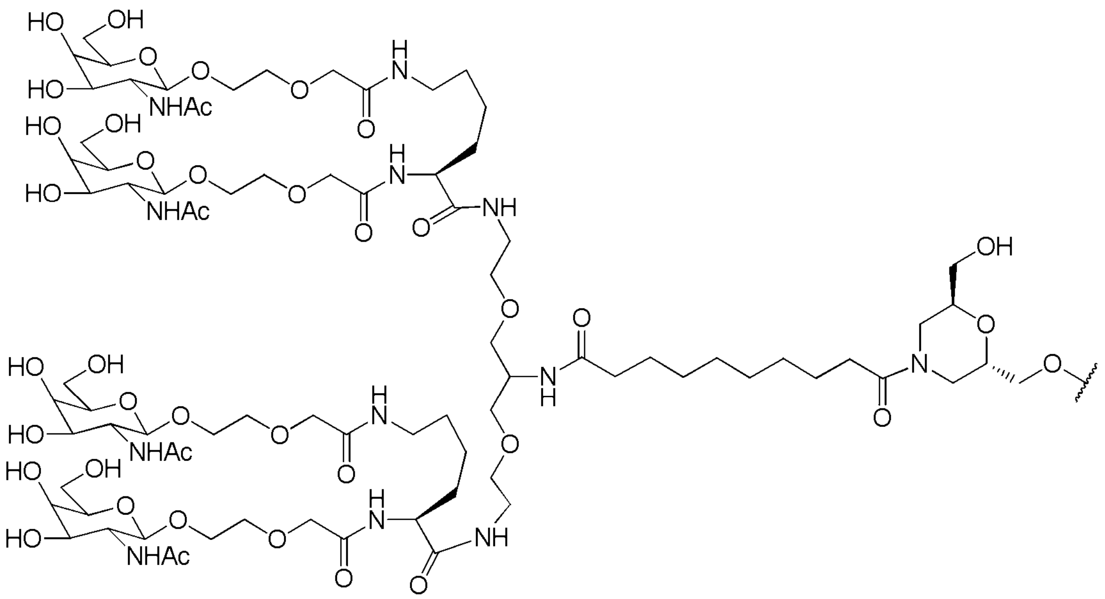 |

(continued)

| No. | Structure |
|---|---|
| 11 | 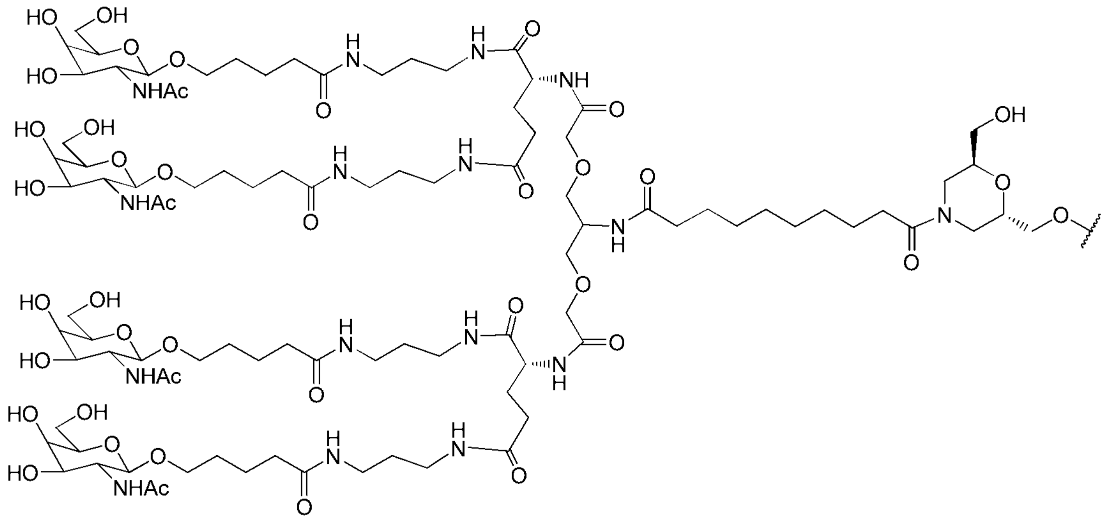 |
| 12 | 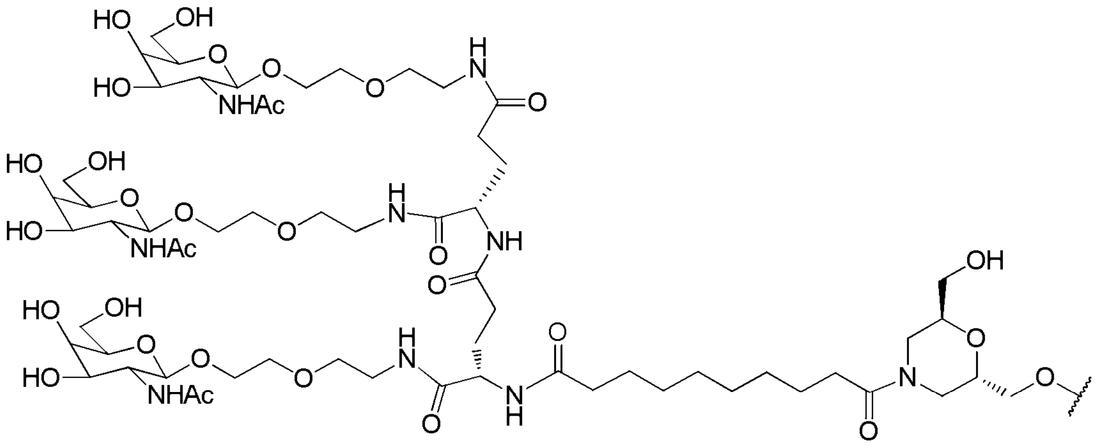 |
| 13 | 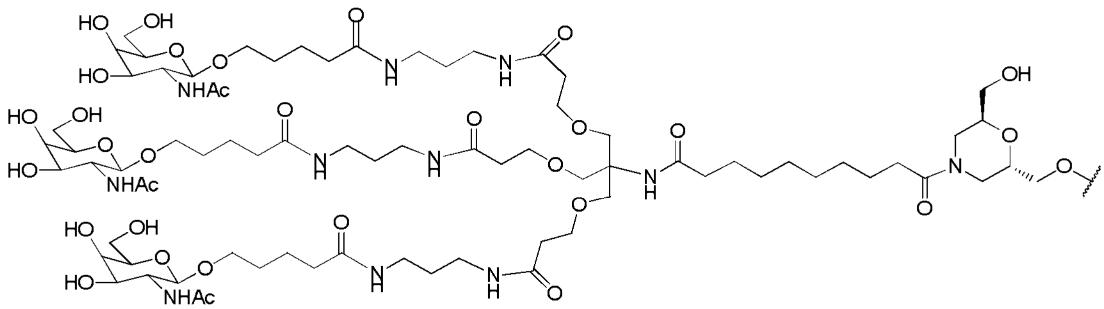 |

(continued)

| No. | Structure |
|---|---|
| 14 | 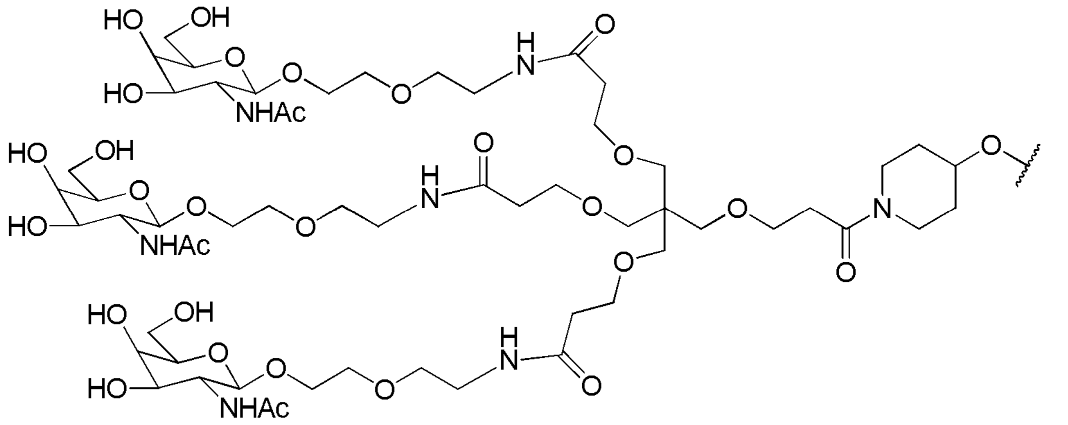 |
| 15 | 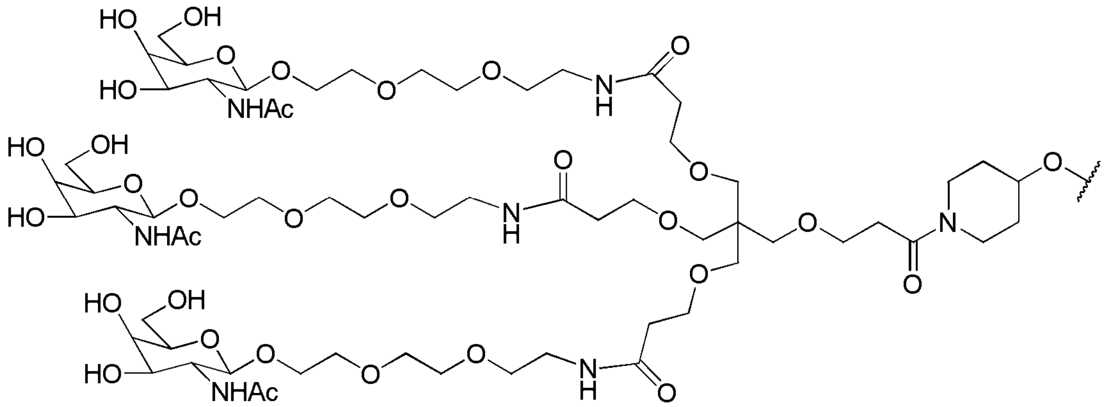 |
| 16 | 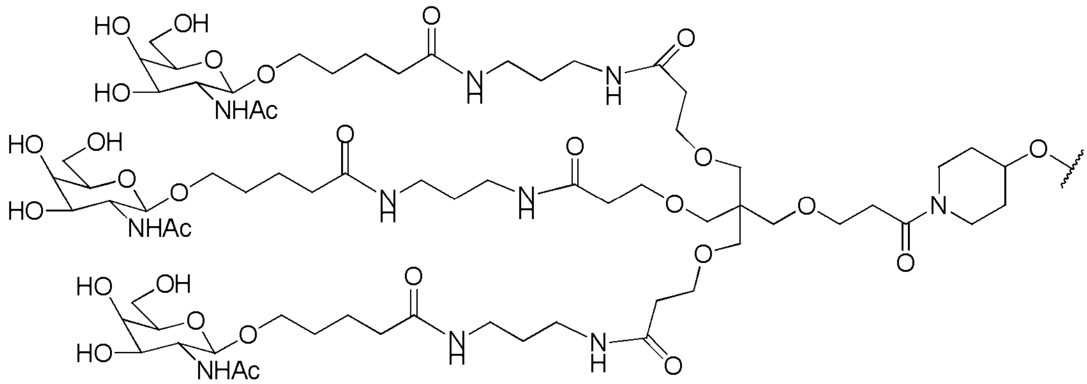 |

In some preferred embodiments, wherein the conjugating group is selected from Table 2 below:

| NO. | Structure |
|---|---|
| 17 | 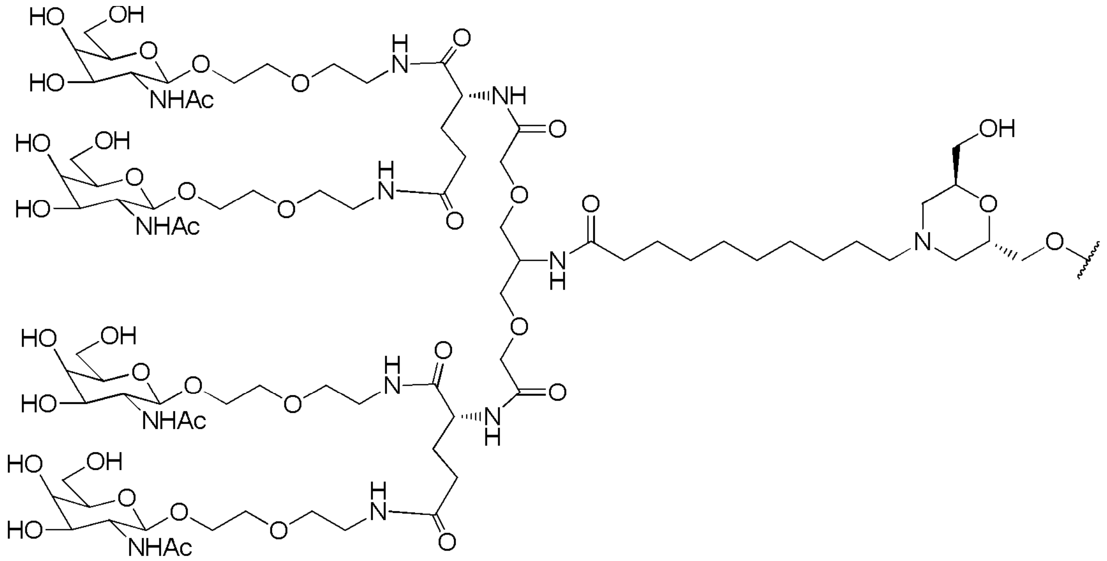 |
| 18 | 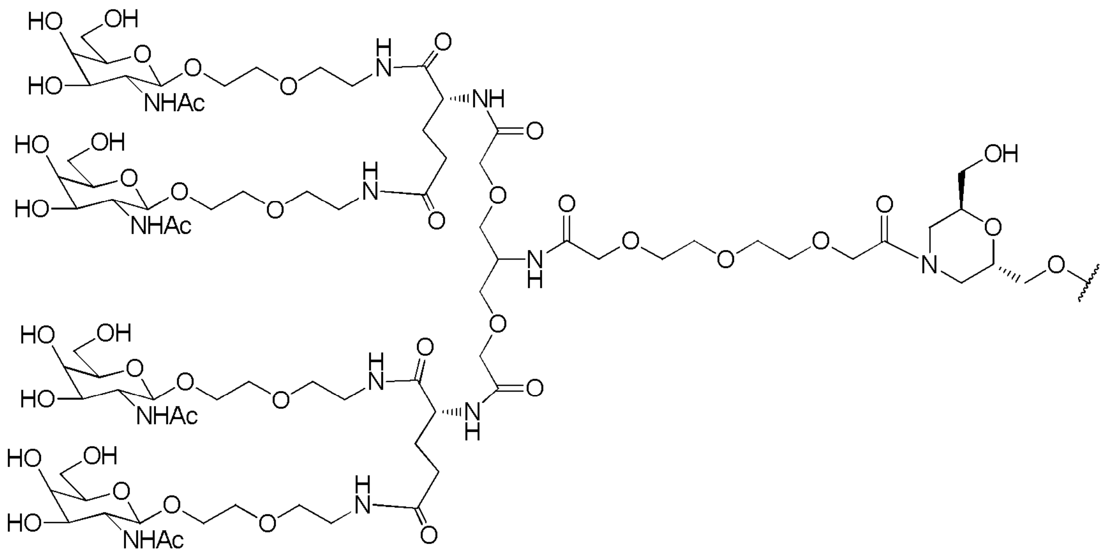 |
| 19 | 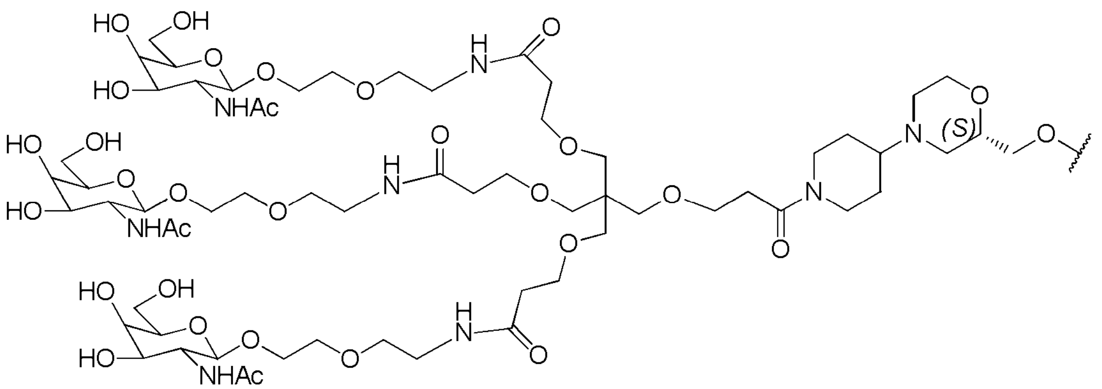 |

(continued)

| NO. | Structure |
|---|---|
| 20 | 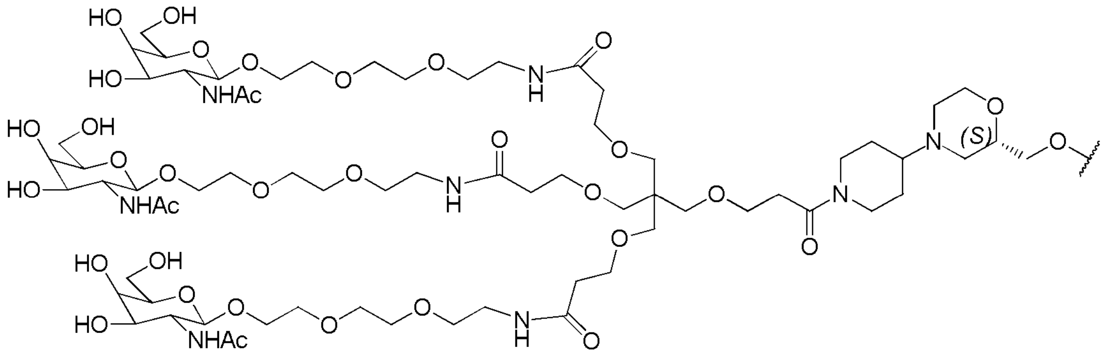 |
| 21 | 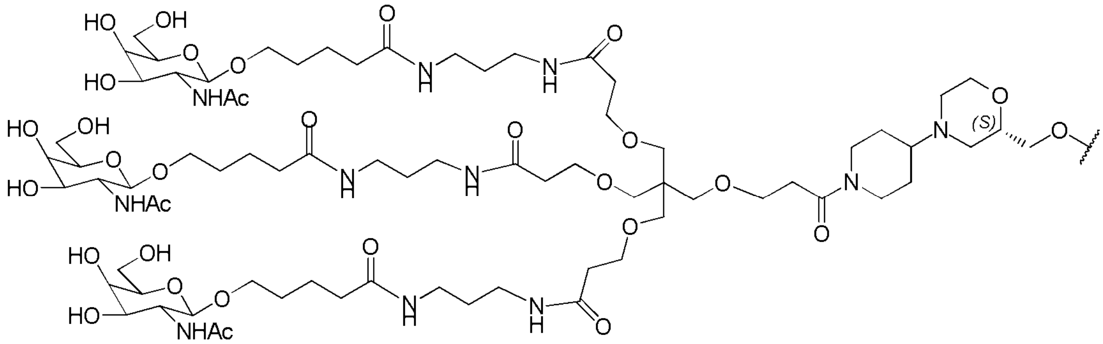 |
| 22 | 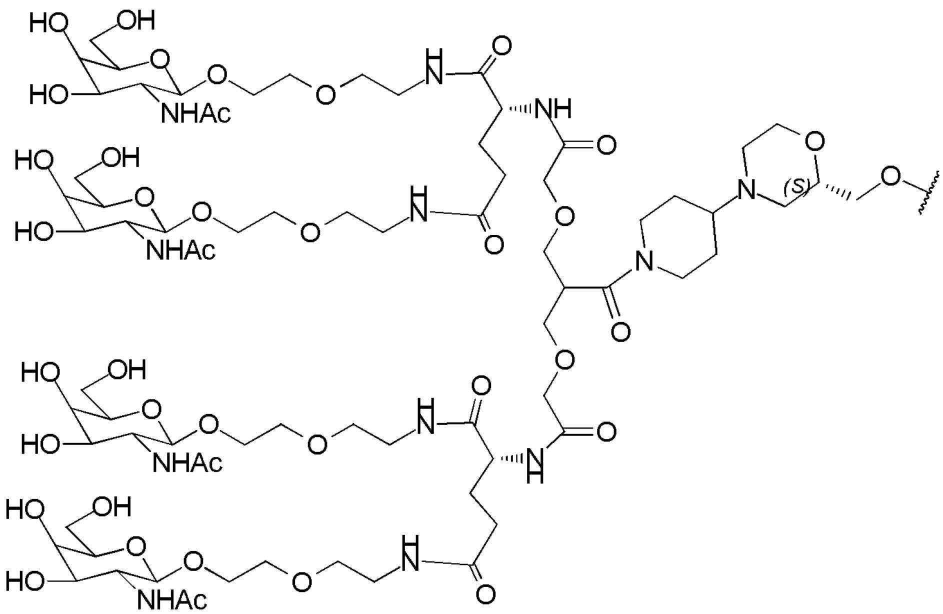 |

(continued)

| NO. | Structure |
|---|---|
| 23-1 | 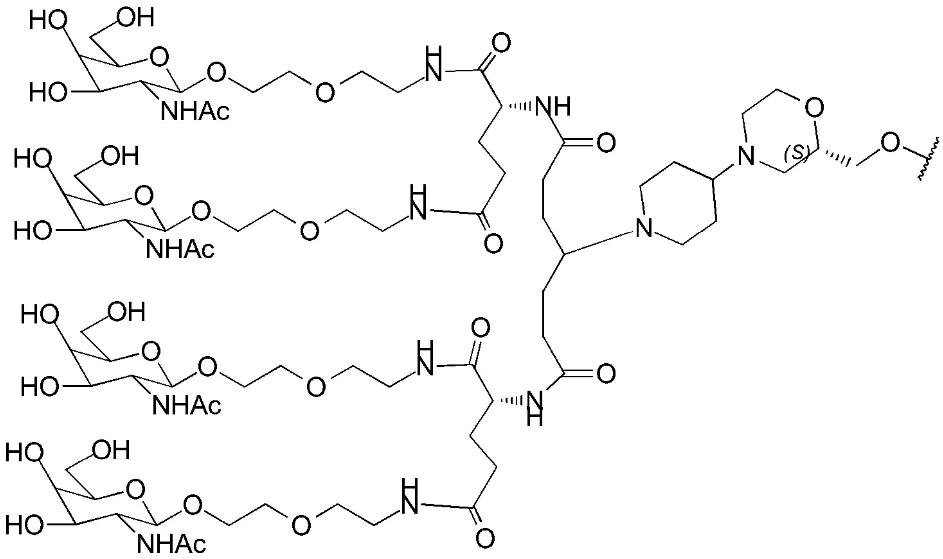 |
| 23-2 | 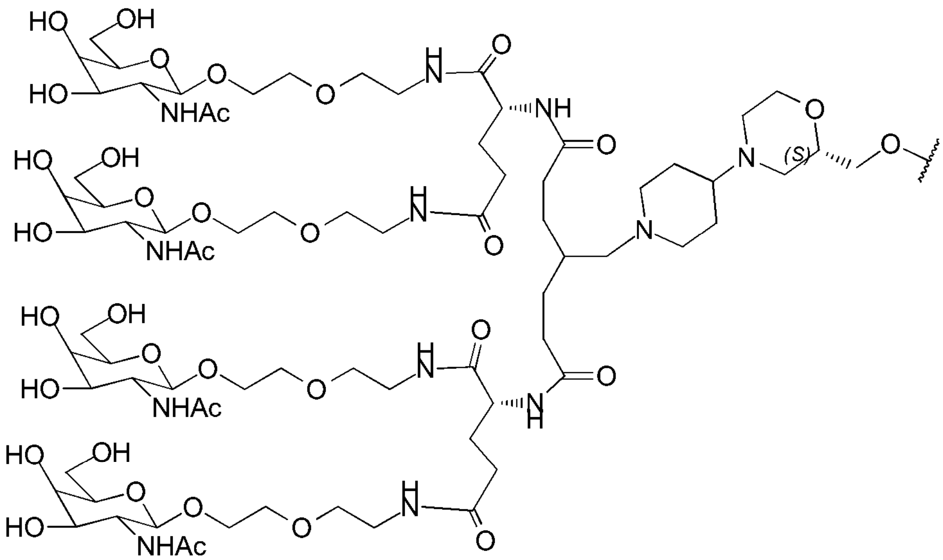 |

(continued)

| NO. | Structure |
| --- | --- |
| 24 | 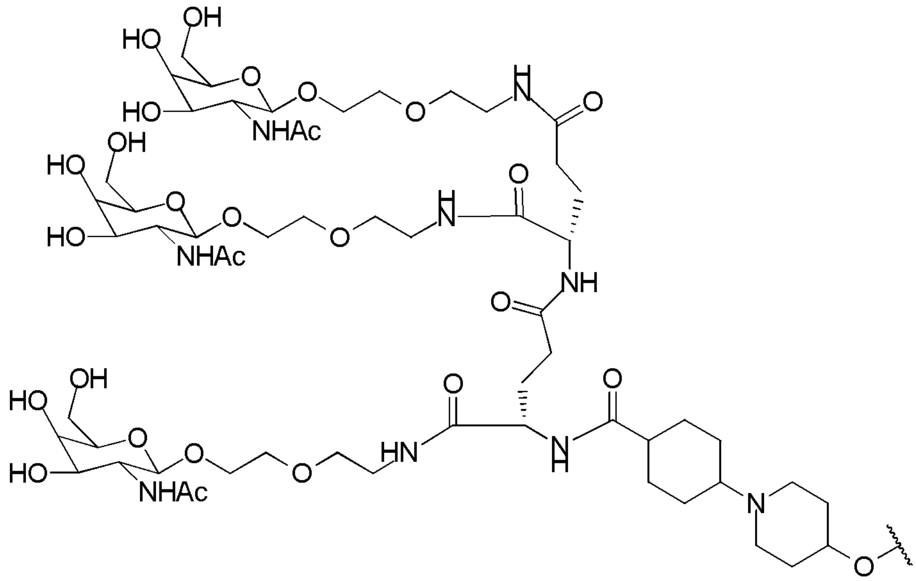 |
| 25 | 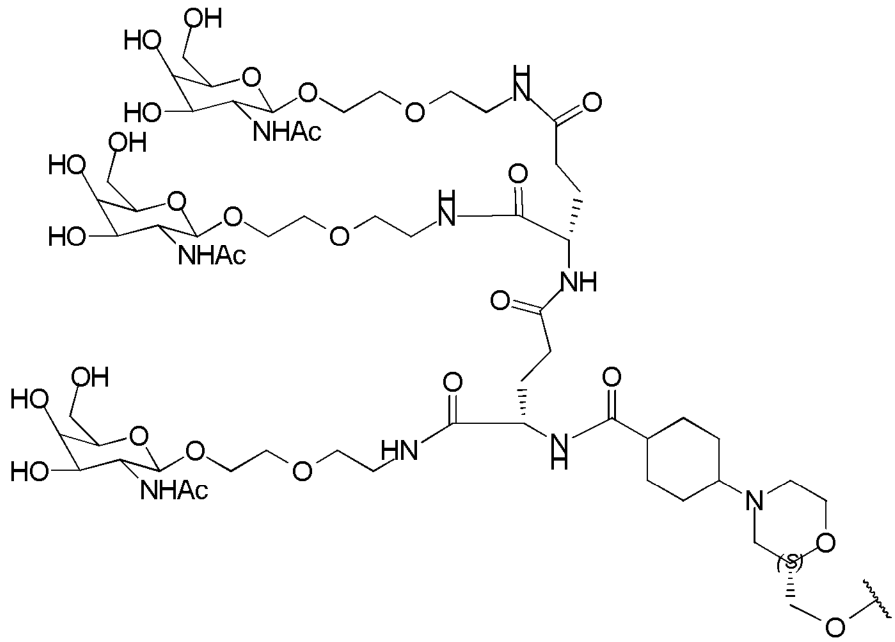 |

(continued)

| NO. | Structure |
|---|---|
| 26 | 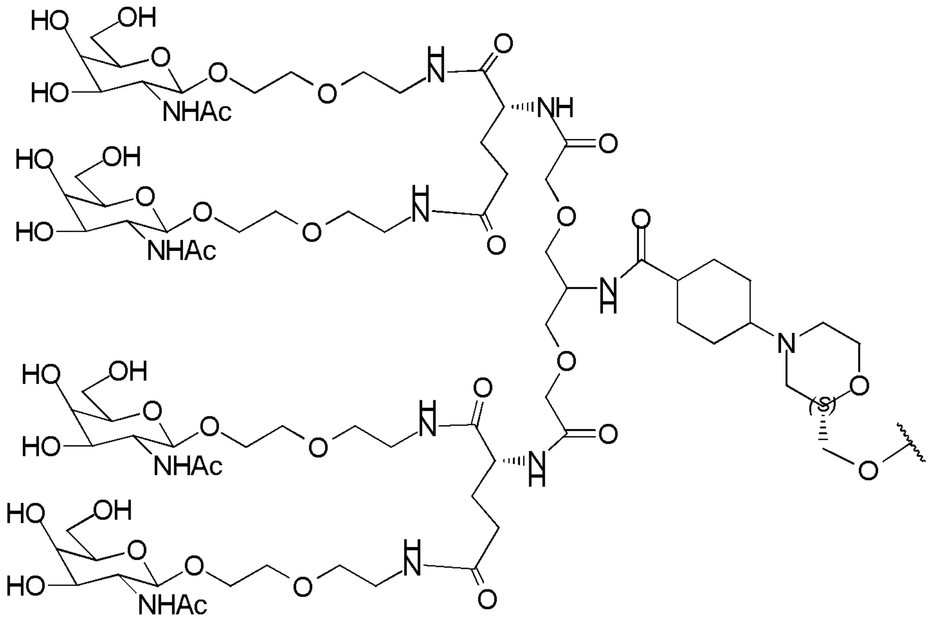 |
| 27 | 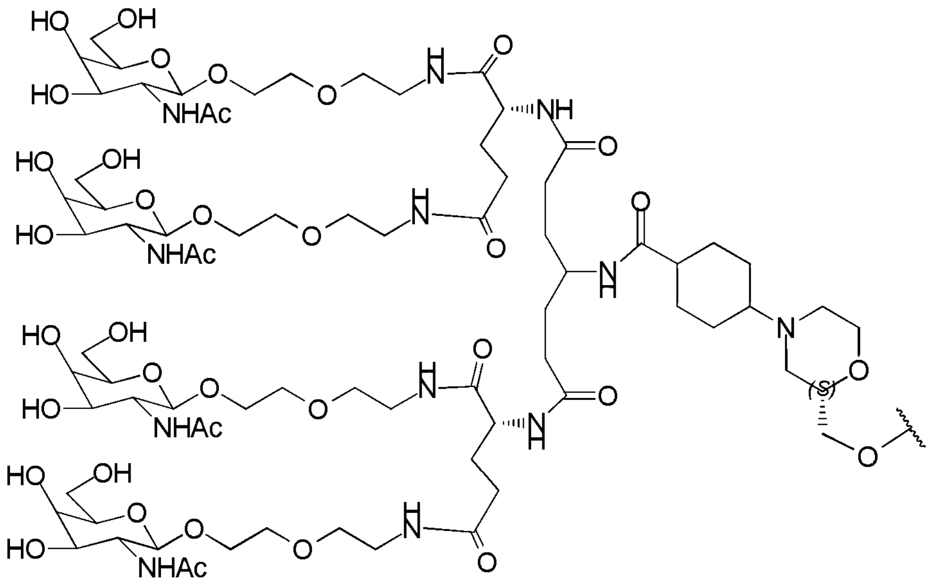 |

(continued)

| NO. | Structure |
|---|---|
| 28 | 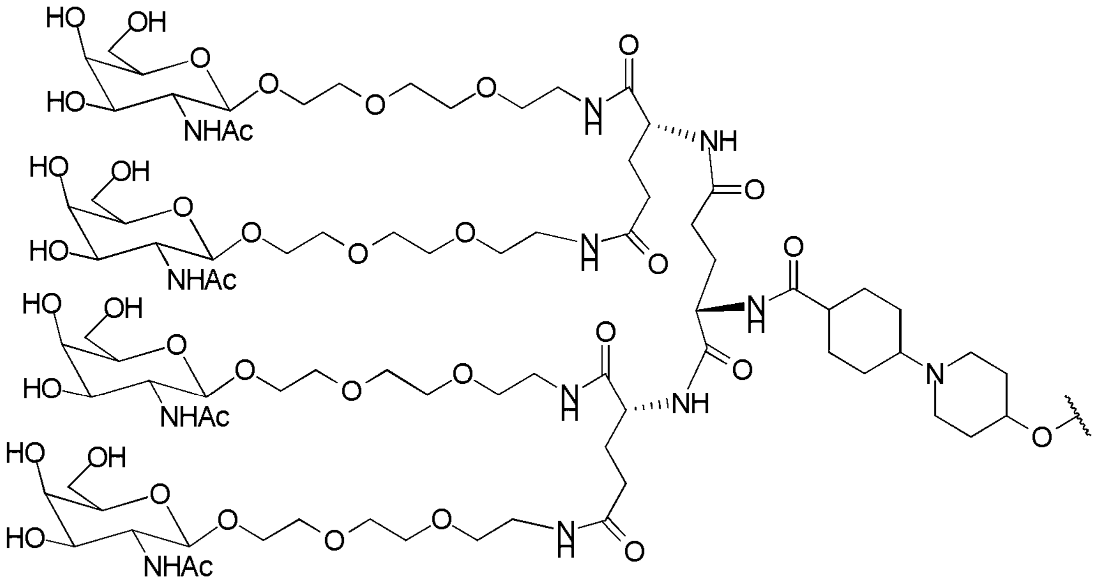 |
| 29 | 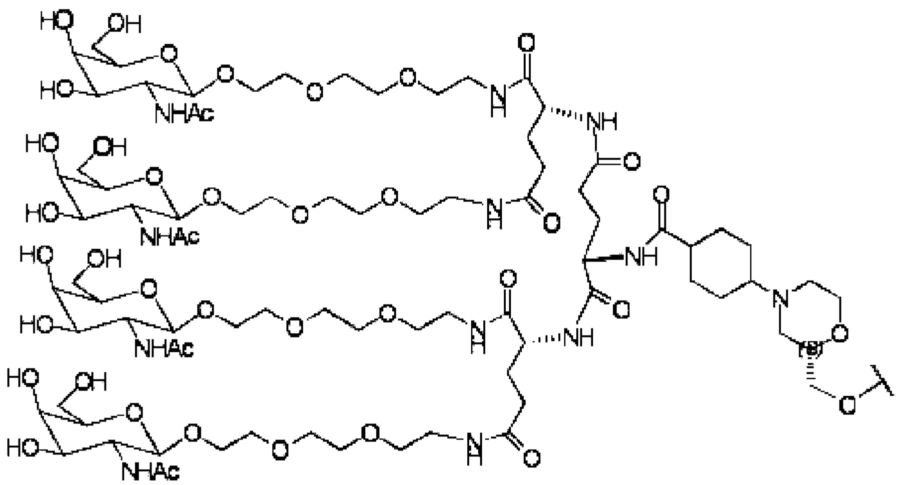 |

In some embodiments, the ligand targets asialoglycoprotein receptor (ASGPR). In some embodiments, the ligand targets asialoglycoprotein receptor (ASGPR) on hepatocytes.

In a preferred embodiment, the ligand has the following structure:

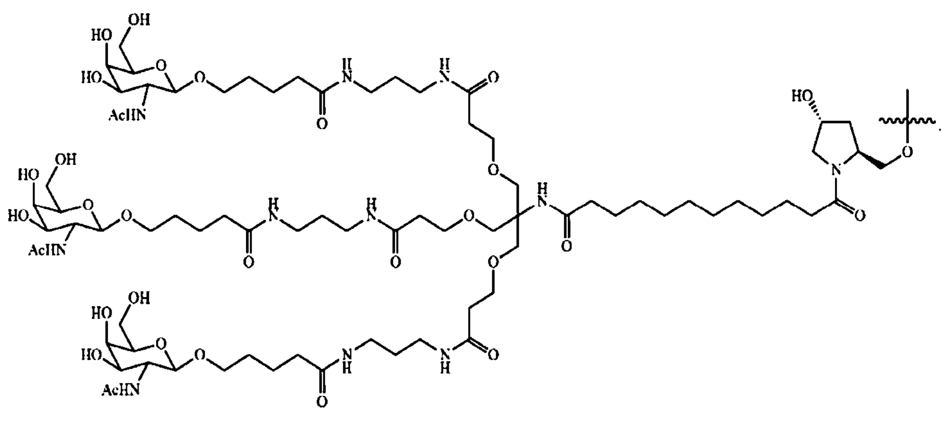

,

wherein

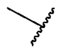

represents the point of attachment to the dsRNA through a phosphate group or a phosphorothioate group.

In a preferred embodiment, the ligand has the following structure:

(NAG37) =

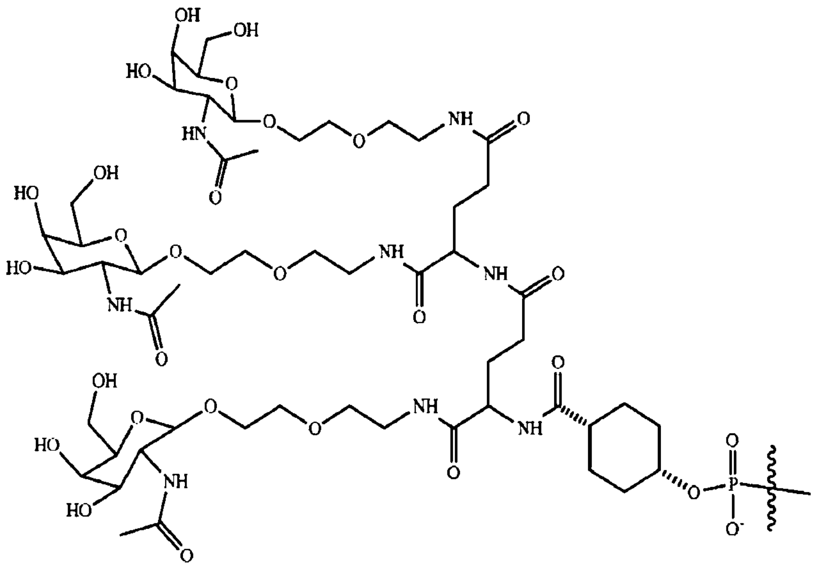

,

wherein

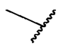

represents the point of attachment to the dsRNA through a phosphate group or a phosphorothioate group.

In a preferred embodiment, the ligand has the following structure:

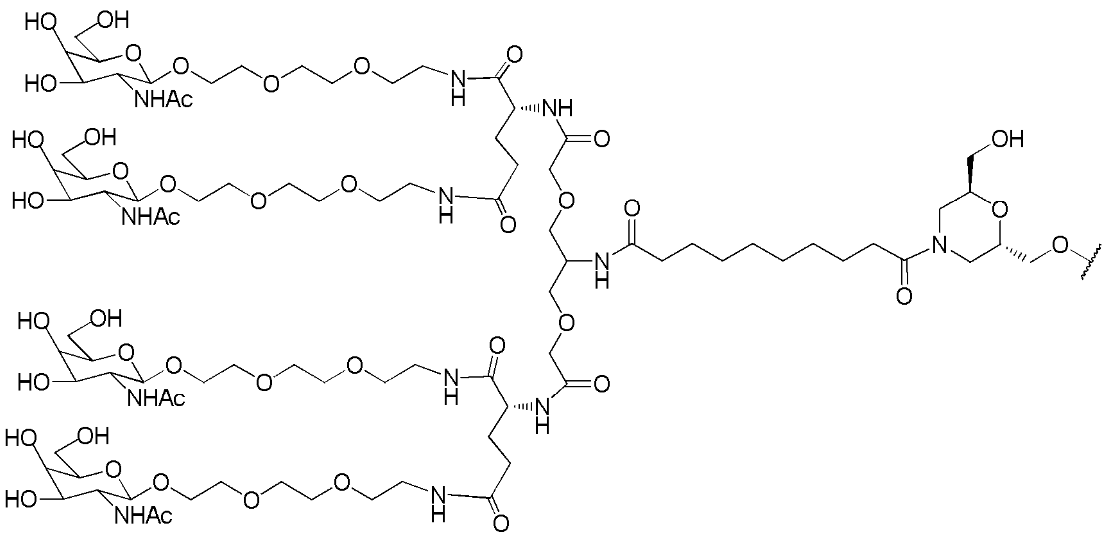

,

wherein

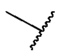

represents the point of attachment to the sense strand of the dsRNA via a phosphate group or a phosphorothioate group.

In a preferred embodiment, the ligand has the following structure:

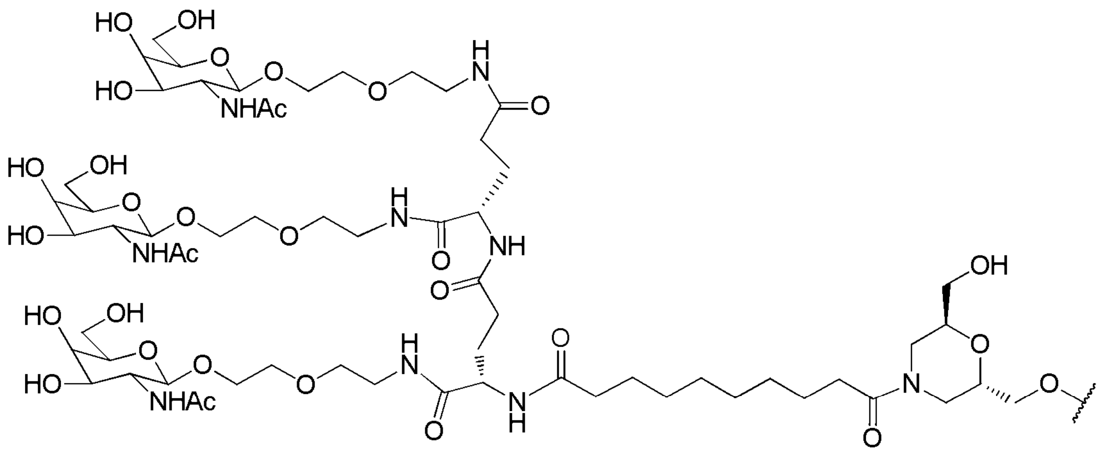

,

wherein

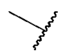

represents the point of attachment to the sense strand of the dsRNA via a phosphate group or a phosphorothioate group.

## IV. Inhibition of MASP2 Gene Expression

The dsRNA of the present disclosure is capable of inhibiting *MASP2* gene expression by at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99%.

Inhibition of *MASP2* gene expression may be manifested by a reduction in the levels of mRNA expressed by a first cell or population of cells (such cells may be present, for example, in a sample derived from a subject), wherein the *MASP2* gene is transcribed and the cell or cells have been treated (e.g., by contacting the cell or cells with the dsRNA of the present disclosure, or by administering the dsRNA of the present disclosure to a subject in which the cells are present or previously present, such that the *MASP2* gene expression is inhibited compared to a second cell or population of cells (one or more control cells) that is substantially identical to the first cell or population of cells but has not been so treated.

In a preferred embodiment, the inhibition is assessed by expressing the level of mRNA in treated cells as a percentage of the level of mRNA in control cells using the following formula. In some specific embodiments, the $2^{-\triangle\triangle Ct}$ value is calculated to compare the difference between the experimental group and the control group, where $\triangle\triangle Ct = [(Ct_{\text{target gene, experimental group}} - Ct_{\text{reference gene, experimental group}}) - (Ct_{\text{target gene, control group}} - Ct_{\text{reference gene, control group}})]$.

Alternatively, inhibition of *MASP2* gene expression, e.g., MASP2 protein expression, may be assessed in terms of a decrease in parameters functionally related to *MASP2* gene expression, e.g., lipid levels, cholesterol levels, such as LDLc levels. *MASP2* gene silencing may be determined by any assay known in the art in any cell expressing MASP2 constitutively or by genomic engineering. The liver is the primary site of MASP2 expression, and other significant sites for expression include.

Inhibition of the expression of the MASP2 protein can be manifested by a reduction in the levels of MASP2 protein expressed by a cell or population of cells (e.g., levels of the protein expressed in a sample derived from a subject). As explained above with respect to the assessment of mRNA inhibition, the inhibition of protein expression levels in a treated cell or population of cells may similarly be expressed as a percentage of the level of the protein in a control cell or population of cells.

A control cell or population of cells that may be used to assess inhibition of *MASP2* gene expression includes a cell or population of cells that has not been exposed to the dsRNA of the present disclosure. For example, the control cell or population of cells may be derived from an individual subject (e.g., a human or animal subject) prior to the treatment of the subject with the dsRNA.

## V. Cells

The present disclosure provides a cell comprising the dsRNA of the present disclosure, wherein the dsRNA of the present disclosure is capable of transcription in the cell.

## VI. Pharmaceutical Composition

The present disclosure provides a pharmaceutical composition comprising the dsRNA or the cell of the present disclosure, and optionally a pharmaceutically acceptable carrier or excipient.
As used herein, "pharmaceutically acceptable" refers to those compounds, materials, compositions, and/or dosage forms which, within the scope of sound medical judgment, are suitable for contact with tissues of human subjects and animal subjects without undue toxicity, irritation, allergic reactions, or other problems or complications, commensurate with a reasonable benefit/risk ratio.
As used herein, a pharmaceutically acceptable carrier refers to a pharmaceutical carrier that facilitates the administration of a dsRNA, a vector or a cell comprising the sequence encoding the same to the human body and/or facilitates absorption or action thereof. For example: diluents, excipients such as water, and fillers such as starch and sucrose; binders such as cellulose derivatives, alginates, gelatin, and polyvinylpyrrolidone; wetting agents such as glycerin; disintegrants such as agar, calcium carbonate, and sodium bicarbonate; absorption accelerators such as quaternary ammonium compounds; surfactants such as cetyl alcohol; adsorption carriers such as kaolin and bentonite; lubricants such as talc, calcium/magnesium stearate, and polyethylene glycol. Additional adjuvants such as flavoring agents and sweeteners can also be added to the composition.
For example, a pharmaceutical composition comprising the dsRNA, vector or cell of the present disclosure may comprise a pharmaceutically acceptable diluent or a sustained release matrix into which the dsRNA or vector of the present disclosure is embedded.
The pharmaceutical composition of the present disclosure may comprise a drug delivery system for delivering dsRNA, such as a nanoparticle, polymer, liposome, PEG, or cationic delivery system.

## VII. Kit

The present disclosure provides a kit comprising the dsRNA, cell or pharmaceutical composition of the present disclosure.
The present disclosure also provides kits for using the dsRNA of the present disclosure and/or performing the methods of the present disclosure. Such kits include one or more dsRNAs, cells, or pharmaceutical compositions of the disclosure, and may further include instructions for use. The instructions for inhibiting expression of MASP2 in a cell by contacting the cell with a dsRNA of the present disclosure in an amount effective to inhibit expression of MASP2 may be recorded in the instructions for use.
In the case where the dsRNA of the present disclosure is contacted with a cell in vitro, optionally, the kit of the present disclosure may further comprise a means for contacting the cell with the dsRNA of the present disclosure (e.g., an injection device) or a means for measuring the inhibitory effect of MASP2 (e.g., a means for measuring the inhibition of MASP2 mRNA or protein). Such means for measuring inhibition of MASP2 may comprise means for obtaining a sample (e.g., a plasma sample) from a subject.
In the case where the dsRNA or pharmaceutical composition of the present disclosure, or a cell into which the dsRNA has been introduced in vitro is administered into the body, the kit of the present disclosure may optionally further comprise a device for administering the dsRNA, the cell or the pharmaceutical composition of the present disclosure to a subject, or a device for determining a therapeutically effective amount or a prophylactically effective amount.

## VIII. Treatment Methods and Pharmaceutical Uses

The present disclosure provides a method of reducing mannan-binding lectin-associated serine protease 2 (MASP2) in a subject, comprising the step of administering to the subject the dsRNA, cell, or pharmaceutical composition of the disclosure.
The present disclosure provides a method of treating, preventing, inhibiting or alleviating a disease or disorder benefiting from reduced expression of mannan-binding lectin-associated serine protease 2 (MASP2) in a subject, comprising the step of administering to the subject the dsRNA, cell, or pharmaceutical composition of the present disclosure. The present disclosure also provides a method of treating, preventing, inhibiting or alleviating at least one symptom in a patient suffering from a disease or disorder benefiting from reduced expression of mannan-binding lectin-associated serine protease 2 (MASP2).
In some embodiments, the disease or disorder benefiting from reduced expression of mannan-binding lectin-

associated serine protease 2 (MASP2) is a MASP2-associated disease. In some embodiments, the MASP2-associated disease is arthritis, IgA nephropathy, thrombotic microangiopathy, venous embolism, diabetic nephropathy, and membranous nephropathy.

In some embodiments, a method of reducing mannan-binding lectin-associated serine protease 2 (MASP2) in a subject, a method of treating, preventing, inhibiting, or alleviating a disease or disorder benefiting from reduced expression of mannan-binding lectin-associated serine protease 2 (MASP2) in a subject, or a method of treating, preventing, inhibiting, or alleviating at least one symptom in a patient suffering from a disease or disorder benefiting from reduced expression of mannan-binding lectin-associated serine protease 2 (MASP2) comprises subcutaneously or intravenously administering the dsRNA or pharmaceutical composition to the subject. In some embodiments, the subject is a human patient.

The present disclosure also relates to the dsRNA, cell or pharmaceutical composition of the present disclosure for use in treating a disease or symptom associated with MASP2 expression in a subject.

The present disclosure also relates to the use of the dsRNA, cell, or pharmaceutical composition of the present disclosure for the manufacture of a medicament for treating, preventing, inhibiting or alleviating a disease or disorder benefiting from reduced expression of mannan-binding lectin-associated serine protease 2 (MASP2) in a subject. The disclosure also relates to the use of the dsRNA, cell, or pharmaceutical composition of the disclosure for the manufacture of a medicament for reducing mannan-binding lectin-associated serine protease 2 (MASP2) in a subject. The disclosure also relates to the use of the dsRNA, cell, or pharmaceutical composition of the disclosure for the manufacture of a medicament for treating, preventing, inhibiting or alleviating at least one symptom in a patient suffering from a disease or disorder benefiting from reduced expression of mannan-binding lectin-associated serine protease 2 (MASP2). The medicament of the present disclosure can be prepared as an emulsion, a microemulsion, a microparticle, and the like.

**Sequence**

The RNA sequence provided by the present disclosure targets the human MASP2 gene (or target gene, target mRNA sequence, target sequence). The target MASP2 mRNA sequence is shown in Genbank Accession No. NM _ 006610.3. The dsRNA sequences provided by the present disclosure are shown in Table 3.

Table 3. Nucleotide sequences of the sense and antisense strands targeting MASP2 mRNA

| SEQ ID NO: | Sequence (5'->3') | SEQ ID NO: | Sequence (5'->3') |
|---|---|---|---|
| 1 | UACGUCCUGCACCGUAACAAA | 21 | UUUGUUACGGUGCAGGACGUAGC |
| 2 | CCUGUGUCCCUACGACUUU | 22 | AAAGUCGUAGGGACACAGGGU |
| 3 | ACGACUUUCUCAAGAUUCA | 23 | UGAAUCUUGAGAAAGUCGUAG |
| 4 | AGCGCAGCCUUGCCCUUAU | 24 | AUAAGGGCAAGGCUGCGCUGU |
| 5 | AGCCUUGCCCUUAUCCGAU | 25 | AUCGGAUAAGGGCAAGGCUGC |
| 6 | AUCCUGAAAGACAGCUUCU | 26 | AGAAGCUGUCUUUCAGGAUGU |
| 7 | UGUGAAGAGACCUUCUACA | 27 | UGUAGAAGGUCUCUUCACAGC |
| 8 | GAAAGUGAAUGAUGGUAAA | 28 | UUUACCAUCAUUCACUUUCAU |
| 9 | AAGUGAAUGAUGGUAAAUA | 29 | UAUUUACCAUCAUUCACUUUC |
| 10 | AGUGAAUGAUGGUAAAUAU | 30 | AUAUUUACCAUCAUUCACUUU |
| 11 | GUGAAUGAUGGUAAAUAUA | 31 | UAUAUUUACCAUCAUUCACUU |
| 12 | AUAUGUGUGUGAGGCUGAU | 32 | AUCAGCCUCACACACAUAUUU |
| 13 | ACAGCAGCAGGUGCACUUU | 33 | AAAGUGCACCUGCUGCUGUGG |
| 14 | GCAGGUGCACUUUUAUAUA | 34 | UAUAUAAAAGUGCACCUGCUG |
| 15 | UUUUAUACAUGAAGGUUAU | 35 | AUAACCUUCAUGUAUAAAAAC |
| 16 | ACAUGAAGGUUAUACUCAU | 36 | AUGAGUAUAACCUUCAUGUAU |
| 17 | ACAAUGACAUAGCACUGAU | 37 | AUCAGUGCUAUGUCAUUGUCA |
| 18 | CAAUGACAUAGCACUGAUU | 38 | AAUCAGUGCUAUGUCAUUGUC |
| 19 | GACAUAGCACUGAUUAAAU | 39 | AUUUAAUCAGUGCUAUGUCAU |

(continued)

| SEQ ID NO: | Sequence (5'->3') | SEQ ID NO: | Sequence (5'->3') |
|---|---|---|---|
| 20 | ACAUAGCACUGAUUAAAUU | 40 | AAUUUAAUCAGUGCUAUGUCA |

Tables 4 and 5 show the modified RNA sequences used in the present disclosure.

The meanings of the abbreviations used herein are as follows:

"A", "U", "G", and "C" represent a natural adenine ribonucleotide, an uracil ribonucleotide, a guanine ribonucleotide, and a cytosine ribonucleotide, respectively.

"d" represents that the nucleotide adjacent to its right side is a deoxyribonucleotide. For example, "dA", "dT", "dG" and "dC" represent adenine deoxyribonucleotide, a thymine deoxyribonucleotide, a guanine deoxyribonucleotide, and a cytosine deoxyribonucleotide, respectively.

"m" represents that the nucleotide adjacent to its left side is a 2'-OCH3 modified nucleotide. For example, "Am", "Um", "Gm", and "Cm" represent 2'-OCH3 modified A, U, G, and C, respectively.

"f" represents that the nucleotide adjacent to its left side is a 2'-fluoro modified nucleotide. For example, "Af", "Uf", "Gf", and "Cf" represent 2'-fluoro modified A, U, G, and C, respectively.

"s" represents that two adjacent nucleotides to the left and right sides are linked by a phosphorothioate linkage.

"s-" represents that the nucleotide adjacent to its left side and the ligand adjacent to its right side are linked via a phosphorothioate linkage.

"SCP1a-X" represents a modified nucleotide X having the following structure:

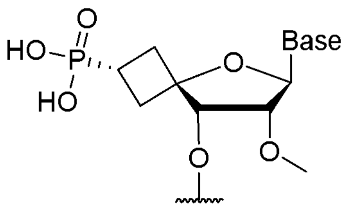

,

"X" represents a nucleotide selected from the group consisting of adenine, guanine, uracil and cytosine, wherein "Base" is a nucleotide base.

"SCP modification" refers to a modified nucleotide having the structure:

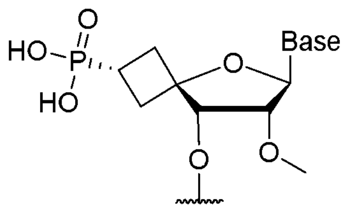

,

wherein "Base" is independently selected from H, a modified or unmodified Base, or a leaving group. Preferably, "Base" is an unmodified Base selected from the group consisting of adenine base, guanine base, uracil base and cytosine base. In other embodiments, Base is a modified base.

The "VP" indicates that the nucleotide adjacent to its right side is a vinyl phosphate-modified nucleotide, which is well known in the art and can be seen, for example, in PCT Publication Nos. WO2011139702, WO2013033230 and WO2019105419.

"L96" represents a GalNAc ligand of the following structure well known in the art, wherein

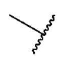

represents the point of attachment to the dsRNA through a phosphate group or a phosphorothioate group. See, for example, PCT Publication Nos. WO2009073809 and WO2009082607.

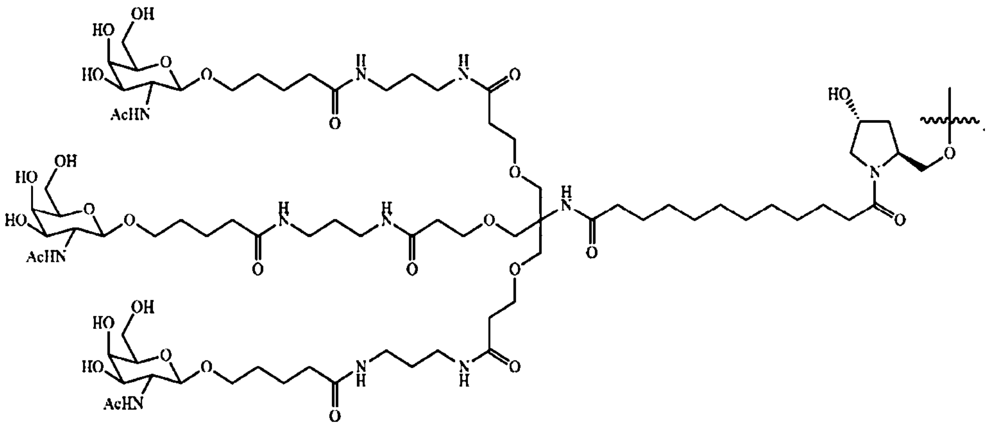

"GL6" represents a GalNAc ligand of the following structure wherein 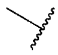 represents the point of attachment to the dsRNA through a phosphate group or a phosphorothioate group

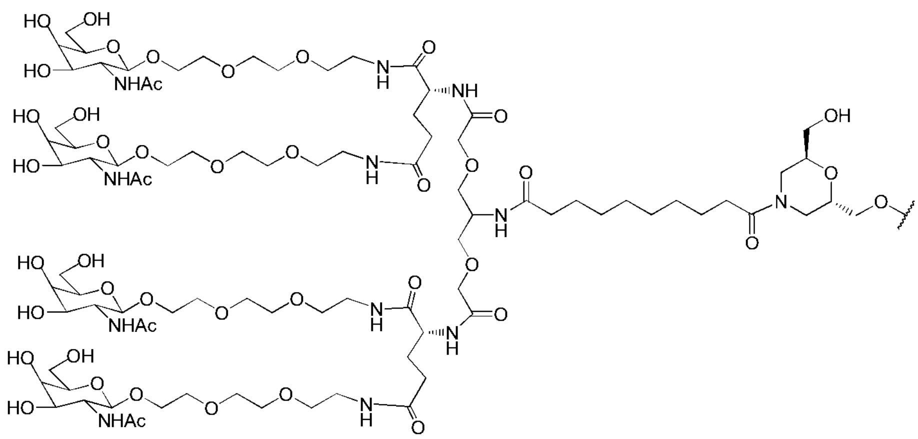

Table 4. Modified siRNA sense strand sequence targeting the MASP2 gene

| Single strand numbering | Sequence (5'->3') | SEQ ID NO |
|---|---|---|
| Sense strand conjugated to a ligand | | |
| SR013810S | UmsAmsCmGmUmCmCfUmGfCfAfCmCmGmUmAmAmCmAmAmAm-L96 | 59 |
| SR013821S | CmsCmsUmGmUmGmUfCfCfCmUmAmCmGmAmCmUmUmsUms-GL6 | 60 |
| SR013822S | AmsCmsGmAmCmUmUfUfCfUmCmAmAmGmAmUmUmCmsAms-GL6 | 61 |
| SR013823S | AmsGmsCmGmCmAmGfCfCfUmUmGmCmCmCmUmUmAmsUms-GL6 | 62 |
| SR013824S | AmsGmsCmCmUmUmGfCfCfCmUmUmAmUmCmCmGmAmsUms-GL6 | 63 |
| SR013825S | AmsUmsCmCmUmGmAfAfAfGmAmCmAmGmCmUmUmCmsUms-GL6 | 64 |

(continued)

| Single strand numbering | Sequence (5'->3') | SEQ ID NO |
|---|---|---|
| Sense strand conjugated to a ligand | | |
| SR013826S | UmsGmsUmGmAmAmGfAfGfAmCmCmUmUmCmUmAmCmsAms-GL6 | 65 |
| SR013827S | GmsAmsAmAmGmUmGfAfAfUmGmAmUmGmGmUmAmAmsAms-GL6 | 66 |
| SR013828S | AmsAmsGmUmGmAmAfUfGfAmUmGmGmUmAmAmAmUmsAms-GL6 | 67 |
| SR013829S | AmsGmsUmGmAmAmUfGfAfUmGmGmUmAmAmAmUmAmsUms-GL6 | 68 |
| SR013830S | GmsUmsGmAmAmUmGfAfUfGmGmUmAmAmAmUmAmUmsAms-GL6 | 69 |
| SR013831S | AmsUmsAmUmGmUmGfUfGfUmGmAmGmGmCmUmGmAmsUms-GL6 | 70 |
| SR013832S | AmsCmsAmGmCmAmGfCfAfGmGmUmGmCmAmCmUmUmsUms-GL6 | 71 |
| SR013833S | GmsCmsAmGmGmUmGfCfAfCmUmUmUmUmAmUmAmUmsAms-GL6 | 72 |
| SR013834S | UmsUmsUmUmAmUmAfCfAfUmGmAmAmGmGmUmUmAmsUms-GL6 | 73 |
| SR013835S | AmsCmsAmUmGmAmAfGfGfUmUmAmUmAmCmUmCmAmsUms-GL6 | 74 |
| SR013836S | AmsCmsAmAmUmGmAfCfAfUmAmGmCmAmCmUmGmAmsUms-GL6 | 75 |
| SR013837S | CmsAmsAmUmGmAmCfAfUfAmGmCmAmCmUmGmAmUmsUms-GL6 | 76 |
| SR013838S | GmsAmsCmAmUmAmGfCfAfCmUmGmAmUmUmAmAmAmsUms-GL6 | 77 |
| SR013839S | AmsCmsAmUmAmGmCfAfCfUmGmAmUmUmAmAmAmUmsUms-GL6 | 78 |
| sense strand unconjugated to a ligand | | |
| SR012563S | UmsAmsCmGmUmCmCfUmGfCfAfCmCmGmUmAmAmCmAmAmAm | 79 |
| SR011804S | CmsCmsUmGmUmGmUfCfCfCmUmAmCmGmAmCmUmsUmsUm | 80 |
| SR011815S | AmsCmsGmAmCmUmUfUfCfUmCmAmAmGmAmUmUmsCmsAm | 81 |
| SR011893S | AmsGmsCmGmCmAmGfCfCfUmUmGmCmCmCmUmUmsAmsUm | 82 |
| SR011895S | AmsGmsCmCmUmUmGfCfCfCmUmUmAmUmCmCmGmsAmsUm | 83 |
| SR011936S | AmsUmsCmCmUmGmAfAfAfGmAmCmAmGmCmUmUmsCmsUm | 84 |
| SR012059S | UmsGmsUmGmAmAmGfAfGfAmCmCmUmUmCmUmAmsCmsAm | 85 |
| SR012082S | GmsAmsAmAmGmUmGfAfAfUmGmAmUmGmGmUmAmsAmsAm | 86 |

(continued)

| sense strand unconjugated to a ligand | | |
|---|---|---|
| SR012083S | AmsAmsGmUmGmAmAfUfGfAmUmGmGmUmAmAmAmsUmsAm | 87 |
| SR012084S | AmsGmsUmGmAmAmUfGfAfUmGmGmUmAmAmAmUmsAmsUm | 88 |
| SR012085S | GmsUmsGmAmAmUmGfAfUfGmGmUmAmAmAmUmAmsUmsAm | 89 |
| SR012097S | AmsUmsAmUmGmUmGfUfGfUmGmAmGmGmCmUmGmsAmsUm | 90 |
| SR012198S | AmsCmsAmGmCmAmGfCfAfGmGmUmGmCmAmCmUmsUmsUm | 91 |
| SR012204S | GmsCmsAmGmGmUmGfCfAfCmUmUmUmUmAmUmAmsUmsAm | 92 |
| SR012256S | UmsUmsUmUmAmUmAfCfAfUmGmAmAmGmGmUmUmsAmsUm | 93 |
| SR012259S | AmsCmsAmUmGmAmAfGfGfUmUmAmUmAmCmUmCmsAmsUm | 94 |
| SR012290S | AmsCmsAmAmUmGmAfCfAfUmAmGmCmAmCmUmGmsAmsUm | 95 |
| SR012291S | CmsAmsAmUmGmAmCfAfUfAmGmCmAmCmUmGmAmsUmsUm | 96 |
| SR012295S | GmsAmsCmAmUmAmGfCfAfCmUmGmAmUmUmAmAmsAmsUm | 97 |
| SR012296S | AmsCmsAmUmAmGmCfAfCfUmGmAmUmUmAmAmAmsUmsUm | 98 |

Table 5. Modified siRNA antisense strand sequence targeting the MASP2 gene

| Single strand numbering | Sequence (5'-> 3') | SEQ ID NO |
|---|---|---|
| SR013514A | UmsUfsUmGmUmUfAmCfGfGmUmGmCmAfGmGfAmCmGmUmAmsGmsCm | 99 |
| SR012755A | AmsAfsAmGfUmCfGmUfAmGfGmGfAmCfAmCfAmGfGmsGfsUm | 100 |
| SR012766A | UmsGfsAmAfUmCfUmUfGmAfGmAfAmAfGmUfCmGfUmsAfsGm | 101 |
| SR012844A | AmsUfsAmAfGmGfGmCfAmAfGmGfCmUfGmCfGmCfUmsGfsUm | 102 |
| SR012846A | AmsUfsCmGfGmAfUmAfAmGfGmGfCmAfAmGfGmCfUmsGfsCm | 103 |
| SR012887A | AmsGfsAmAfGmCfUmGfUmCfUmUfUmCfAmGfGmAfUmsGfsUm | 104 |
| SR013010A | UmsGfsUmAfGmAfAmGfGmUfCmUfCmUfUmCfAmCfAmsGfsCm | 105 |
| SR013033A | UmsUfsUmAfCmCfAmUfCmAfUmUfCmAfCmUfUmUfCmsAfsUm | 106 |
| SR013034A | UmsAfsUmUfUmAfCmCfAmUfCmAfUmUfCmAfCmUfUmsUfsCm | 107 |
| SR013035A | AmsUfsAmUfUmUfAmCfCmAfUmCfAmUfUmCfAmCfUmsUfsUm | 108 |
| SR013036A | UmsAfsUmAfUmUfUmAfCmCfAmUfCmAfUmUfCmAfCmsUfsUm | 109 |
| SR013048A | AmsUfsCmAfGmCfCmUfCmAfCmAfCmAfCmAfUmAfUmsUfsUm | 110 |
| SR013149A | AmsAfsAmGfUmGfCmAfCmCfUmGfCmUfGmCfUmGfUmsGfsGm | 111 |
| SR013155A | UmsAfsUmAfUmAfAmAfAmGfUmGfCmAfCmCfUmGfCmsUfsGm | 112 |
| SR013207A | AmsUfsAmAfCmCfUmUfCmAfUmGfUmAfUmAfAmAfAmsAfsCm | 113 |
| SR013210A | AmsUfsGmAfGmUfAmUfAmAfCmCfUmUfCmAfUmGfUmsAfsUm | 114 |
| SR013241A | AmsUfsCmAfGmUfGmCfUmAfUmGfUmCfAmUfUmGfUmsCfsAm | 115 |
| SR013242A | AmsAfsUmCfAmGfUmGfCmUfAmUfGmUfCmAfUmUfGmsUfsCm | 116 |
| SR013246A | AmsUfsUmUfAmAfUmCfAmGfUmGfCmUfAmUfGmUfCmsAfsUm | 117 |
| SR013247A | AmsAfsUmUfUmAfAmUfCmAfGmUfGmCfUmAfUmGfUmsCfsAm | 118 |
| SR017694A | UmsUfsUmAfCmCfAmUfCmAfUmUfCmAfCmUfUmUfCmsAmsUm | 119 |
| SR018082A | (SCP1a-U)sUfsUmAfCmCfAmUfCmAfUmUfCmAfCmUfUmUfCmsAmsUm | 120 |
| SR018083A | (SCP1a-U)sdTsUmAmdCCmdAUmCmAmUmdTCmAfCmUmUmUmCmsAm-sUm | 121 |

(continued)

| Single strand numbering | Sequence (5'-> 3') | SEQ ID NO |
|---|---|---|
| SR017693A | UmsUfsUmAfCmCfAmUfCmAfUmUfCmAfCmUfUmUfCmsAfs | 122 |

Table 6. Paired siRNA sense and antisense strands targeting the MASP2 gene

| siRNA conjugated to a ligand | | |
|---|---|---|
| double strand numbering | sense strand numbering | antisense strand numbering |
| DR007102 | SR013810S | SR013514A |
| DR007112 | SR013821S | SR012755A |
| DR007113 | SR013822S | SR012766A |
| DR007114 | SR013823S | SR012844A |
| DR007115 | SR013824S | SR012846A |
| DR007116 | SR013825S | SR012887A |
| DR007117 | SR013826S | SR013010A |
| DR007118 | SR013827S | SR013033A |
| DR007119 | SR013828S | SR013034A |
| DR007120 | SR013829S | SR013035A |
| DR007121 | SR013830S | SR013036A |
| DR007122 | SR013831S | SR013048A |
| DR007123 | SR013832S | SR013149A |
| DR007124 | SR013833S | SR013155A |
| DR007125 | SR013834S | SR013207A |
| DR007126 | SR013835S | SR013210A |
| DR007127 | SR013836S | SR013241A |
| DR007128 | SR013837S | SR013242A |
| DR007129 | SR013838S | SR013246A |
| DR007130 | SR013839S | SR013247A |
| DR009198 | SR013827S | SR017694A |
| DR009427 | SR013827S | SR018082A |
| DR009428 | SR013827S | SR018083A |
| DR009197 | SR013827S | SR017693A |
| siRNA unconjugated to a ligand | | |
| double strand numbering | double strand numbering | double strand numbering |
| DR006925 | SR012563S | SR013514A |
| DR006166 | SR011804S | SR012755A |
| DR006177 | SR011815S | SR012766A |
| DR006255 | SR011893S | SR012844A |
| DR006257 | SR011895S | SR012846A |
| DR006298 | SR011936S | SR012887A |
| DR006421 | SR012059S | SR013010A |
| DR006444 | SR012082S | SR013033A |

(continued)

| siRNA unconjugated to a ligand | | |
|---|---|---|
| double strand numbering | double strand numbering | double strand numbering |
| DR006445 | SR012083S | SR013034A |
| DR006446 | SR012084S | SR013035A |
| DR006447 | SR012085S | SR013036A |
| DR006459 | SR012097S | SR013048A |
| DR006560 | SR012198S | SR013149A |
| DR006566 | SR012204S | SR013155A |
| DR006618 | SR012256S | SR013207A |
| DR006621 | SR012259S | SR013210A |
| DR006652 | SR012290S | SR013241A |
| DR006653 | SR012291S | SR013242A |
| DR006657 | SR012295S | SR013246A |
| DR006658 | SR012296S | SR013247A |

Hereinafter, the content of the present disclosure will be further described with reference to Examples. It is to be understood that the following examples are merely illustrative and should not be construed as limiting the scope of the disclosure.

## Examples

Unless otherwise specified, the materials used in the examples are commercially available from the following sources.

Huh7 cell line was purchased from Cobioer Biosciences Co., LTD., Cat. No. CBP60202;
Hep3B cell line was purchased from Cobioer Biosciences Co., LTD., Cat. No. CBP60197;
PHH cells were purchased from Shanghai Yiheng Technology Co., Ltd., Cat. No. QYLF-HPMC;
PMH cells were purchased from Biocytogen Pharmaceuticals (Beijing) Co., Ltd., Cat. No. 110872;
HEK293A cell line was purchased from Cobioer Biosciences Co., LTD., Cat. No. CBP60436;
Balb/c mice are purchased from Vital River Laboratory Animal Technology Co., Ltd., Cat. No. Balb/c.

### Example 1 Preparation of Compound E7

### 1. Preparation of Intermediate 3-4

*1.1 Preparation of Compound 2*

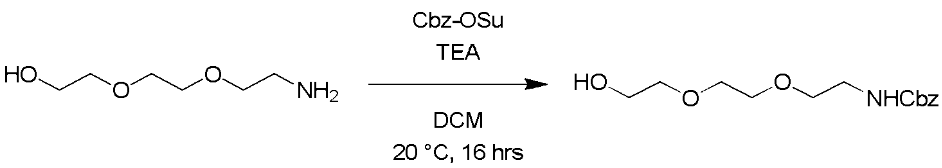


1 2

To Compound 1 (300 g, 2.01 mol) in DCM (1.80 L), benzyl(2,5-dioxopyrrolidin-1-yl) carbonate (600 g, 2.40 mol) was added slowly, and TEA (203 g, 2.01 mol, 280 mL) was added dropwise at 15°C. The mixture was then stirred at 25°C for 16 h until TLC (dichloromethane: methanol = 10:1) showed retention of reactant 1 (Rf = 0.32) and detection of a significant new spot (Rf = 0.52). The reaction mixture was washed with saturated sodium bicarbonate solution (1.00 L × 2).

The organic phase was washed with brine (1.00 L), dried over anhydrous Na2SO4, and subjected to vacuum concentration. Compound 2 (about 385 g) was obtained as a yellow oil without further purification.

*1.2 Preparation of Compound 2A*

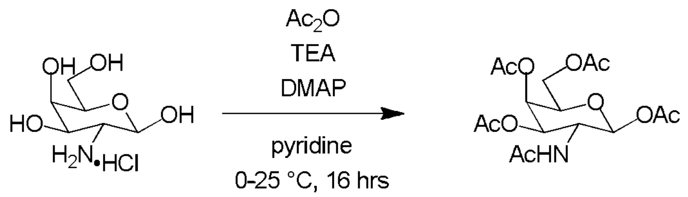


**4** **2A**

To a solution of Compound 4 (350 g, 1.62 mol, HCl) and $Ac_2O$ (994 g, 9.74 mol, 912 mL) in pyridine (1.75 L), DMAP (19.8 g, 162 mmol) was added in one portion and TEA (164 g, 1.62 mol, 226 mL) was added dropwise at 0-15°C. The mixture was stirred at 25°C for 16 h until LCMS (product: RT = 0.687 min) showed complete consumption of the starting reactant. EtOAc (1.40 L) was added to the mixture at 25°C and stirred for 30 min. The resulting mixture was then filtered, and the filter cake was washed with EtOAc (300 mL). The filter cake was ground with water (1.45 L) at 25°C for 30 min. The mixture was filtered and the filter cake was washed with water (175 mL × 3). The filter cake was collected to obtain Compound 2A (about 580 g) as a white solid.

*1.3 Preparation of Compound 2B*

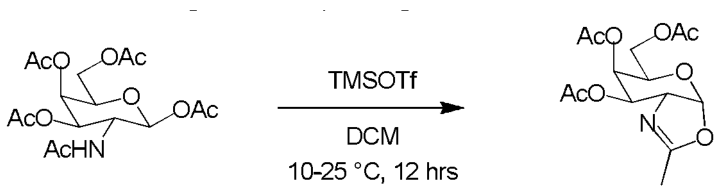


**2A** **2B**

Three reactions were performed in parallel.

To a solution of Compound 2A (200 g, 514 mmol) in DCM (800 mL), TMSOTf (137 g, 616 mmol, 111 mL) was added dropwise over 0.5 h at 10-15°C. The mixture was then stirred at 25°C for 3 h until TLC (dichloromethane: methanol = 20:1) showed complete consumption of Compound 2A ($R_f$ = 0.54) and formation of a new spot ($R_f$ = 0.24). The three reactions were combined. The mixture was cooled to 0-15°C and slowly poured into $NaHCO_3$ (300 g dissolved in 3.00 L of water) at 0-5°C. The organic phase was separated, and the aqueous phase was extracted with DCM (1.00 L × 3). The combined organic phases were dried over $Na_2SO_4$, filtered, and subjected to vacuum concentration. Compound 2B (about 507 g) was obtained as a yellow oil for the next step without further purification.

*1.4 Preparation of Compound 3*

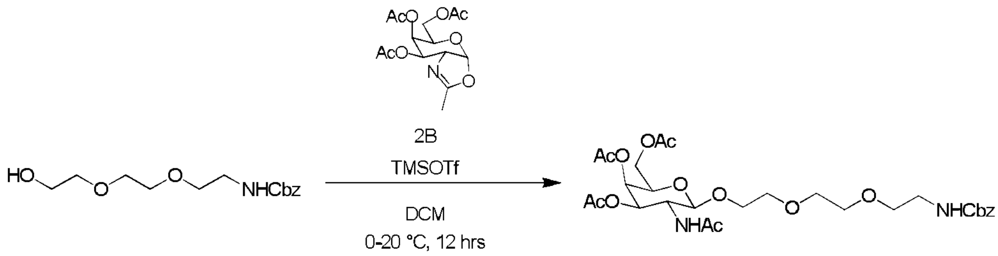


2 3

To a mixture of Compound 2B (250 g, 759 mmol) and Compound 2 (151 g, 531 mmol) in DCM (1.00 L), TMSOTf (84.4 g, 380 mmol, 69.0 mL) was added dropwise at 0-10°C. The mixture was stirred at 20°C for 12 h until TLC (dichloromethane: methanol = 20:1) showed complete consumption of Compound 2 ($R_f$ = 0.33) and formation of a new spot ($R_f$ = 0.03). The combined reaction mixture was cooled to 0-5°C and then poured into $NaHCO_3$ (100 g in 1 L of water) and stirred at 5-10°C for 10 min to separate the phases. The aqueous phase was extracted with DCM (500 mL × 2). The combined organic phases were dried over $Na_2SO_4$, filtered, and subjected to vacuum concentration. Compound 3 (about 360 g) was obtained as a yellow oil without further purification.

$^1$H NMR : (400 MHz, DMSO).

δ=7.79-7.37 (m, 1H), 7.35-7.26 (m, 5H), 5.21-5.20 (m, 1H), 5.00-4.95 (m, 3H), 4.55-4.53 (m, 1H), 4.03-3.86 (m, 3H), 3.61-3.59 (m, 1H), 3.59-3.57 (m, 1H), 3.48-3.40 (m, 6H), 3.39-3.31 (m, 2H), 3.14-3.13 (m, 2H), 2.09 (s, 3H), 1.99 (s, 3H), 1.88 (s, 3H), 1.76-1.74 (m, 3H)

1.5 Preparation of Intermediate 3-4 (TFA salt)

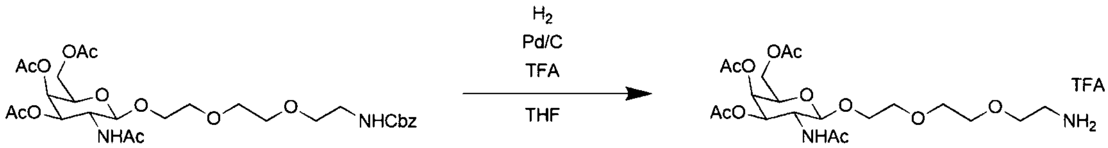


3

Intermediate 3-4 (TFA salt)

Three reactions were performed in parallel.

To a mixture of Pd/C (18.0 g, 16.3 mmol, 10% content) in THF (1.80 L), Compound 3 (180 g, 293 mmol) and TFA (33.5 g, 293 mmol, 21.8 mL) were added under an argon atmosphere. The suspension was degassed and purged three times with hydrogen. The mixture was stirred under $H_2$ (50 Psi) at 30°C for 2 h until LCMS (product: RT = 0.697 min) showed consumption of Compound 3 and detection of the product peak. The three reactions were combined. The mixture was filtered through celite and the filtrate was concentrated under reduced pressure to remove the solvent. Intermediate 3-4 (TFA salt) (393 g, 660 mmol, yield 74.8%, purity 99.6%, TFA) was obtained as a yellow solid without further purification.

$^1$H NMR : (400 MHz, DMSO-d6)

δ = 7.92 (d, *J* = 9.1 Hz, 4H), 5.27-5.17 (m, 1H), 5.03-4.91 (m, 1H), 4.60-4.50 (m, 1H), 4.09-3.97 (m, 4H), 3.85 (s, 2H), 3.65-3.46 (m, 10H), 3.04-2.92 (m, 2H), 2.10 (s, 3H), 2.00 (s, 3H), 1.94-1.86 (m, 3H), 1.82-1.71 (m, 4H).

**2. Preparation of Intermediate 3-5**

*2.1 Preparation of Compound 5*

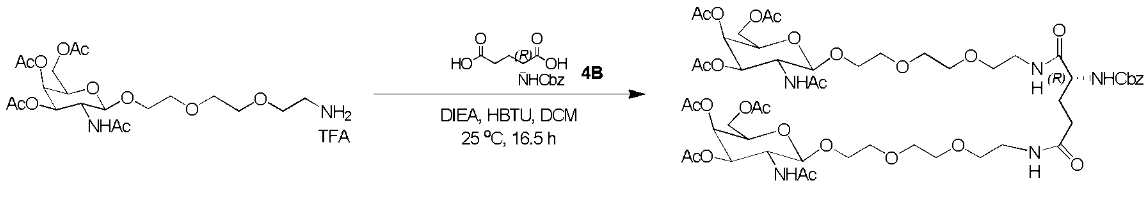


3-4

5

To a solution of Compound 4B (10.0 g, 35.5 mmol, 1.00 eq) and Compound 3-4 (46.3 g, 78.2 mmol, 2.20 eq, TFA) prepared above in DCM (1.00 L), DIEA (30.3 g, 234 mmol, 40.8 mL, 6.60 eq) was added in one portion at 25°C. The mixture was stirred at 25°C for half an hour. HBTU (30.3 g, 234 mmol, 40.8 mL, 6.60 eq) was added to the mixture. The mixture was stirred at 25°C for 16 h until LCMS (product: RT = 0.681 min) showed completion of the reaction. The mixture was subjected to vacuum concentration. The mixture was added with 0.50 N HCl (200 mL × 2) at 20°C, and then extracted with DCM (3 × 500 mL). The combined organic phases were washed with saturated $NaHCO_3$ (3 × 800 mL) until the pH = 8, then washed with brine (3 × 500 mL), dried over $Na_2SO_4$, and subjected to vacuum concentration. The residue was purified by column chromatography ($SiO_2$, DCM: MeOH = 50:1-15:1). The residue was subjected to vacuum concentration at 40°C and purified by preparative-MPLC (column: 800 g Agela C18; mobile phase: [water-ACN]; 15-45%, 25 min; 45%, 10 min). Compound 5 (about 180 g + 75.0 g + 87.0 g + 40.0 g + 38.0 g) was obtained by vacuum drying as a yellow solid.

417.0 g of Compound 3-4 was converted to Compound 5 over 9 batches.

*2.2 Preparation of Intermediate 3-3*

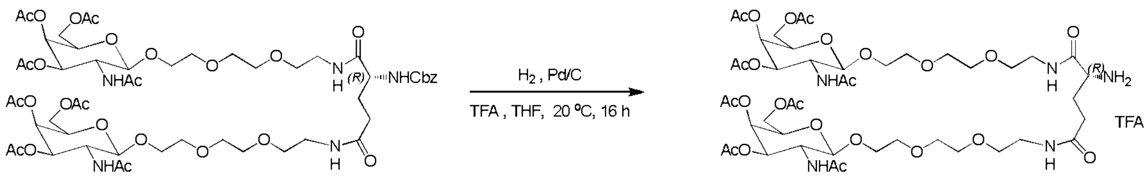


5

3-3

To Pd/C (3.00 g, 10% content) in THF (300 mL), Compound 5 (73.0 g, 61.7 mmol, 1.00 eq) and TFA (7.04 g, 61.7 mmol, 4.57 mL, 1.00 eq) were added under an argon atmosphere. The suspension was degassed and purged three times with hydrogen. The mixture was stirred under $H_2$ (20 Psi) at 20°C for 16 h until TLC (dichloromethane: methanol = 8:1, $R_f$ = 0.0) showed completion of the reaction. The mixture was filtered through celite, and the filtrate was concentrated under pressure to remove the solvent to obtain Compound 3-3 (about 33.4 g + 129 g + 75.0 g) as a white solid.

$^1H$ NMR: (400 MHz, DMSO)

$\delta$=8.53 (t, $J$ = 5.2 Hz, 1H), 8.18 (d, $J$ = 2.4 Hz, 3H), 8.03 (t, $J$ = 5.2 Hz, 1H), 7.84 (dd, $J$ = 3.6 Hz, 2H), 5.22 (d, $J$ = 3.2 Hz, 2H), 4.96 (dd, $J$ = 3.2 Hz, 2H), 4.55 (d, $J$ =8.4 Hz, 2H), 4.02 (t, J=8.8 Hz, 6H), 3.77-3.59 (m, 5H), 3.58-3.45 (m, 21H), 3.40-3.20 (m, 4H), 2.18 (t, $J$ = 7.6 Hz, 2H), 2.17 (d, $J$ =8.0 Hz, 6H), 2.10 (s, 6H), 1.99 (s, 6H), 1.90-1.80 (m, 8H), 1.77 (s, 6H).

**3. Preparation of Compound E7**

*3.1 Preparation of Compound 3*

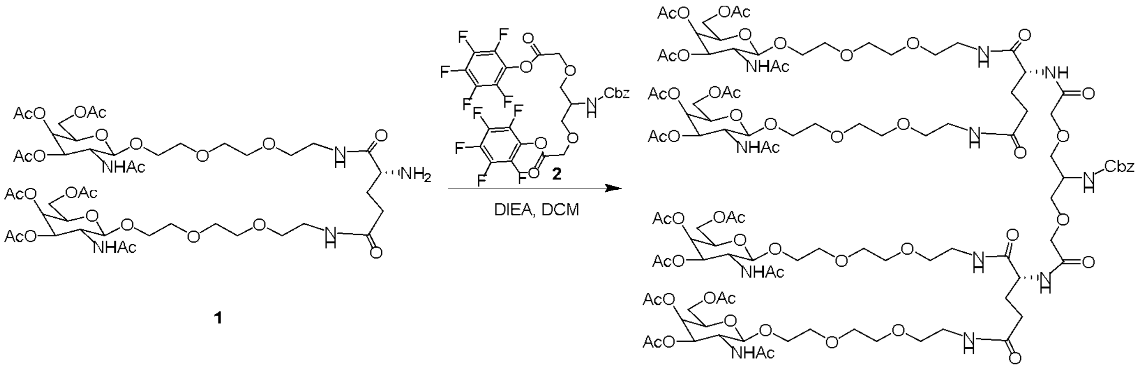


1

3

Compound 1 (2.00 g, 1.87 mmol, prepared as described above for intermediate 3-3) was dissolved in DCM (20.0 mL) at room temperature. DIEA (0.135 mL, 0.814 mmol) and Compound 2 (0.550 g, 0.814 mmol) were added sequentially to the solution, which was then replaced with nitrogen three times. The reaction mixture was stirred at 25°C for 16 h until Liquid Chromatography-Tandem Mass Spectrometry (LC-MS/MS) detected the MS response of the product, and Thin Layer Chromatography (dichloromethane/methanol = 5/1) showed the disappearance of the starting material and formation of a new spot. The reaction mixture was concentrated under reduced pressure. The resulting crude product was purified by column chromatography (dichloromethane/methanol = 5/1) to obtain Compound 3 (about 780 mg) as a white solid.

$^1$H NMR (400 MHz, $CD_3OD$)

$\delta$=7.28-7.42 (m, 5H), 5.30-5.34 (m, 4H), 5.04-5.14 (m, 6H), 4.63-4.67 (m, 4H), 4.36-4.44 (m, 2H), 4.00-4.20 (m, 23H), 3.91-3.95 (m, 4H), 3.69-3.77 (m, 9H), 3.52-3.67 (m, 32H), 3.34-3.43 (m, 9H), 2.29-2.31 (m, 4H), 2.14 (s, 12H), 2.03 (s, 12H), 1.92-1.96 (m, 24H). LCMS: m/z = 1221.6 $(M/2+H)^+$.

*3.2 Preparation of Compound 4*

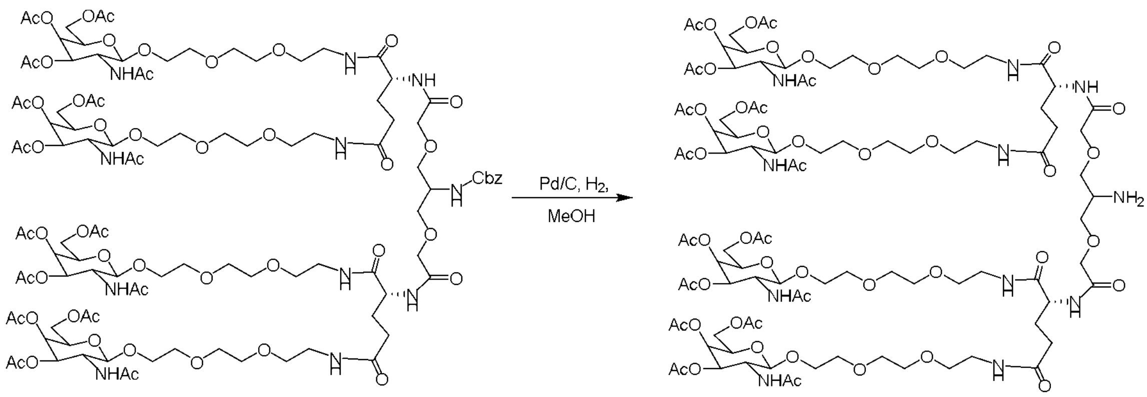


3

4

Compound 3 (1.10 g, 0.451 mmol) was dissolved in MeOH (10.0 mL) at room temperature, and wet Pd/C (0.050 g, 0.451 mmol) with a mass fraction of 10% was added to the solution. The reaction mixture was replaced with hydrogen three times and then stirred at 25°C for 18 h under a hydrogen atmosphere (14.696 psi) until LC-MS/MS detected the MS response of the product, and TLC (dichloromethane/methanol = 10/1, color development: phosphomolybdic acid) showed complete consumption of the starting material and formation of a new spot. The reaction mixture was filtered and the filtrate was concentrated under reduced pressure to obtain Compound 4 (about 840 mg) as a white solid.

$^1$H NMR (400 MHz, $CD_3OD$)

$\delta$=5.32-5.34 (m 4H), 5.06-5.10 (m, 4H), 4.63-4.65 (m, 4H), 4.38-4.40 (m, 2H), 3.99-4.20 (m, 20H), 3.90-3.97 (m, 4H), 3.69-3.76 (m, 6H), 3.50-3.68 (m, 36H), 3.35-3.44 (m, 11H), 2.28-2.38 (m, 4H), 2.15 (s, 12H), 2.03 (s, 12H), 1.90-1.94 (m, 24H). LCMS: m/z = 1154.7 $(M/2+H)^+$.

*3.3 Preparation of Compound 6*

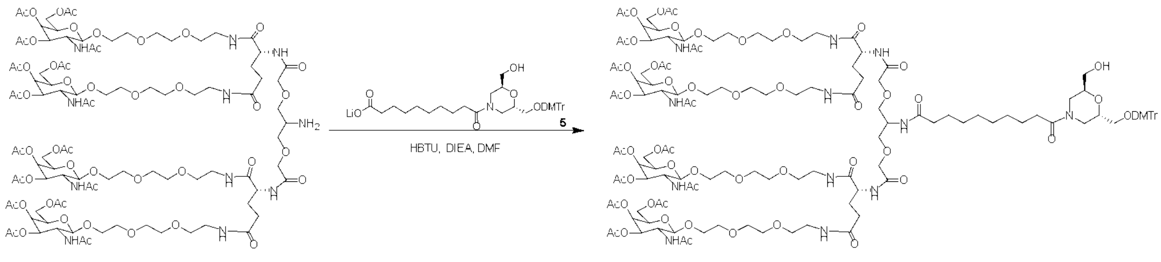


4 6

Compound 5 (232 mg, 0.364 mmol) was dissolved in DCM (10.0 mL) at room temperature, and HBTU (207 mg, 0.546 mmol), DIEA (0.181 mL, 1.09 mmol), and Compound 4 (840 mg, 0.364 mmol) were added sequentially to the solution, which was then replaced with nitrogen three times The reaction mixture was stirred at 25°C for 1 h until LC-MS/MS detected the disappearance of the starting material, and TLC (dichloromethane/methanol = 5/1) showed the disappearance of the starting material and formation of a new spot. The reaction mixture was concentrated under reduced pressure. The resulting crude product was purified by column chromatography (dichloromethane/methanol = 8/1-5/1) to obtain Compound 6 (about 620 mg) as a white solid.

$^1$H NMR (400 MHz, $CD_3OD$)

$\delta$=7.41-7.43 (m, 2H), 7.23-7.34 (m, 7H), 6.83-6.90 (m, 4H), 5.31-5.35 (m, 4H), 5.01-5.12 (m, 4H), 4.63-4.65 (m, 4H), 4.41-4.45 (m, 2H), 4.31-4.33 (m, 1H), 3.99-4.22 (m, 22H), 3.87-3.97 (m, 6H), 3.58-3.81 (m, 45H), 3.34-3.43 (m, 10H), 2.19-2.40 (m, 10H), 2.14 (s, 12H), 2.02 (s, 12H), 1.92-1.96 (mz, 24H), 1.48-1.63 (m, 4H), 1.28-1.38 (m, 8H). LCMS: m/z = 1460.0 (M/2+H)$^+$.

*4. Preparation of Compound E7*

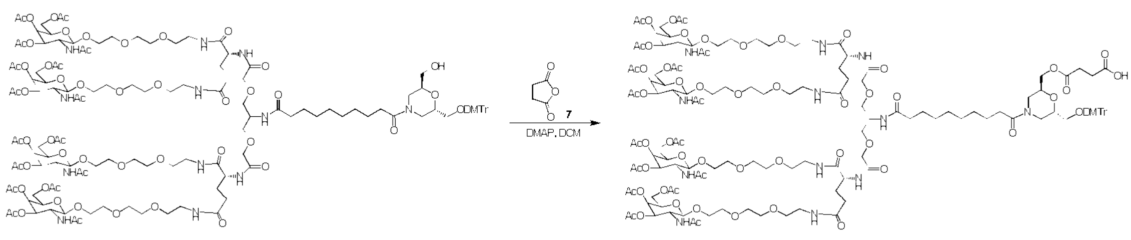


6 E7

Compound 6 (300 mg, 0.103 mmol) was dissolved in DCM (10.0 mL) at room temperature, and DIEA (0.102 mL, 0.618 mmol), Compound 7 (10.3 mg, 0.103 mmol), and DMAP (12.6 mg, 0.103 mmol) were added sequentially to the solution, which was then replaced with nitrogen three times. The reaction mixture was stirred at 25°C for 2 h until LC-MS/MS detected the disappearance of the starting material. The reaction mixture was concentrated under reduced pressure. The resulting crude product was separated by preparative-MPLC (prep-HPLC, column: Waters Xbridge BEH C18 100*30 mm*10 $\mu$m; mobile phase: water-ACN; B%: 17%-57%, 5 min) to obtain Compound E7 (53.0 mg, yield 17.08%, purity 78.94%) as a white solid.

$^1$H NMR (400 MHz, $CD_3OD$) $\delta$=7.41-7.45 (m, 2H), 7.17-7.34 (m, 7H), 6.85-6.89 (m, 4H), 5.32-5.36 (m, 4H), 5.03-5.13 (m, 4H), 4.63-4.67 (m, 4H), 4.38-4.47 (m, 2H), 4.32-4.34 (m, 1H), 4.01-4.26 (m, 22H), 3.88-4.00 (m, 6H), 3.77-3.81 (m, 7H), 3.49-3.76 (m, 45H), 3.33-3.47 (m, 10H), 2.56-2.62 (m, 2H), 2.45-2.55 (m, 3H), 2.21-2.38 (m, 7H), 2.14 (s, 12H), 2.05-2.11 (m, 2H), 2.02 (s, 12H), 1.92-1.96 (m, 24H), 1.47-1.68 (m, 4H), 1.28-1.34 (m, 8H)

MS: m/z = 3022.36 (M+H)$^+$.

**Example 2 Preparation of siRNA**

The siRNA of the present disclosure was prepared using a solid phase phosphoramidite method well known in the art. Specific methods can be found, for example, in PCT Publication Nos. WO2016081444 and WO2019105419, and are briefly described below.

Preparation of siRNA unconjugated to a ligand

*1.1 Synthesis of Sense Strand (SS Strand)*

Through solid-phase phosphoramidite synthesis method, the blank CPG solid-phase support was used as the initial cycle, and nucleoside monomers were linked one by one from 3'-5' direction according to the nucleotide sequence of the sense chain. Each addition of a nucleoside monomer involves four sequential reactions: deprotection, coupling, capping, and oxidation or sulfurization. The synthesis conditions for oligonucleotides at a 5 μmol scale were as follows:

Commercially available phosphoramidites with 2'-F, 2'-O-methyl, and other modifications were used. The nucleoside monomers were supplied as a 0.05 mol/L acetonitrile solution. The reaction conditions for each step were identical: the temperature was 25°C, and deprotection was performed three times using a 3% trichloroacetic acid-dichloromethane solution. For the coupling reaction, the activator used was a 0.25 mol/L ETT-acetonitrile solution, with coupling conducted twice. For capping, 10% acetic anhydride-acetonitrile and pyridine/N-methylimidazole/acetonitrile (10:14:76, v/v/v) were used, with capping performed twice. For oxidation: a 0.05 mol/L iodine/tetrahydrofuran/pyridine/-water solution (70:20:10, v/v/v) was used, with oxidation performed twice. For thiosulfurization, 0.2 mol/L PADS in acetonitrile/3-methylpyridine (1:1, v/v) was used, with thiosulfurization performed twice.

*1.2 Synthesis of Antisense Strand (AS Strand)*

Through solid phase phosphoramidite synthesis method, the blank CPG solid support was used as the initial cycle, and nucleoside monomers were linked one by one from 3'-5' direction according to the nucleotide sequence of antisense strand. Each addition of a nucleoside monomer involves four sequential reactions: deprotection, coupling, capping, and oxidation or sulfurization, and the synthesis conditions for the 5 μmol-scale oligonucleotide of the antisense strand were the same as those for the sense strand.

*1.3 Purification and annealing of oligonucleotides*

1.3.1 Aminolysis

The synthesized solid-phase support (either the sense strand or the antisense strand) was added into a 5 mL centrifuge tube. A 3% diethylamine/ammonia solution (v/v) was added, and the reaction was carried out in a constant-temperature water bath at 35°C for 16 hours (or at 55°C for 8 hours). The mixture was filtered, and the solid-phase support was washed three times with ethanol/water, using 1 mL each time. After the filtrate was centrifuged and concentrated, the crude product was purified.

1.3.2 Purification

The methods for purification and desalting are well-known to those skilled in the art. For example, a strong anionic filler was used to pack the column, and a sodium chloride-sodium hydroxide system was employed for elution and purification, followed by collecting and pooling the products. A gel filler purification column could be used for desalting, with pure water as the elution system.

1.3.3 Annealing

According to Table 6, the sense strand (SS strand) and the antisense strand (AS strand) were mixed in a molar ratio (SS strand/AS strand = 1/1.05), heated to 70-95°C in a water bath for 3-5 min, naturally cooled to room temperature, and the system was freeze-dried to obtain the product.

The following siRNAs in Table 6 were finally obtained: DR006925, DR006166, DR006177, DR006255, DR006257, DR006298, DR006421, DR006444, DR006445, DR006446, DR006447, DR006459, DR006560, DR006566, DR006618, DR006621, DR006652, DR006653, DR006657 and DR006658. Among them, DR006925 is a siRNA in the prior art and was used as a positive control in the disclosure.

**2. Preparation of siRNA with Sense Strand Conjugated to a Ligand**

2.1 Conjugation of ligand to CPG Support

Conjugation of Compound E7 to CPG Support

Compound E7 (53 mg, 0.018 mmol) and HBTU (13.3 mg, 0.035 mmol) were mixed and dissolved in acetonitrile (5 mL) by shaking. DIEA (9.0 mg, 0.07 mmol) and DMAP (2.1 mg, 0.018 mmol) were then added and dissolved by shaking until clear. Blank carrier Resin (550 mg, CPG pore size 1000Å) was weighed and added to the reaction mixture, which was then placed on a shaker at 20°C overnight. A sample was taken and monitored until Thin Layer Chromatography (TLC) (developing solvent: DCM/methanol = 4/1; color development: phosphomolybdic acid) showed completion of the reaction. The reaction mixture was filtered through a sand core funnel. The filter cake was washed with anhydrous acetonitrile (20 mL*5), collected, and suction-filtered under reduced pressure using an oil pump for 6 h to obtain 530 mg of an off-white solid.

The above condensed product (530 mg) was transferred to a 50 mL round-bottom flask. CapC (DMAP/acetonitrile), CapB (*N*-methylimidazole/pyridine/acetonitrile), and CapA (acetic anhydride/acetonitrile) were added sequentially. The mixture was then placed on a shaker at room temperature overnight. After filtration, the filter cake was washed with acetonitrile (20 mL*4), collected, and suction-filtered under reduced pressure using an oil pump for 8 h to obtain 200 mg of an off-white solid for solid-phase synthesis.

2.2 Synthesis of Sense Strand (SS Strand)

Using the solid-phase phosphoramidite method, the E7 solid support prepared above was used as the starting cycle, and nucleoside monomers were linked one by one from the 3' to 5' direction according to the arrangement of sense strand nucleotides. Each linking of a nucleoside monomer involved four steps of deprotection, coupling, capping, and oxidation or thiolation to synthesize 5 umol oligonucleotide with the synthesis conditions as follows:

Nucleoside monomers were provided in a 0.05 mol/L acetonitrile solution. The conditions for each step were the same: temperature 25°C; deprotection for 3 times using a 3% trichloroacetic acid-dichloromethane solution; coupling twice using a 0.25 mol/L ETT-acetonitrile solution as an activator; capping twice using 10% acetic anhydride-acetonitrile and pyridine/N-methylimidazole/acetonitrile (10:14:76, v/v/v); oxidation twice using 0.05 mol/L of iodine in tetrahydrofuran/pyridine/water (70:20:10, v/v/v); thiolation twice using 0.2 mol/L PADS in acetonitrile/3-methylpyridine (1:1, v/v).

2.3 Synthesis of Antisense Strand (AS Strand)

Using the solid-phase phosphoramidite method, a blank CPG solid support was used as the starting cycle, and nucleoside monomers were linked one by one from the 3' to 5' direction according to the arrangement of antisense strand nucleotides. Each linkage of a nucleoside monomer involved four steps of deprotection, coupling, capping, and oxidation or thiolation. The conditions for the synthesis of a 5 μmol oligonucleotide for the antisense strand were the same as those for the sense strand.

2.4 Purification and annealing of oligonucleotides

2.4.1 Ammonolysis

The synthesized solid support (sense or antisense strand) was transferred to a 5 mL centrifuge tube, followed by the addition of 3% diethylamine/ammonia (v/v). The mixture was allowed to react in a thermostatic water bath at 35°C for 16 h (or 55°C for 8 h), and then filtered. The solid support was washed three times with ethanol/water, 1 mL each time. The filtrate was concentrated by centrifugation, and the crude product was purified.

2.4.2 Purification

The methods for purification and desalting are well known to those skilled in the art. For example, a strong anionic packing column and a sodium chloride-sodium hydroxide system may be used for elution and purification. The product can be collected in tubes and desalted using a gel packing purification column, with pure water as the eluent.

2.4.3 Annealing

According to Table 6, the sense strand (SS Strand) was mixed with the antisense strand (AS Strand) at a molar ratio (SS Strand/AS Strand = 1/1.05). The mixture was heated in a water bath to 70-95°C, held for 3-5 min, and then allowed to cool naturally to room temperature. The system was freeze-dried to obtain the product.

The following siRNAs in Table 6 were finally obtained: DR007102, DR007112-DR007130, DR009198, DR009427, DR009428 and DR009197. Among them, DR007102 is a siRNA in the prior art, which was used as a positive

control in the disclosure.

**Example 3 Screening for Activity in Human Primary Hepatocyte (PHH)**

*Cell transfection*

1.4 mL of rat tail collagen solution (Sigma, C3867) was added to 40.6 mL of DNase/RNase-Free Distilled Water and mixed well. The mixture was added to a 96-well culture plate at 40 μL/well, and coated overnight at 4°C. The coating medium was discarded the next day.

On Day 2, prior to use, the coated cell plate was rinsed with DPBS, which was then aspirated. PHHs (Shanghai Xuanyi Biotechnology Co., Ltd., Cat#: QYLF-HPMC) were recovered at 37°C, transferred into the recovery medium, centrifuged, resuspended, and counted. PHHs were plated in a 96-well plate at 90 μL/well ($2\times10^4$ cells/well). The complete medium was replaced after 4 h, and transfection was performed after 18 h.

On Day 3, the 20 μM siRNA stock solution was diluted with Opti-MEM. 198 μL of Opti-MEM was added to 2 μL of 20 μM siRNA stock solution and mixed well by pipetting, as the first concentration point. Serial dilutions were made as required for the experiment.

On Day 3, 0.9 μL of RNAiMAX (Thermo, 13778150) was diluted with 14.1 μL of Opti-MEM, mixed gently by pipetting, and allowed to stand at room temperature for 5 min. Then, 15 μL of the prepared RNAi-MAX mixture and 15 μL of the diluted compound were mixed gently by pipetting (avoiding bubbles), and allowed to stand at room temperature for 10 min. The mixture was transferred to a 96-well plate at 10 μL/well. The plate was extracted after incubation in a 5% $CO_2$ incubator at 37°C for 24 h before RNA extraction.

*RNA Extraction*

Cellular RNA was extracted using a nucleic acid extractor (Hangzhou Allsheng, Auto-pure96) according to the protocol of the high-throughput cell RNA extraction kit (FireGen, FG0412).

*RNA Reverse Transcription*

The denatured reaction mixture was prepared with reference to PrimeScript™ II 1st Strand cDNA Synthesis Kit (Takara, 6210B). Each well contained 1 μL of Oligo dT Primer, 1 μL of dNTP Mixture, and 12.5 μL of template RNA. The denaturation reaction was performed by incubation in a conventional PCR instrument at 65°C for 5 min. The mixture was rapidly cooled on ice for 2 min.

The reverse transcription reaction mixture was prepared with reference to PrimeScript™ II 1st Strand cDNA Synthesis Kit (Takara, 6210B). Each well contained 4 μL of 5×Prime Script II Buffer, 0.5 μL of RNase Inhibitor, and 1 μL of PrimeScript II RTase.

The denatured reaction mixture (14.5 μL) was mixed gently with the reverse transcription reaction mixture. Reverse transcription was performed by incubation in a conventional PCR instrument at 42°C for 45 min, followed by incubation at 95°C for 5 min to inactivate the enzyme. The reverse transcription product (cDNA) was then cooled at 4°C.

After reverse transcription, 30 μL of DNase/RNase-Free Distilled Water was added to the cDNA sample in each well.

*Fluorescence Quantitative PCR*

Fluorescence quantitative PCR reaction was performed in a 20 μL system (ABI, QuantStudio3) with reference to the procedure of TaqMan™ Fast Advanced Master Mix (ABI, 4444965). The reaction program was: (50°C, 2 min) × 1 Cycle; (95°C, 20 s) × 1 Cycle; (95°C, 1 s; 60°C, 24 s) × 40 Cycles.

The information of the primers is shown in Table 7.

Table 7 Primer Information

| Primer name | Sequence Information (5'-3') | SEQ ID NO | Fluorescent group |
|---|---|---|---|
| hACTB-PF | CCTTCCTTCCTGGGCATGG | 41 | / |
| hACTB-PR | TCTTCATTGTGCTGGGTGCC | 42 | / |
| hACTB-P | AACACAGTGCTGTCTGGCGGCACCA | 43 | 5'TET, 3'BHQ2 |
| hMASP2-PF | TGAGCCTGTTTGTGGACTATC | 44 | / |

(continued)

| Primer name | Sequence Information (5'-3') | SEQ ID NO | Fluorescent group |
|---|---|---|---|
| hMASP2-PR | CTGCTGTGGTTCCACCTAAT | 45 | / |
| hMASP2-P | CGCACAACAGGAGGGCGTATATATGG | 46 | 5'6-FAM, 3'BHQ1 |

*Data statistics*

Calculation of $2^{-\Delta\Delta Ct}$ values and conversion to percentages to obtain residual inhibition rate;

$\Delta\Delta Ct$ = [(Ct of target gene of experimental group - Ct of endogenous reference of experimental group) - (Ct of target gene of control group - Ct of endogenous reference of control group)].

The target gene was hMASP2, and the endogenous reference was hACTB.

The final concentration of siRNA was 10 nM, and the activity of siRNA was screened in a human primary hepatocyte. The screening results are shown in Table 8.

Table 8. Results of high-throughput screening for activity in a human primary hepatocyte

| Compound Number | Residual inhibition rate | SD |
|---|---|---|
| DR006925 | 31.48% | 9.24% |
| DR006166 | 21.60% | 2.33% |
| DR006177 | 23.85% | 3.34% |
| DR006255 | 8.93% | 2.23% |
| DR006257 | 13.47% | 2.63% |
| DR006298 | 15.93% | 1.20% |
| DR006421 | 13.74% | 2.04% |
| DR006444 | 18.76% | 2.26% |
| DR006445 | 24.83% | 3.27% |
| DR006446 | 25.05% | 3.24% |
| DR006447 | 28.92% | 0.70% |
| DR006459 | 28.97% | 6.32% |
| DR006560 | 24.49% | 1.50% |
| DR006566 | 12.63% | 2.89% |
| DR006618 | 29.87% | 1.26% |
| DR006621 | 31.26% | 2.57% |
| DR006652 | 19.07% | 2.83% |
| DR006653 | 29.37% | 5.21% |
| DR006657 | 24.61% | 0.92% |
| DR006658 | 15.91% | 5.91% |

**Example 4 Screening for Activity in a Monkey Primary Hepatocyte (PCH)**

*Cell transfection*

100 μL of coating medium was added to each well of 96-well cell culture plates, incubated at 37 °C for 30 minutes, and the coating medium was aspirated after completion.

Primary monkey hepatocytes (PCH, CCH-100CY-PQ, purchased from MILECELL BIOTECHNOLOGY INC.)

were resuscitated at 37 °C. The cells were resuspended in Thawing Medium, centrifuged, and counted, then plated in 96-well plates at 90 μL/well (2 × $10^4$ cells/well).

Compound dilution: A20 μM compound stock solution was diluted in Opti-MEM. Specifically, 198 μL of Opti-MEM was added to 2 μL of the compound stock solution, and the mixture was mixed thoroughly by pipetting to serve as the first concentration point.

Transfection procedure: 0.9 μL of RNAiMAX (Thermo, 13778150) was diluted in 14.1 μL of Opti-MEM, gently pipetted to mix thoroughly, and allowed to stand at room temperature for 5 min. Then, 15 μL of the prepared RNAiMAX mixture and 15 μL of the diluted compound were combined, gently pipetted to mix thoroughly (avoiding bubbles), and allowed to stand at room temperature for 10 min. This mixture was then added to the 96-well plate at 10 μL/well. After incubation in a 5% $CO_2$ incubator at 37 °C for 4 h, the culture medium was replaced, and RNA was extracted the next day.

RNA extraction and PCR procedures were performed as described in Example 3, with the following primers used:

Table 9 Primer Information

| Primer name | Sequence Information (5'-3') | SEQ ID NO | Fluorescent group |
|---|---|---|---|
| mkGAPDH-PF | TCAAGATCGTCAGCAACGCC | 47 | / |
| mkGAPDH-PR | ACAGTCTTCTGGGTGGCAGT | 48 | / |
| mkGAPDH-P | ACCAACTGCTTAGCACCCCTGGCCA | 49 | 5'TET, 3'BHQ2 |
| mkMASP2-PF | TGAGCCTGTTTGTGGACTATC | 50 | / |
| mkMASP2-PR | CTGCTGTGCTTCCACCTAATA | 51 | / |
| mkMASP2-P | AGGAGGGCGTATATATGGAGGGCAAA | 52 | 5'6-FAM, 3'BHQ1 |

The target gene was mkMASP2, and the endogenous reference was mkGAPDH.

The initial concentration of the compound was selected to be 10nM, and the activity of 34 siRNA compounds was screened at 5 concentration points (10mM, 1nM, 0.1 nM, 0.01 nM, 0.001 nM) by 10 times dilution. The experimental results are shown in Table 10, where the percentage value indicates the residual inhibition rate, and the calculation method is the same as that of Example 3.

Table 10 Results of 5-point IC50 activity screening experiment in a monkey primary hepatocyte (PCH)

| | 10nM | 1nM | 0.1nM | 0.01nM | 0.001nM | 5-point IC50 value (nM) |
|---|---|---|---|---|---|---|
| DR006925 | 16.1% | 14.3% | 28.8% | 77.3% | 86.8% | 0.038 |
| DR006166 | 11.9% | 13.9% | 27.7% | 72.9% | 98.3% | 0.029 |
| DR006177 | 11.2% | 12.8% | 27.0% | 71.1% | 87.2% | 0.03 |
| DR006255 | 11.8% | 12.6% | 21.1% | 60.1% | 88.8% | 0.016 |
| DR006257 | 9.6% | 10.8% | 21.2% | 71.8% | 99.8% | 0.023 |
| DR006298 | 12.7% | 16.2% | 43.1% | 77.1% | 93.1% | 0.062 |
| DR006421 | 15.3% | 15.5% | 21.7% | 72.9% | 97.0% | 0.022 |
| DR006444 | 15.3% | 21.1% | 28.6% | 63.4% | 105.7% | 0.02 |
| DR006445 | 15.8% | 16.7% | 26.2% | 71.1% | 109.2% | 0.023 |
| DR006446 | 14.5% | 14.0% | 25.5% | 64.1% | 104.9% | 0.019 |
| DR006447 | 14.9% | 15.9% | 35.3% | 70.0% | 71.3% | 0.055 |
| DR006459 | 21.2% | 22.1% | 42.7% | 75.7% | 82.1% | 0.066 |
| DR006560 | 18.6% | 22.5% | 38.1% | 75.5% | 88.9% | 0.048 |
| DR006566 | 16.1% | 14.9% | 21.8% | 54.8% | 84.7% | 0.013 |
| DR006618 | 15.8% | 15.7% | 32.5% | 71.6% | 84.4% | 0.036 |
| DR006621 | 15.0% | 16.7% | 31.0% | 76.4% | 94.1% | 0.036 |
| DR006652 | 15.3% | 20.0% | 30.3% | 75.7% | 107.1% | 0.031 |
| DR006653 | 18.6% | 19.2% | 35.3% | 74.8% | 97.6% | 0.038 |

(continued)

| | 10nM | 1nM | 0.1nM | 0.01nM | 0.001nM | 5-point IC50 value (nM) |
|---|---|---|---|---|---|---|
| DR006657 | 16.3% | 18.1% | 39.6% | 83.4% | 92.1% | 0.062 |
| DR006658 | 15.2% | 16.0% | 20.2% | 48.8% | 72.0% | 0.009 |

**Example 5 Activity Screening Experiment in Primary Hepatocyte (PMH) of Transgenic Mouse**

*Ad libitum feeding*

Primary liver cells from transgenic mice (purchased from Biocytogen Pharmaceuticals (Beijing) Co., Ltd.) were isolated, counted, and plated in 96-well plates at 100 μL/well ($1 \times 10^4$ cells/well).

Ad libitum feeding: 10 μL of the diluted compound (at the final concentration) was added to 90 μL of Opti-MEM, mixed thoroughly, then added to the corresponding wells and incubated in a 5% $CO_2$ incubator at 37 °C for 24 hours.

RNA extraction and PCR procedures were performed as described in Example 3, with the following primers used:

Table 11 Primer Information:

| Primer name | Sequence Information (5'-3') | SEQ ID NO | Fluorescent group |
|---|---|---|---|
| hMASP2-PF | TGAGCCTGTTTGTGGACTATC | 53 | / |
| hMASP2-PR | CTGCTGTGGTTCCACCTAAT | 54 | / |
| hMASP2-P | CGCACAACAGGAGGGCGTATATATGG | 55 | 5'6-FAM, 3'BHQ1 |
| mGAPDH-PF | CGGCAAATTCAACGGCACAG | 56 | |
| mGAPDH-PR | CCACGACATACTCAGCACCG | 57 | |
| mGAPDH-P | ACCATCTTCCAGGAGCGAGACCCCACT | 58 | 5'TET, 3'BHQ2 |

The target gene was hMASP2, and the endogenous reference was mGAPDH.

The compound was initially set at 20 nM and serially diluted 5-fold to generate 5 concentration points (20 nM, 4 nM, 0.8 nM, 0.16 nM, 0.032 nM) for 5-point $IC_{50}$ activity screening in transgenic mouse primary hepatocytes under free uptake conditions. The experimental results are presented in Table 12, where the percentage values represent the residual inhibition rate, calculated as described in Example 3.

Table 12 Results of PMH 5-point IC50 activity screening experiment

| Compound Number | 20nM | 4nM | 0.8nM | 0.16nM | 0.032nM | 5-point IC50 value (nM) |
|---|---|---|---|---|---|---|
| DR007112 | 18.6% | 14.8% | 33.4% | 47.8% | 66.8% | 0.153 |
| DR007113 | 9.2% | 8.6% | 26.8% | 34.0% | 67.8% | 0.071 |
| DR007114 | 32.3% | 28.1% | 27.7% | 37.5% | 67.2% | 0.136 |
| DR007115 | 39.6% | 32.2% | 21.0% | 68.6% | 78.3% | 0.189 |
| DR007116 | 27.2% | 22.1% | 29.9% | 52.9% | 87.6% | 0.182 |
| DR007117 | 25.5% | 10.9% | 32.1% | 29.8% | 65.7% | 0.053 |
| DR007118 | 21.9% | 16.6% | 15.3% | 25.0% | 55.4% | 0.097 |
| DR007119 | 16.7% | 16.8% | 30.7% | 74.6% | 89.5% | 0.385 |
| DR007120 | 16.6% | 8.5% | 17.1% | 23.1% | 44.6% | 0.022 |
| DR007121 | 25.4% | 22.7% | 36.3% | 50.0% | 82.0% | 0.169 |
| DR007122 | 40.9% | 53.4% | 60.9% | 92.5% | 132.0% | 1.958 |
| DR007123 | 13.3% | 34.0% | 35.8% | 55.3% | 86.3% | 0.269 |
| DR007124 | 25.1% | 25.1% | 37.1% | 50.1% | 63.5% | 0.182 |
| DR007125 | 12.3% | 17.9% | 30.2% | 49.5% | 66.3% | 0.153 |

(continued)

| Compound Number | 20nM | 4nM | 0.8nM | 0.16nM | 0.032nM | 5-point IC50 value (nM) |
|---|---|---|---|---|---|---|
| DR007126 | 26.9% | 32.0% | 26.1% | 27.5% | 55.7% | 0.036 |
| DR007127 | 22.9% | 20.6% | 17.7% | 53.4% | 94.8% | 0.163 |
| DR007128 | 9.0% | 17.5% | 24.9% | 35.5% | 46.6% | 0.021 |
| DR007129 | 13.9% | 23.2% | 13.3% | 44.9% | 71.0% | 0.158 |
| DR007130 | 17.9% | 25.8% | 18.8% | 26.3% | 55.2% | 0.091 |
| DR007102 | 34.1% | 48.5% | 50.1% | 83.7% | 95.6% | 0.934 |

**Example 6 Activity Screening in Human Primary Hepatocyte (PHH)**

Following the method described in Example 3, the compound was initially set at 10 nM and serially diluted 4-fold to generate 7 concentration points (10 nM, 2.5 nM, 0.625 nM, 0.156 nM, 0.039 nM, 0.00976 nM, 0.00244 nM) for siRNA compound activity screening. The experimental results are shown in Table 13.

Table 12 Results of 7-point IC50 screening experiment for human primary hepatocyte (PHH) activity

| Compound Number | 10nM | 2.5nM | 0.625n M | 0.156nM | 0.039nM | 0.00976nM | 0.00244nM | IC50 (nM) |
|---|---|---|---|---|---|---|---|---|
| DR009198 | 27.1% | 16.9% | 14.8% | 42.0% | 47.0% | 74.0% | 94.9% | 0.0425 |
| DR009427 | 35.8% | 23.8% | 25.5% | 26.7% | 21.2% | 57.7% | 58.5% | 0.0080 |
| DR009428 | 24.2% | 17.5% | 26.2% | 30.7% | 31.4% | 89.4% | 76.6% | 0.0319 |
| DR009197 | 23.9% | 44.4% | 40.5% | 43.1% | 43.5% | 92.6% | 89.3% | 0.0651 |
| DR007118 | 28.7% | 26.2% | 30.9% | 23.4% | 36.4% | 67.6% | 75.5% | 0.0201 |

**Example 7 Pharmacodynamic Activity Screening in Mice with AAV**

AAV virus carrying the human MASP2 gene fragment (Obio Technology (Shanghai) Corp., Ltd., H27393, pAAV-TBG-hMASP2-tWPA) was injected into mice via the tail vein at a viral dose of 1 x 10^11 vg. The day of virus injection was designated as day -14 of modeling. Mouse serum was collected on D-4 and D0, and MASP2 protein (Abcam, ab278121) was detected by ELISA to serve as the baseline for the administration groups, with 4 mice per group. On Day 0, siRNA drug or normal saline was administered subcutaneously at a dose of 3 mg/kg. Serum was collected at each time point post-administration (D7, D14, D21, D28, D42, D49) to detect MASP2 protein levels. The experimental results are shown in Table 13.

Table 13 Results of pharmacodynamic activity screening experiments in mice with AAV (values in the table are all percentages from baseline)

| compound | Before administration | | D7 | | D14 | | D21 | | D28 | | D42 | | D49 | |
|---|---|---|---|---|---|---|---|---|---|---|---|---|---|---|
| | Mean value | SD | Mean value | SD | Mean value | SD | Mean value | SD | Mean value | SD | Mean value | SD | Mean value | SD |
| normal saline | 110.53 | 24.51 | 125.85 | 10.23 | 99.33 | 19.27 | 85.73 | 14.77 | 71.03 | 25.37 | 70.78 | 6.90 | 80.90 | 3.50 |
| DR007102 | 106.43 | 8.29 | 37.40 | 13.38 | 21.63 | 9.15 | 23.47 | 19.44 | 29.33 | 12.85 | 38.37 | 20.47 | 45.10 | 11.40 |
| DR007112 | 114.63 | 53.20 | 37.65 | 28.23 | 41.50 | 34.02 | 32.90 | 26.30 | 26.85 | 19.15 | 39.70 | 21.08 | 49.30 | 26.10 |
| DR007113 | 91.70 | 43.32 | 7.05 | 4.74 | 4.40 | 2.37 | 7.30 | 6.26 | 13.48 | 9.90 | 44.95 | 15.68 | 62.40 | 12.10 |
| DR007114 | 85.58 | 48.59 | 18.43 | 10.75 | 13.45 | 4.50 | 13.88 | 4.32 | 21.63 | 6.69 | 31.85 | 13.52 | 37.70 | 11.60 |
| DR007115 | 88.00 | 26.85 | 32.05 | 17.26 | 22.63 | 9.22 | 20.43 | 13.25 | 21.53 | 13.39 | 39.98 | 21.01 | 47.20 | 22.70 |
| DR007116 | 98.90 | 37.47 | 39.73 | 28.71 | 46.63 | 20.95 | 68.88 | 30.97 | 65.30 | 22.81 | 76.60 | 9.72 | 74.70 | 8.10 |
| DR007117 | 112.85 | 24.19 | 37.98 | 19.79 | 33.33 | 3.36 | 29.63 | 4.99 | 34.73 | 9.46 | 60.53 | 16.29 | 62.30 | 6.70 |
| DR007118 | 96.95 | 19.17 | 14.45 | 12.03 | 9.30 | 7.59 | 10.53 | 9.52 | 15.45 | 15.68 | 26.55 | 13.11 | 35.40 | 12.00 |
| DR007119 | 103.03 | 32.98 | 113.88 | 12.34 | 85.70 | 18.45 | 88.53 | 17.55 | 89.23 | 10.98 | 81.25 | 3.13 | 95.00 | 18.70 |
| DR007120 | 95.90 | 32.11 | 2.20 | 1.99 | 2.63 | 1.14 | 4.43 | 2.69 | 6.73 | 4.52 | 34.10 | 21.40 | 48.10 | 23.50 |
| DR007121 | 99.35 | 29.75 | 19.28 | 15.87 | 12.85 | 3.26 | 23.33 | 9.34 | 21.40 | 4.46 | 41.63 | 13.74 | 43.40 | 12.00 |
| DR007123 | 89.23 | 24.53 | 61.23 | 24.49 | 67.33 | 6.64 | 79.00 | 20.16 | 77.33 | 14.91 | 81.08 | 12.17 | 88.70 | 19.00 |
| DR007124 | 101.38 | 26.42 | 8.98 | 1.58 | 4.20 | 0.90 | 4.78 | 1.10 | 5.40 | 0.99 | 10.43 | 6.30 | 14.90 | 5.70 |
| DR007125 | 109.95 | 6.85 | 13.38 | 6.07 | 11.68 | 3.09 | 22.88 | 10.95 | 27.80 | 8.08 | 53.98 | 14.82 | 70.80 | 24.30 |
| DR007126 | 106.85 | 14.24 | 4.75 | 4.54 | 3.33 | 2.29 | 6.13 | 2.46 | 12.30 | 6.18 | 40.65 | 3.22 | 56.10 | 15.50 |
| DR007127 | 96.18 | 11.57 | 11.10 | 5.79 | 7.40 | 2.07 | 9.28 | 3.75 | 12.20 | 7.36 | 15.05 | 11.76 | 25.20 | 19.10 |
| DR007128 | 95.48 | 29.51 | 16.05 | 12.09 | 5.03 | 2.60 | 9.75 | 5.80 | 12.45 | 6.67 | 46.75 | 15.67 | 55.00 | 19.20 |
| DR007129 | 101.75 | 23.29 | 5.68 | 3.30 | 4.45 | 1.80 | 7.63 | 4.51 | 11.45 | 4.82 | 28.38 | 9.15 | 45.50 | 13.50 |
| DR007130 | 97.03 | 32.92 | 5.95 | 3.56 | 6.55 | 3.23 | 10.73 | 5.99 | 15.28 | 7.67 | 38.58 | 11.82 | 37.40 | 12.10 |

**Example 8 Screening of Pharmacodynamic Activity in Tg Mice**

Fifty-five MASP2 transgenic mice (Biocytogen Pharmaceuticals (Beijing) Co., Ltd.) were grouped based on their baseline (D0) MASP2 protein levels. Each group contained five mice, in which the DR007102-injected group had three mice. On Day 0, siRNA drug or normal saline was administered subcutaneously at a dose of 3 mg/kg. Serum was collected at each post-administration time point (D7, D14, D21, D28, D42, D56) to determine MASP2 protein levels. The experimental results are presented in Table 14.

Table 14 Results of pharmacodynamic activity screening experiments in Tg mice (values in the table are absolute values of MASP2)

| Compounds | D0 | | D7 | | D14 | | D21 | | D28 | | D42 | | D56 | |
|---|---|---|---|---|---|---|---|---|---|---|---|---|---|---|
| | mean value | SD | mean value | SD | mean value | SD | mean value | SD | mean value | SD | mean value | SD | mean value | SD |
| Normal saline | 72.93 | 6.99 | 79.83 | 10.98 | 89.77 | 6.44 | 66.08 | 18.88 | 54.18 | 8.33 | 68.67 | 11.44 | 56.28 | 6.26 |
| DR007113 | 63.43 | 13.28 | 2.79 | 2.75 | 5.38 | 1.58 | 9.00 | 2.68 | 11.66 | 2.43 | 33.82 | 9.19 | 43.49 | 8.60 |
| DR007118 | 66.23 | 2.19 | 3.71 | 1.61 | 6.22 | 2.13 | 8.59 | 2.04 | 8.60 | 2.46 | 17.30 | 4.20 | 22.39 | 5.58 |
| DR007120 | 64.86 | 7.25 | 6.71 | 2.28 | 1.87 | 1.45 | 13.63 | 2.80 | 14.66 | 2.21 | 36.50 | 5.96 | 46.08 | 3.80 |
| DR007121 | 52.68 | 10.44 | 12.28 | 3.74 | 15.05 | 4.44 | 20.44 | 6.80 | 19.11 | 2.73 | 29.97 | 8.90 | 35.78 | 6.30 |
| DR007124 | 63.29 | 11.03 | 7.59 | 2.61 | 13.63 | 1.81 | 14.21 | 3.98 | 11.61 | 2.01 | 19.38 | 2.67 | 23.42 | 7.25 |
| DR007126 | 57.94 | 8.81 | 5.73 | 0.84 | 8.54 | 1.57 | 13.56 | 1.07 | 16.59 | 1.98 | 33.84 | 4.62 | 49.95 | 7.00 |
| DR007127 | 54.71 | 7.06 | 9.46 | 1.17 | 11.90 | 2.14 | 14.70 | 1.20 | 13.16 | 1.55 | 25.36 | 3.53 | 29.85 | 4.77 |
| DR007128 | 61.03 | 3.31 | 5.09 | 1.05 | 9.66 | 3.18 | 11.91 | 1.45 | 13.76 | 2.71 | 29.90 | 5.14 | 32.88 | 4.99 |
| DR007129 | 60.04 | 11.15 | 7.90 | 3.06 | 12.80 | 2.55 | 12.36 | 2.14 | 18.86 | 5.71 | 34.35 | 4.23 | 48.07 | 10.86 |
| DR007130 | 54.06 | 5.55 | 8.67 | 3.12 | 15.78 | 5.17 | 14.16 | 2.71 | 20.55 | 4.26 | 40.12 | 8.60 | 50.58 | 8.74 |
| DR007102 | 77.26 | 14.24 | 26.63 | 9.52 | 22.02 | 7.43 | 14.51 | 3.38 | 31.22 | 3.18 | 51.89 | 5.63 | 54.08 | 6.85 |

## Claims

1. A double-stranded nucleotide (dsRNA) for inhibiting the expression of mannan-binding lectin associated serine protease 2 (MASP2) in a cell, the dsRNA comprises a sense strand and an antisense strand that form a double-stranded region, wherein the sense strand and the antisense strand are each independently 15-30 nucleotides in length, and the antisense strand comprises a nucleotide sequence of at least 15 contiguous nucleotides from the nucleotide sequence set forth in any one of SEQ ID NOs: 22-40.

2. The dsRNA of claim 1, wherein the sense strand comprises a nucleotide sequence of at least 15 contiguous nucleotides from the nucleotide sequence set forth in any one of SEQ ID NOs: 2-20.

3. The dsRNA of claim 1 or 2, wherein the dsRNA is an siRNA.

4. The dsRNA of any one of claims 1-3, wherein the antisense strand is 15-27 nucleotides, preferably 17-25 nucleotides, more preferably 18-23 nucleotides, more preferably 19-22 nucleotides, or most preferably 21 amino acids in length.

5. The dsRNA of any one of claims 1-4, wherein the antisense strand comprises a nucleotide sequence of at least 16 contiguous nucleotides, a nucleotide sequence of at least 17 contiguous nucleotides, a nucleotide sequence of at least 18 contiguous nucleotides, a nucleotide sequence of at least 19 contiguous nucleotides, or a nucleotide sequence of at least 20 contiguous nucleotides from the nucleotide sequence set forth in any one of SEQ ID NOs: 22-40, and preferably the antisense strand comprises the nucleotide sequence set forth in any one of SEQ ID NOs: 22-40.

6. The dsRNA of any one of claims 1-5, wherein the sense strand comprises a nucleotide sequence of at least 16 contiguous nucleotides, a nucleotide sequence of at least 17 contiguous nucleotides, or a nucleotide sequence of at least 18 contiguous nucleotides from the nucleotide sequence set forth in any one of SEQ ID NOs: 2-20, and preferably the sense strand comprises the nucleotide sequence set forth in any one of SEQ ID NOs: 2-20.

7. The dsRNA of any one of claims 1-6, wherein the siRNA comprises any of the following paired of sense strand sequences and antisense strand sequences:

| | | | | |
|---|---|---|---|---|
| (1) | sense strand: | SEQ ID NO: 2, | antisense strand: | SEQ ID NO: 22; |
| (2) | sense strand: | SEQ ID NO: 3, | antisense strand: | SEQ ID NO: 23; |
| (3) | sense strand: | SEQ ID NO: 4, | antisense strand: | SEQ ID NO: 24; |
| (4) | sense strand: | SEQ ID NO: 5, | antisense strand: | SEQ ID NO: 25; |
| (5) | sense strand: | SEQ ID NO: 6, | antisense strand: | SEQ ID NO: 26; |
| (6) | sense strand: | SEQ ID NO: 7, | antisense strand: | SEQ ID NO: 27; |
| (7) | sense strand: | SEQ ID NO: 8, | antisense strand: | SEQ ID NO: 28; |
| (8) | sense strand: | SEQ ID NO: 9, | antisense strand: | SEQ ID NO: 29; |
| (9) | sense strand: | SEQ ID NO: 10, | antisense strand: | SEQ ID NO: 30; |
| (10) | sense strand: | SEQ ID NO: 11, | antisense strand: | SEQ ID NO: 31; |
| (11) | sense strand: | SEQ ID NO: 12, | antisense strand: | SEQ ID NO: 32; |
| (12) | sense strand: | SEQ ID NO: 13, | antisense strand: | SEQ ID NO: 33; |
| (13) | sense strand: | SEQ ID NO: 14, | antisense strand: | SEQ ID NO: 34; |
| (14) | sense strand: | SEQ ID NO: 15, | antisense strand: | SEQ ID NO: 35; |
| (15) | sense strand: | SEQ ID NO: 16, | antisense strand: | SEQ ID NO: 36; |
| (16) | sense strand: | SEQ ID NO: 17, | antisense strand: | SEQ ID NO: 37; |
| (17) | sense strand: | SEQ ID NO: 18, | antisense strand: | SEQ ID NO: 38; |
| (18) | sense strand: | SEQ ID NO: 19, | antisense strand: | SEQ ID NO: 39; and |
| (19) | sense strand: | SEQ ID NO: 20, | antisense strand: | SEQ ID NO: 40. |

8. The dsRNA of any one of claims 1-7, wherein substantially all nucleotides of the sense strand and substantially all nucleotides of the antisense strand are modified nucleotides, or all nucleotides of the sense strand and all nucleotides of the antisense strand are modified nucleotides.

**9.** The dsRNA of claim 8, wherein each of the sense strand and the antisense strand independently comprises one or more nucleotide modifications selected from the group consisting of a 2'-O-methyl modified nucleotide, a 2'-fluoro modified nucleotide, a 2'-deoxy modified nucleotide, and a phosphorothioate internucleotide linkage modification.

**10.** The dsRNA of claim 8, wherein the sense strand and/or the antisense strand comprises an SCP-modified nucleotide.

**11.** The dsRNA of any one of claims 8-10, wherein the 3' end and/or 5' end of the sense strand and/or the antisense strand comprises 1-5 phosphorothioate internucleotide linkages, preferably 2-3 phosphorothioate internucleotide linkages.

**12.** The dsRNA of any one of claims 1-11, wherein the antisense strand has a length of 21 nucleotides and has

(i) 2'-O-methyl modified nucleotides at positions 1, 3, 5, 7, 9, 11, 13, 15, 17, 19, and 21, and 2'-fluoro modified nucleotides at positions 2, 4, 6, 8, 10, 12, 14, 16, 18, and 20 (numbered from the 5' end); and/or
(ii) phosphorothioate internucleotide linkages between nucleotide positions 1 and 2, between nucleotide positions 2 and 3, between nucleotide positions 19 and 20, and between nucleotide positions 20 and 21 (numbered from the 5' end).

**13.** The dsRNA of any one of claims 1-11, wherein the antisense strand has a length of 21 nucleotides and has

(i) an SCP modification at position 1 (numbered from the 5' end);
(ii) 2'-fluoro modifications at positions 2, 4, 6, 8, 10, 12, 14, 16, and 18 (numbered from the 5' end);
(iii) 2'-O-methyl modifications at positions 3, 5, 7, 9, 11, 13, 15, 17, 19, 20, and 21 (numbered from the 5' end); and/or
(iv) phosphorothioate internucleotide linkages between nucleotide positions 1 and 2, between nucleotide positions 2 and 3, between nucleotide positions 19 and 20, and between nucleotide positions 20 and 21 (numbered from the 5' end).

**14.** The dsRNA of any one of claims 1-11, wherein the antisense strand has a length of 21 nucleotides and has

(i) 2'-deoxy modifications at positions 2, 5, 7, and 12 (numbered from the 5' end);
(ii) an SCP modification at position 1 (numbered from the 5' end);
(iii) a 2'-fluoro modification at position 14 (numbered from the 5' end);
(iv) 2'-O-methyl modifications at positions 3, 4, 6, 8, 9, 10, 11, 13, 15, 16, 17, 18, 19, 20, and 21 (numbered from the 5' end); and/or
(v) phosphorothioate internucleotide linkages between nucleotide positions 1 and 2, between nucleotide positions 2 and 3, between nucleotide positions 19 and 20, and between nucleotide positions 20 and 21 (numbered from the 5' end).

**15.** The dsRNA of any one of claims 1-11, wherein the sense strand has a length of 19 nucleotides and has:

(i) 2'-O-methyl modified nucleotides at positions 1 to 6, and 10 to 19, and 2'-fluoro modified nucleotides at positions 7-9 (numbered from the 5' end); and/or
(ii) phosphorothioate internucleotide linkages between nucleotide positions 1 and 2, between nucleotide positions 2 and 3, and between nucleotide positions 18 and 19 (numbered from the 5' end).

**16.** The dsRNA of any one of claims 1-11, wherein the sense strand has a length of 19 nucleotides and has:

(i) 2'-O-methyl modified nucleotides at positions 1 to 6, and 10 to 19, and 2'-fluoro modified nucleotides at positions 7-9 (numbered from the 5' end); and/or
(ii) phosphorothioate internucleotide linkages between nucleotide positions 1 and 2, between nucleotide positions 2 and 3, between nucleotide positions 17 and 18, and between nucleotide positions 18 and 19 (numbered from the 5' end).

**17.** The dsRNA of any one of claims 1-14 and 16, wherein the antisense strand is selected from any one of the modified nucleotide sequences set forth in SEQ ID NOs: 100-122, and/or the sense strand is selected from any one of the modified nucleotide sequences set forth in SEQ ID NOs: 80-98.

**18.** The dsRNA of any one of claim 17, wherein the dsRNA comprises any of the following paired of modified sense strand

sequences and modified antisense strand sequences

| | | | |
|---|---|---|---|
| sense strand: | SEQ ID NO: 80, | antisense strand: | SEQ ID NO: 100; |
| sense strand: | SEQ ID NO: 81, | antisense strand: | SEQ ID NO: 101; |
| sense strand: | SEQ ID NO: 82, | antisense strand: | SEQ ID NO: 102; |
| sense strand: | SEQ ID NO: 83, | antisense strand: | SEQ ID NO: 103; |
| sense strand: | SEQ ID NO: 84, | antisense strand: | SEQ ID NO: 104; |
| sense strand: | SEQ ID NO: 85, | antisense strand: | SEQ ID NO: 105; |
| sense strand: | SEQ ID NO: 86, | antisense strand: | SEQ ID NO: 106; |
| sense strand: | SEQ ID NO: 87, | antisense strand: | SEQ ID NO: 107; |
| sense strand: | SEQ ID NO: 88, | antisense strand: | SEQ ID NO: 108; |
| sense strand: | SEQ ID NO: 89, | antisense strand: | SEQ ID NO: 109; |
| sense strand: | SEQ ID NO: 90, | antisense strand: | SEQ ID NO: 110; |
| sense strand: | SEQ ID NO: 91, | antisense strand: | SEQ ID NO: 111; |
| sense strand: | SEQ ID NO: 92, | antisense strand: | SEQ ID NO: 112; |
| sense strand: | SEQ ID NO: 93, | antisense strand: | SEQ ID NO: 113; |
| sense strand: | SEQ ID NO: 94, | antisense strand: | SEQ ID NO: 114; |
| sense strand: | SEQ ID NO: 95, | antisense strand: | SEQ ID NO: 115; |
| sense strand: | SEQ ID NO: 96, | antisense strand: | SEQ ID NO: 116; |
| sense strand: | SEQ ID NO: 97, | antisense strand: | SEQ ID NO: 117; and |
| sense strand: | SEQ ID NO: 98, | antisense strand: | SEQ ID NO: 118. |

**19.** The dsRNA of any one of claims 1-15, wherein the dsRNA is further conjugated to a ligand moiety comprising N-acetylgalactosamine, and preferably the sense strand is conjugated to the ligand moiety.

**20.** The dsRNA of claim 19, wherein the ligand is GL6 having the following structure:

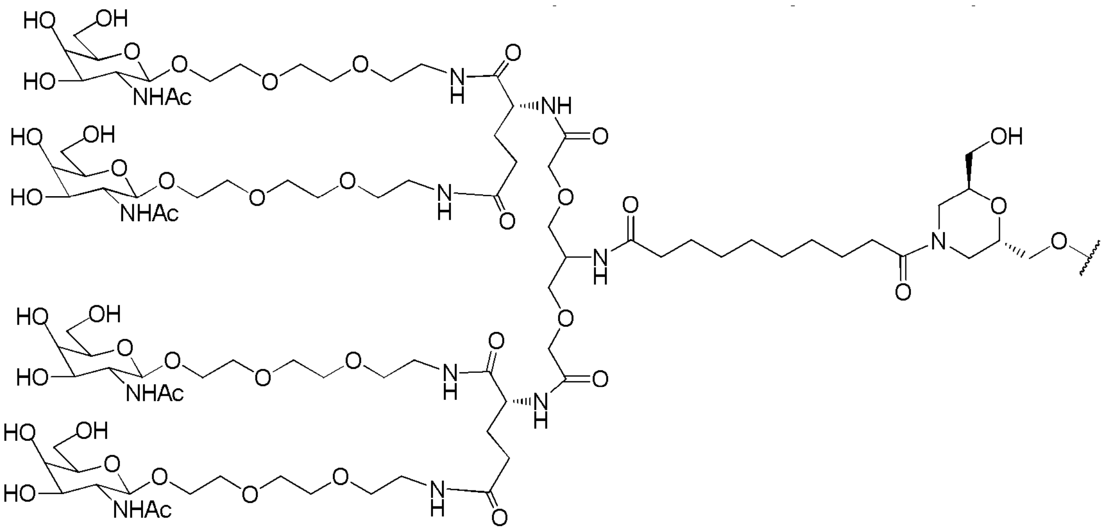

wherein

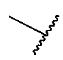

represents the point of attachment to the sense strand (preferably the 3' end of the sense strand) of the dsRNA via a phosphate group or a phosphorothioate group.

**21.** The dsRNA of claim 20, wherein the antisense strand comprises a sequence selected from any one of the modified nucleotide sequences set forth in SEQ ID NOs: 100-122, and/or the sense strand comprises a sequence selected from

any one of the modified nucleotide sequences set forth in SEQ ID NOs: 60-78.

**22.** The dsRNA of claim 21, wherein the dsRNA comprises any of the following paired of modified sense strand sequences and modified antisense strand sequences

| | | | |
|---|---|---|---|
| sense strand: | SEQ ID NO: 60, | antisense strand: | SEQ ID NO: 100; |
| sense strand: | SEQ ID NO: 61, | antisense strand: | SEQ ID NO: 101; |
| sense strand: | SEQ ID NO: 62, | antisense strand: | SEQ ID NO: 102; |
| sense strand: | SEQ ID NO: 63, | antisense strand: | SEQ ID NO: 103; |
| sense strand: | SEQ ID NO: 64, | antisense strand: | SEQ ID NO: 104; |
| sense strand: | SEQ ID NO: 65, | antisense strand: | SEQ ID NO: 105; |
| sense strand: | SEQ ID NO: 66, | antisense strand: | SEQ ID NO: 106; |
| sense strand: | SEQ ID NO: 67, | antisense strand: | SEQ ID NO: 107; |
| sense strand: | SEQ ID NO: 68, | antisense strand: | SEQ ID NO: 108; |
| sense strand: | SEQ ID NO: 69, | antisense strand: | SEQ ID NO: 109; |
| sense strand: | SEQ ID NO: 70, | antisense strand: | SEQ ID NO: 110; |
| sense strand: | SEQ ID NO: 71, | antisense strand: | SEQ ID NO: 111; |
| sense strand: | SEQ ID NO: 72, | antisense strand: | SEQ ID NO: 112; |
| sense strand: | SEQ ID NO: 73, | antisense strand: | SEQ ID NO: 113; |
| sense strand: | SEQ ID NO: 74, | antisense strand: | SEQ ID NO: 114; |
| sense strand: | SEQ ID NO: 75, | antisense strand: | SEQ ID NO: 115; |
| sense strand: | SEQ ID NO: 76, | antisense strand: | SEQ ID NO: 116; |
| sense strand: | SEQ ID NO: 77, | antisense strand: | SEQ ID NO: 117; |
| sense strand: | SEQ ID NO: 78, | antisense strand: | SEQ ID NO: 118; |
| sense strand: | SEQ ID NO: 66, | antisense strand: | SEQ ID NO: 119; |
| sense strand: | SEQ ID NO: 66, | antisense strand: | SEQ ID NO: 120; |
| sense strand: | SEQ ID NO: 66, | antisense strand: | SEQ ID NO: 121; and |
| sense strand: | SEQ ID NO: 66, | antisense strand: | SEQ ID NO: 122. |

**23.** A cell comprising the dsRNA of any one of claims 1-22.

**24.** A pharmaceutical composition comprising the dsRNA of any one of claims 1-22, or the cell of claim 23, and optionally a pharmaceutically acceptable carrier or excipient.

**25.** A kit comprising the dsRNA of any one of claims 1-22, the cell of claim 23, or the pharmaceutical composition of claim 24.

**26.** A method of treating a disease or disorder benefiting from reduced expression of mannan-binding lectin associated serine protease 2 (MASP2) in a subject, comprising a step of administering to the subject the dsRNA of any one of claims 1-22, the cell of claim 23, or the pharmaceutical composition of claim 24.

**27.** A method of preventing at least one symptom in a subject that has a disease or disorder benefiting from reduced expression of mannan-binding lectin associated serine protease 2 (MASP2), comprising a step of administering to the subject the dsRNA of any one of claims 1-22, the cell of claim 23, or the pharmaceutical composition of claim 24.

**28.** The method of claim 26 or 27, wherein the disease or disorder benefiting from reduced expression of mannan-binding lectin associated serine protease 2 (MASP2) is a MASP2-mediated or MASP2-related disease.

**29.** The method of claim 28, wherein the MASP2-mediated or MASP2-related disease is selected from the group consisting of arthritis, IgA nephropathy, thrombotic microangiopathy, venous embolism, diabetic nephropathy, and membranous nephropathy.

**30.** A method of reducing the level of mannan-binding lectin associated serine protease 2 (MASP2) in a subject, comprising a step of administering to the subject the dsRNA of any one of claims 1-22, the cell of claim 23, or the pharmaceutical composition of claim 24.

**INTERNATIONAL SEARCH REPORT**

International application No.
**PCT/CN2024/092668**

**A. CLASSIFICATION OF SUBJECT MATTER**

C12N 15/113(2010.01)i; A61K 48/00(2006.01)i; C12N 15/113(2010.01)i; C12N 15/12(2006.01)i

According to International Patent Classification (IPC) or to both national classification and IPC

**B. FIELDS SEARCHED**

Minimum documentation searched (classification system followed by classification symbols)

IPC: C12N A61K

Documentation searched other than minimum documentation to the extent that such documents are included in the fields searched

Electronic data base consulted during the international search (name of data base and, where practicable, search terms used)

CNTXT, ENTXT, ENTXTC, VEN, CNKI, 万方数据库, WANFANG DATABASE, pubmed, ISI web of science, stn: 甘露聚糖结合凝集素相关丝氨酸蛋白酶2, 核苷酸, 配体, 四, 四价, 乙酰基半乳糖胺, four, GalNAc, GL6, MASP2, quadruple, rna, scp, shRNA, sirna, siRNA, tetraGalNAc, tetravalent, , masp-2, tetra+, SEQ ID NOs: 1-40

**C. DOCUMENTS CONSIDERED TO BE RELEVANT**

| Category* | Citation of document, with indication, where appropriate, of the relevant passages | Relevant to claim No. |
|---|---|---|
| X | US 2023125933 A1 (ALNYLAM PHARMACEUTICALS, INC.) 27 April 2023 (2023-04-27) claims 1-70 | 1-19 |
| X | HOLERS, V.M. et al. "Key Components of the Complement Lectin Pathway Are Not Only Required for the Development of Inflammatory Arthritis but Also Regulate the Transcription of Factor D" *Frontiers in Immunology*, Vol. vol. 111, 21 February 2020 (2020-02-21), abstract | 1-19 |
| X | CN 110506116 A (KYOWA HAKKO KIRIN CO., LTD.) 26 November 2019 (2019-11-26) claims 1-16 | 1-19 |
| Y | US 2023125933 A1 (ALNYLAM PHARMACEUTICALS, INC.) 27 April 2023 (2023-04-27) claims 1-70 | 20-30 |
| Y | CN 112055598 A (DICERNA PHARMACEUTICALS, INC.) 08 December 2020 (2020-12-08) claims 1-43 | 20-30 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 August 2024** | **12 August 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2024/092668

C. DOCUMENTS CONSIDERED TO BE RELEVANT

| Category* | Citation of document, with indication, where appropriate, of the relevant passages | Relevant to claim No. |
|---|---|---|
| Y | US 2022186229 A1 (DICEMA PHARMACEUTICALS, INC.) 16 June 2022 (2022-06-16) 20-30 | claims 1-51 |
| A | WO 2009073809 A2 (ALNYLAM PHARMACEUTICALS, INC. et al.) 11 June 2009 (2009-06-11) claims 1-20 | 1-30 |
| A | US 2023078200 A1 (AMGEN INC.) 16 March 2023 (2023-03-16) claims 1-63 | 1-30 |
| A | US 2020032270 A1 (KYOWA KIRIN CO., LTD.) 30 January 2020 (2020-01-30) claims 1-16 | 1-30 |
| A | ULASHCHIK, E.A. et al. "Synthesis of GalNAc-Oligonucleotide Conjugates Using GalNAc Phosphoramidite and Triple-GalNAc CPG Solid Support" *Methods in Molecular Biology,* Vol. vol. 2282, 01 January 2021 (2021-01-01), abstract | 1-30 |
| A | CHEN, C.M. et al. "Chemical Modifications of Nucleic Acid Drugs and their Delivery Systems for Gene-based Therapy" *Medicinal Research Reviews,* 05 January 2018 (2018-01-05), abstract | 1-30 |

Form PCT/ISA/210 (second sheet) (July 2022)

**INTERNATIONAL SEARCH REPORT**

International application No.
**PCT/CN2024/092668**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/092668**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **26-30**
because they relate to subject matter not required to be searched by this Authority, namely:

Claims 26-30 relate to a method for treatment of a disease in a living human or animal body, which falls within methods for treatment of diseases as defined in PCT Rule 39.1(iv). Therefore, a search is carried out on the basis of the corresponding pharmaceutical use of dsRNA.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/092668**

| Patent document cited in search report | | | Publication date (day/month/year) | Patent family member(s) | | | Publication date (day/month/year) |
|---|---|---|---|---|---|---|---|
| US | 2023125933 | A1 | 27 April 2023 | WO | 2021168148 | A1 | 26 August 2021 |
| | | | | EP | 4107165 | A1 | 28 December 2022 |
| CN | 110506116 | A | 26 November 2019 | CA | 3055715 | A1 | 13 September 2018 |
| | | | | US | 2020032270 | A1 | 30 January 2020 |
| | | | | TW | 201837172 | A | 16 October 2018 |
| | | | | EP | 3594345 | A1 | 15 January 2020 |
| | | | | EP | 3594345 | A4 | 19 May 2021 |
| | | | | AU | 2018232367 | A1 | 03 October 2019 |
| | | | | KR | 20190128674 | A | 18 November 2019 |
| | | | | JPWO | 2018164186 | A1 | 09 January 2020 |
| | | | | WO | 2018164186 | A1 | 13 September 2018 |
| CN | 112055598 | A | 08 December 2020 | MX | 2020009072 | A | 08 October 2020 |
| | | | | WO | 2019168687 | A1 | 06 September 2019 |
| | | | | JP | 2021517826 | A | 29 July 2021 |
| | | | | JP | 7453921 | B2 | 21 March 2024 |
| | | | | US | 2023365974 | A1 | 16 November 2023 |
| | | | | IL | 277058 | A | 29 October 2020 |
| | | | | EP | 3740247 | A1 | 25 November 2020 |
| | | | | EP | 3740247 | A4 | 08 June 2022 |
| | | | | KR | 20200127211 | A | 10 November 2020 |
| | | | | US | 2021062197 | A1 | 04 March 2021 |
| | | | | US | 11572562 | B2 | 07 February 2023 |
| | | | | CA | 3092092 | A1 | 06 September 2019 |
| US | 2022186229 | A1 | 16 June 2022 | AU | 2020221892 | A1 | 19 August 2021 |
| | | | | KR | 20210132661 | A | 04 November 2021 |
| | | | | WO | 2020167593 | A1 | 20 August 2020 |
| | | | | JP | 2022520653 | A | 31 March 2022 |
| | | | | BR | 112021015651 | A2 | 05 October 2021 |
| | | | | EP | 3908661 | A1 | 17 November 2021 |
| | | | | IL | 285367 | A | 30 September 2021 |
| | | | | CA | 3128059 | A1 | 20 August 2020 |
| | | | | CL | 2021002120 | A1 | 01 April 2022 |
| | | | | MX | 2021009754 | A | 08 September 2021 |
| | | | | SG | 11202108532 | RA | 29 September 2021 |
| WO | 2009073809 | A2 | 11 June 2009 | CA | 2708153 | A1 | 11 June 2009 |
| | | | | CA | 2708153 | C | 26 September 2017 |
| | | | | EP | 3705125 | A1 | 09 September 2020 |
| | | | | EP | 3705125 | B1 | 05 July 2023 |
| | | | | AU | 2008333811 | A1 | 11 June 2009 |
| | | | | AU | 2008333811 | B2 | 01 May 2014 |
| | | | | US | 2012136042 | A1 | 31 May 2012 |
| | | | | US | 8450467 | B2 | 28 May 2013 |
| | | | | EP | 3141265 | A1 | 15 March 2017 |
| | | | | JP | 2021080299 | A | 27 May 2021 |
| | | | | JP | 2023010881 | A | 20 January 2023 |
| | | | | EP | 4223299 | A2 | 09 August 2023 |
| | | | | EP | 4223299 | A3 | 30 August 2023 |
| | | | | US | 2019184018 | A1 | 20 June 2019 |
| | | | | US | 10806791 | B2 | 20 October 2020 |
| | | | | US | 2013178512 | A1 | 11 July 2013 |

Form PCT/ISA/210 (patent family annex) (July 2022)

**INTERNATIONAL SEARCH REPORT**
**Information on patent family members**

International application No.
**PCT/CN2024/092668**

| Patent document cited in search report | Publication date (day/month/year) | Patent family member(s) | | | Publication date (day/month/year) |
|---|---|---|---|---|---|
| | | US | 8828956 | B2 | 09 September 2014 |
| | | CA | 3043911 | A1 | 02 July 2009 |
| | | JP | 2011505426 | A | 24 February 2011 |
| | | JP | 5635412 | B2 | 03 December 2014 |
| | | US | 2018326070 | A1 | 15 November 2018 |
| | | AU | 2008340355 | A1 | 02 July 2009 |
| | | AU | 2008340355 | B2 | 22 January 2015 |
| | | JP | 2022190116 | A | 22 December 2022 |
| | | US | 2014179761 | A1 | 26 June 2014 |
| | | US | 2016375137 | A9 | 29 December 2016 |
| | | US | 9814777 | B2 | 14 November 2017 |
| | | EP | 4321177 | A2 | 14 February 2024 |
| | | EP | 4321177 | A3 | 08 May 2024 |
| | | EP | 3156077 | A1 | 19 April 2017 |
| | | EP | 3156077 | B1 | 09 March 2022 |
| | | CA | 3146103 | A1 | 11 June 2009 |
| | | US | 2022143189 | A1 | 12 May 2022 |
| | | US | 11666653 | B2 | 06 June 2023 |
| | | US | 2015119445 | A1 | 30 April 2015 |
| | | US | 9352048 | B2 | 31 May 2016 |
| | | JP | 2016034974 | A | 17 March 2016 |
| | | JP | 6215284 | B2 | 18 October 2017 |
| | | WO | 2009082607 | A2 | 02 July 2009 |
| | | WO | 2009082607 | A3 | 17 December 2009 |
| | | WO | 2009082607 | A8 | 28 January 2010 |
| | | CA | 2930393 | A1 | 11 June 2009 |
| | | CA | 2930393 | C | 29 November 2022 |
| | | EP | 2229186 | A2 | 22 September 2010 |
| | | CA | 2708173 | A1 | 02 July 2009 |
| | | CA | 2708173 | C | 02 February 2016 |
| | | EP | 2231195 | A2 | 29 September 2010 |
| | | EP | 2231195 | B1 | 29 March 2017 |
| | | US | 2015119444 | A1 | 30 April 2015 |
| | | US | 9370582 | B2 | 21 June 2016 |
| | | CA | 2910760 | A1 | 02 July 2009 |
| | | CA | 2910760 | C | 09 July 2019 |
| | | JP | 2019023241 | A | 14 February 2019 |
| | | US | 2015011615 | A1 | 08 January 2015 |
| | | US | 9370581 | B2 | 21 June 2016 |
| | | JP | 2019147846 | A | 05 September 2019 |
| | | JP | 6850827 | B2 | 31 March 2021 |
| | | EP | 4074344 | A1 | 19 October 2022 |
| | | US | 2009239814 | A1 | 24 September 2009 |
| | | US | 8106022 | B2 | 31 January 2012 |
| | | JP | 2014139232 | A | 31 July 2014 |
| | | JP | 5843914 | B2 | 13 January 2016 |
| | | JP | 2011505425 | A | 24 February 2011 |
| | | JP | 5519523 | B2 | 11 June 2014 |
| | | WO | 2009073809 | A3 | 30 December 2009 |
| | | JP | 2021020928 | A | 18 February 2021 |

Form PCT/ISA/210 (patent family annex) (July 2022)

**INTERNATIONAL SEARCH REPORT**
**Information on patent family members**

International application No.
**PCT/CN2024/092668**

| Patent document cited in search report | Publication date (day/month/year) | Patent family member(s) | Publication date (day/month/year) |
|---|---|---|---|
| | | US 2020297853 A1 | 24 September 2020 |
| | | US 11110174 B2 | 07 September 2021 |
| | | US 2009247608 A1 | 01 October 2009 |
| | | US 2022008541 A1 | 13 January 2022 |
| | | JP 2015025007 A | 05 February 2015 |
| | | JP 2018029599 A | 01 March 2018 |
| | | JP 6542853 B2 | 10 July 2019 |
| | | US 2019099493 A1 | 04 April 2019 |
| | | JP 2017002082 A | 05 January 2017 |
| | | US 2016051691 A1 | 25 February 2016 |
| | | US 9867882 B2 | 16 January 2018 |
| US 2023078200 A1 | 16 March 2023 | AU 2020399636 A1 | 02 June 2022 |
| | | MX 2022007005 A | 25 August 2022 |
| | | EP 4073252 A1 | 19 October 2022 |
| | | JP 2023504744 A | 06 February 2023 |
| | | WO 2021119034 A1 | 17 June 2021 |
| | | CA 3163322 A1 | 17 June 2021 |
| US 2020032270 A1 | 30 January 2020 | CA 3055715 A1 | 13 September 2018 |
| | | TW 201837172 A | 16 October 2018 |
| | | EP 3594345 A1 | 15 January 2020 |
| | | EP 3594345 A4 | 19 May 2021 |
| | | AU 2018232367 A1 | 03 October 2019 |
| | | KR 20190128674 A | 18 November 2019 |
| | | JPWO 2018164186 A1 | 09 January 2020 |
| | | WO 2018164186 A1 | 13 September 2018 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 9011860 B
- WO 2021113682 A1
- WO 2022096394 A1
- WO 2011139702 A
- WO 2013033230 A
- WO 2019105419 A
- WO 2009073809 A
- WO 2009082607 A
- WO 2016081444 A


### Non-patent literature cited in the description


- **RICKLIN et al.** *Nat. Immunol.*, 2010, vol. 11, 785-797